# EUROPÄISCHE PATENTSCHRIFT

(11) **EP 0 541 793 B1**
(45) Veröffentlichungstag und Bekanntmachung des Hinweises auf die Patenterteilung: **08.01.1997**
(21) Anmeldenummer: 92924469.7
(22) Anmeldetag: 26.05.1992
(51) Int. Cl.: C09K 19/30, C07C 43/192, C07C 25/24, C07C 43/225, C07C 22/04, C07C 22/00

(54) **VINYLVERBINDUNGEN UND FLÜSSIGKRISTALLINES MEDIUM**
VINYL COMPOUNDS AND LIQUID-CRYSTAL MEDIUM
COMPOSES VINYLIQUES ET MILIEU DE CRISTAUX LIQUIDES

(30) Priorität: 05.06.1991 DE 4118425; 05.12.1991 DE 4140135; 17.03.1992 DE 4208551; 03.04.1992 DE 4211150
(43) Veröffentlichungstag der Anmeldung: 19.05.1993
(73) Patentinhaber: MERCK PATENT GmbH, D-64271 Darmstadt (DE)
(72) Erfinder: BARTMANN, Ekkehard, D-6106 Erzhausen (DE); PLACH, Herbert, D-6100 Darmstadt (DE); EIDENSCHINK, Rudolf, D-6500 Mainz (DE); REIFFENRATH, Volker, D-6101 Rossdorf (DE); PAULUTH, Detlef, D-6100 Darmstadt (DE); POETSCH, Eike, D-6109 Mühltal 6 (DE); SCHOEN, Sabine, D-6100 Darmstadt (DE); MEYER, Volker, D-6112 Gross-Zimmern 2 (DE); JUNGE, Michael, D-6102 Pfungstadt 2 (DE); HITTICH, Reinhard, D-6101 Modautal 1 (DE)
(86) Internationale Anmeldenummer: EP9201185
(87) Internationale Veröffentlichungsnummer: WO9221734

(56) Entgegenhaltungen:
- EP-A- 0 325 796
- EP-A- 0 330 216
- EP-A- 0 377 469
- EP-A- 0 480 217
- WO-A-88/10251
- WO-A-90/13610
- WO-A-91/06522

## Beschreibung

Die Erfindung betrifft neue Vinylverbindungen der Formel I, 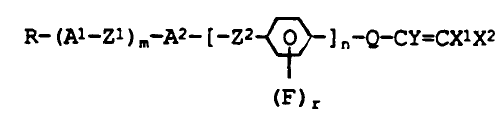 worin
- R: H, einen unsubstituierten, einen einfach durch CN oder CF₃ oder einen mindestens einfach durch Halogen substituierten Alkyl- oder Alkenylrest mit 1 bis 15 C-Atomen, wobei in diesen Resten auch eine oder mehrere CH₂-Gruppen jeweils unabhängig voneinander durch -O-, -S-, 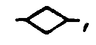 -CO-, -CO-O-, -O-CO- oder -O-CO-O- so ersetzt sein können, daß O-Atome nicht direkt miteinander verknüpft sind,
- A¹ und A²: jeweils unabhängig voneinander einen
(a) trans-1,4-Cyclohexylenrest, worin auch eine oder mehrere nicht benachbarte CH₂-ruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bi-cyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
- Z¹ und Z²: jeweils unabhängig voneinander -CO-O-, -O-CO-, -CH₂O-, -OCH₂-, -CH₂CH₂-, -CH=CH-, -C≡C- oder eine Einfachbindung, einer der Reste Z¹ und Z² auch - (CH₂)₄- oder -CH=CH-CH₂CH₂-,
- Q: eine Einfachbindung, -O-, -CH₂-CH₂-, -C≡C-, trans-CH=CH-, -COO- oder -CH₂O-,
- X¹: H, F oder Cl,
- X²: F, Cl, CF₃, OCF₃, OCHF₂ oder SF₅
- Y: H, F oder Cl,
- r: 0 bis 4,
- n: 0 oder 1 und
- m: 0, 1, 2 oder 3
bedeutet,
mit der Maßgabe,
daß im Falle Y = H und Q = Einfachbindung X¹ und X² Cl bedeuten.

Die Erfindung betrifft weiterhin die Verwendung dieser Verbindungen als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen oder dem Effekt der dynamischen Streuung beruhen.

Der Erfindung lag die Aufgabe zugrunde, neue stabile flüssigkristalline oder mesogene Verbindungen aufzufinden, die als Komponenten flüssigkristalliner Medien geeignet sind und insbesondere gleichzeitig eine vergleichsweise geringe Viskosität besitzen sowie eine relativ hohe dielektrische Anisotropie.

Es wurde nun gefunden, daß Verbindungen der Formel I als Komponenten flüssigkristalliner Medien vorzüglich geeignet sind. Insbesondere verfügen sie über vergleichsweise niedrige Viskositäten. Mit ihrer Hilfe lassen sich stabile flüssigkristalline Medien mit breitem Mesophasenbereich und vorteilhaften Werten für die optische und dielektrische Anisotropie erhalten. Diese Medien weisen ferner ein sehr gutes Tieftemperaturverhalten auf.

Difluorstyrolderivate und entsprechende flüssigkristalline Medien wie z.B. mit Verbindungen der Formel 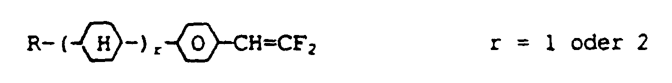 sind bereits aus EP 0 325 796 A1 bekannt. Diese Verbindungen neigen jedoch zu chemischer Instabilität durch HF-Abspaltung.

Verschiedene Verbindungen mit flüssigkristallinen Eigenschaften, in denen terminal eine CF₃-Gruppe gebunden ist, sind bereits bekannt (USP 4,330,426; USP 4,684,476; J.C. Liang and S. Kumar, Mol. Cryst. Liq. Cryst. 1987; Vol. 142, pp. 77-84). Diese Verbindungen haben jedoch oft einen stark smektogenen Charakter und sind für viele praktische Anwendungen weniger geeignet.

Im Hinblick auf die verschiedensten Einsatzbereiche derartiger Verbindungen mit hohem Δε war es jedoch wünschenswert, weitere Verbindungen zur Verfügung zu haben, die auf die jeweiligen Anwendungen genau maßgeschneiderte Eigenschaften aufweisen.

Mit der Bereitstellung von Verbindungen der Formel I wird außerdem ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel I flüssigkristallinen Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Insbesondere Verbindungen der Formel I, worin X¹ = H und X² = F bedeutet, zeichnen sich durch ihren hohen Klärpunkt bei gleichzeitig niedriger Viskosität aus. Die Vinylether der Formel I (Q = 0) weisen eine ungewöhnlich höhe Stabilität auf. Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, insbesondere elektroopische Anzeigeelemente, die derartige Medien enthalten.

Der Einfachheit halber bedeuten im folgenden A³ einen Rest der Formel 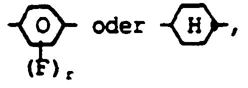 Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bi einen Bicyclo(2,2,2)-octylenrest, wobei Cyc und/oder Phe unsubstituiert oder ein- oder zweifach durch F oder CN substituiert sein können. Z ist CY=CX¹X².

A¹ und A² sind vorzugsweise ausgewählt aus der Gruppe Cyc, Che, Phe, Pyr, Pyd und Dio, wobei vorzugsweise nur einer der im Molekül vorhandenen Reste A¹ und A² Che, Phe, Pyr, Pyd oder Dio ist.

Die Verbindungen der Formel I umfassen dementsprechend Verbindungen mit zwei Ringen der Teilformeln Ia und Ib:

R-A²-A³-Q-Z Ia

R-A²-Z²-A³-Q-Z Ib

Verbindungen mit drei Ringen der Teilformeln Ic bis If:

R-A¹-A²-A³-Q-Z Ic

R-A¹-Z¹-A²-Z²-A³-Q-Z Id

R-A¹-Z¹-A²-A³-Q-Z Ie

R-A¹-A²-Z²-A³-Q-Z If

sowie Verbindungen mit vier Ringen der Teil formeln Ig bis Im:

R-A¹-A¹-A²-A³-Q-Z Ig

R-A¹-Z¹-A¹-A²-A³-Q-Z Ih

R-A¹-A¹-Z¹-A²-A³-Q-Z Ii

R-A¹-A¹-A²-Z¹-A³-Q-Z Ij

R-A¹-Z¹-A¹-Z¹-A²-A³-Q-Z Ik

R-A¹-A¹-Z¹-A²-Z²-A³-Q-Z Il

R-A¹-Z¹-A¹-Z¹-A²-Z²-A³-Q-Z Im

Darunter sind besonders diejenigen der Teilformeln Ia, Ib, Ic, Id, Ie, If, Ii und Il bevorzugt.

Die bevorzugten Verbindungen der Teilformel Ia umfassen diejenigen der Teilformeln Iaa bis Iah:

R-Phe-A³-Q-Z Iaa

R-Phe-A³-Q-Z Iab

R-Dio-A³-Q-Z Iac

R-Pyr-A³-Q-Z Iad

R-Pyd-A³-Q-Z Iae

R-Cyc-A³-Q-Z Iaf

R-Cyc-A³-Q-Z Iag

R-Che-A³-Q-Z Iah

Darunter sind diejenigen der Formeln Iaa, Iab, Iac, Iad, Iaf und Iag besonders bevorzugt.

Die bevorzugten Verbindungen der Teilformel Ib umfassen diejenigen der Teilformeln Iba und Ibb:

R-Cyc-CH₂CH₂-A³-Q-Z Iba

R-Cyc-COO-A³-Q-Z Ibb

Die bevorzugten Verbindungen der Teilformel Ic umfassen diejenigen der Teilformeln Ica bis Ico:

R-Phe-Phe-A³-Q-Z Ica

R-Phe-Phe-A³-Q-Z Icb

R-Phe-Dio-A³-Q-Z Icc

R-Cyc-Cyc-A³-Q-Z Icd

R-Phe-Cyc-A³-Q-Z Ice

R-Cyc-Cyc-A³-Q-Z Icf

R-Pyd-Phe-A³-Q-Z Icg

R-Pyr-Phe-A³-Q-Z Ich

R-Phe-Pyr-A³-Q-Z Ici

R-Cyc-Pyr-A³-Q-Z Icj

R-Cyc-Phe-A³-Q-Z Ick

R-Cyc-Phe-A³-Q-Z Icl

R-Dio-Phe-A³-Q-Z Icm

R-Che-Phe-A³-Q-Z Icn

R-Phe-Che-A³-Q-Z Ico

Darunter sind diejenigen der Formeln Ica, Icc, Icd, Ice, Ici und Icj besonders bevorzugt.

Die bevorzugten Verbindungen der Teilformel Id umfassen diejenigen der Teilformeln Ida bis Idm:

R-Phe-Z¹-Phe-Z¹-A³-Q-Z Ida

R-Phe-Z¹-Phe-Z¹-A³-Q-Z Idb

R-Phe-Z¹-Dio-Z¹-A³-Q-Z Idc

R-Cyc-Z¹-Cyc-Z¹-A³-Q-Z Idd

R-Cyc-Z¹-Cyc-Z¹-A³-Q-Z Ide

R-Pyd-Z¹-Phe-Z¹-A³-Q-Z Idf

R-Phe-Z¹-Pyd-Z¹-A³-Q-Z Idg

R-Pyr-Z¹-Phe-Z¹-A³-Q-Z Idh

R-Phe-Z¹-Pyr-Z¹-A³-Q-Z Idi

R-Phe-Z¹-Cyc-Z¹-A³-Q-Z Idj

R-Cyc-Z¹-Phe-Z¹-A³-Q-Z Idk

R-Cyc-Z¹-Phe-Z¹-A³-Q-Z Idl

R-Dio-Z¹-Phe-Z¹-A³-Q-Z Idm

Die bevorzugten Verbindungen der Teilformel Ie umfassen diejenigen der Teilformeln Iea bis Iel:

R-Pyr-Z¹-Phe-A³-Q-Z Iea

R-Dio-Z¹-Phe-A³-Q-Z Ieb

R-Phe-Z¹-Phe-A³-Q-Z Iec

R-Cyc-Z¹-Phe-A³-Q-Z Ied

R-Cyc-Z¹-Phe-A³-Q-Z Iee

R-Phe-Z¹-Cyc-A³-Q-Z Ief

R-Cyc-Z¹-Cyc-A³-Q-Z Ieg

R-Cyc-Z¹-Cyc-A³-Q-Z Ieh

R-Phe-Z¹-Dio-A³-Q-Z Iei

R-Pyd-Z¹-Phe-A³-Q-Z Iej

R-Phe-Z¹-Pyr-A³-Q-Z Iek

R-Cyc-Z¹-Pyr-A³-Q-Z Iel

Die bevorzugten Verbindungen der Teilformel If umfassen diejenigen der Teilformeln Ifa bis Ifr:

R-Pyr-Phe-Z¹-A³-Q-Z Ifa

R-Pyr-Phe-OCH₂-A³-Q-Z Ifb

R-Phe-Phe-Z¹-A³-Q-Z Ifc

R-Phe-Phe-OOC-A³-Q-Z Ifd

R-Phe-Phe-Z¹-A³-Q-Z Ife

R-Cyc-Cyc-Z¹-A³-Q-Z Iff

R-Cyc-Cyc-Z¹-A³-Q-Z Ifg

R-Cyc-Cyc-CH₂CH₂-A³-Q-Z Ifh

R-Pyd-Phe-Z¹-A³-Q-Z Ifi

R-Dio-Phe-Z¹-A³-Q-Z Ifj

R-Phe-Cyc-Z¹-A³-Q-Z Ifk

R-Phe-Cyc-Z¹-A³-Q-Z Ifl

R-Phe-Pyd-Z¹-A³-Q-Z Ifm

R-Che-Phe-Z¹-A³-Q-Z Ifn

R-Phe-Che-Z¹-A³-Q-Z Ifo

R-Cyc-Phe-Z¹-A³-Q-Z Ifp

R-Cyc-Phe-OOC-A³-Q-Z Ifq

R-Cyc-Phe-Z¹-A³-Q-Z Ifr

R bedeutet vorzugsweise Alkyl, ferner Alkoxy. A¹ und/oder A² bedeuten bevorzugt Phe, Cyc, Che, Pyr oder Dio. Bevorzugt enthalten die Verbindungen der Formel I nicht mehr als einen der Reste Bi, Pyd, Pyr, Dio oder Dit.

Bevorzugt sind auch Verbindungen der Formel I sowie aller Teilformeln, in denen A¹ und/oder A² ein- oder zweifach durch F oder einfach durch CN substituiertes 1,4-Phenylen bedeutet. Insbesondere sind dies 2-Fluor-1,4-phenylen, 3-Fluor-1,4-phenylen und 3,5-Difluor-1,4-phenylen sowie 2-Cyan-1,4-phenylen und 3-Cyan-1,4-phenylen. In einer besonders bevorzugten Ausführungsform ist A² 3, 5-Difluor1,4-phenylen und m oder 2.

Z¹ und Z² bedeuten bevorzugt eine Einfachbindung, -CO-O-, -O-CO- und -CH₂CH,-, in zweiter Linie bevorzugt -CH₂O- und -OCH₂-.
- A³: ist vorzugsweise 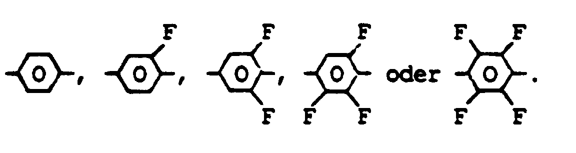
- Z: ist vorzugsweise

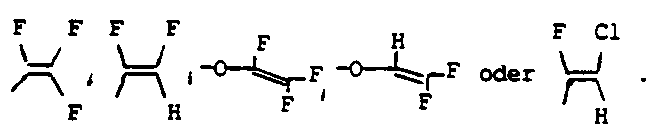

Falls einer der Reste Z¹ und Z² -(CH₂)₄- oder -CH=CH-CH₂CH₂-bedeutet, so ist der andere Rest Z¹ oder Z² (falls vorhanden) vorzugsweise die Einfachbindung.

Bevorzugte Verbindungen dieses Types entsprechen der Teilformel I' 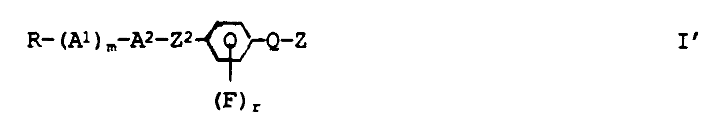 worin Z² -(CH₂)₄- oder -CH=CH-CH₂CH₂- bedeutet und R, A¹, A², m, Q und Z die angegebene Bedeutung haben. Auch die bevorzugten Bedeutungen für R, A¹, A², m, Q und Z entsprechen denen für die Verbindungen der Formel I.

m ist vorzugsweise 1 oder 0, insbesondere bevorzugt 0.

Besonders bevorzugte Verbindungen entsprechen den Teilformeln I1 bis I10.

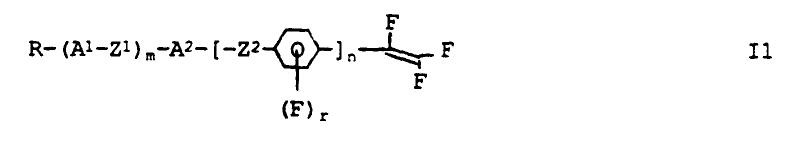

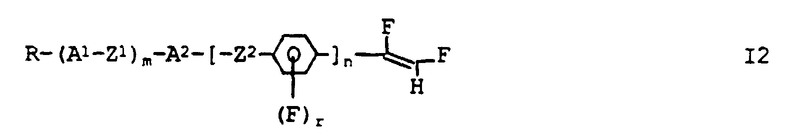

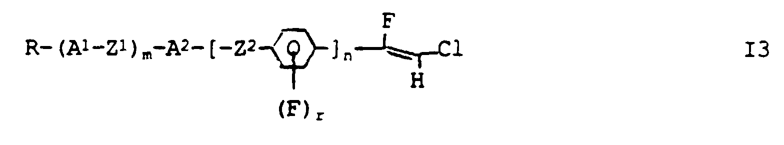

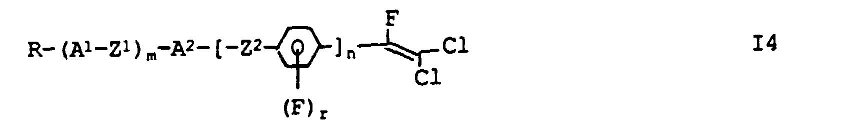

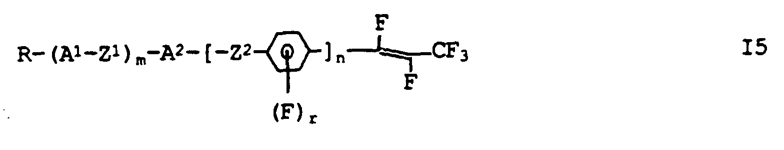

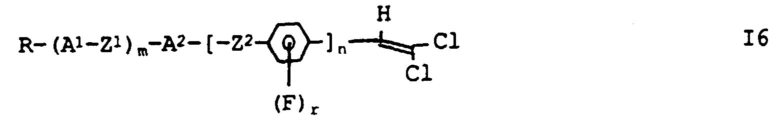

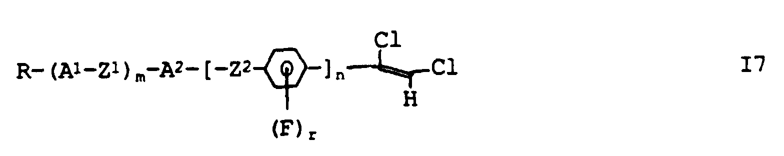

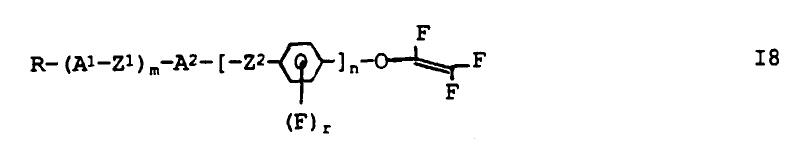

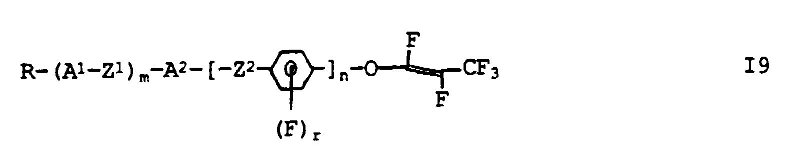

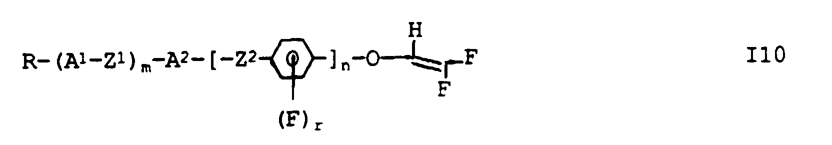

Falls R einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetra-decoxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R einen Alkylrest bedeutet, in dem eine CH₂-Gruppe durch unsubstituiertes oder substituiertes -CH=CH- und eine benachbarte CH₂-Gruppe durch CO oder CO-O oder O-CO-ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxy-propyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Metha- cryloyloxyethyl, 3-Methacryloyloxypropyl, 4-Methacryloyloxy- butyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sei, vorzugsweise jedoch in ω-Position.

Verbindungen der Formel I, die über für Polymerisationsreaktionen geeignete Flügelgruppen R verfügen, eignen sich zur Darstellung flüssigkristalliner Polymerer.

Verbindungen der Formeln I mit verzweigten Flügelgruppen R können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verbindungen der Formel I mit S_{A}-Phasen eignen sich beispielsweise für thermisch adressierte Displays.

Verzweigte Gruppen dieser Art enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R einen Alkylrest darstellt, in dem zwei oder mehr CH₂-ruppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxypentyl, 6,6-Bis-carboxy-hexyl, 7,7-Bis-carboxy-heptyl, 8,8-Bis-carboxy-octyl, 9,9-Bis-carboxy-nonyl, 10,10-Bis-carboxydecyl, Bis-(methoxycarbonyl)-methyl, 2,2-Bis-(methoxycarbonyl)-ethyl, 3,3-Bis-(methoxycarbonyl)-propyl, 4,4-Bis(methoxycarbonyl)-butyl, 5,5-Bis-(methoxy-carbonyl)-pentyl, 6,6-Bis-(methoxycarbonyl)-hexyl, 7,7-Bis-(methoxycarbonyl)heptyl, 8,8-Bis-(methoxycarbonyl)-octyl, Bis-(ethoxycarbonyl)-methyl, 2,2-Bis-(ethoxycarbonyl)-ethyl, 3,3-Bis(ethoxycarbonyl)-propyl, 4,4-Bis-(ethoxycarbonyl)-butyl, 5,5-Bis-(ethoxycarbonyl)-hexyl.

Verbindungen der Formel I, die über für Polykondensationen geeignete Flügelgruppen R verfügen, eignen sich zur Darstellung flüssigkristalliner Polykondensate.

Formel I umfaßt sowohl die Racemate dieser Verbindungen als auch die optischen Antipoden sowie deren Gemische.

Unter diesen Verbindungen der Formel I sowie den Unterformeln sind diejenigen bevorzugt, in denen mindestens einer der darin enthaltenden Reste eine der angegebenen bevorzugten Bedeutungen hat.

In den Verbindungen der Formel I sind diejenigen Stereoisomeren bevorzugt, in denen die Ringe Cyc und Piperidin trans-1,4-disubstituiert sind. Diejenigen der vorstehend genannten Formeln, die eine oder mehrere Gruppen Pyd, Pyr und/oder Dio enthalten, umschließen jeweils die beiden 2,5-Stellungsisomeren.

Einige ganz besonders bevorzugte kleinere Gruppen von Verbindungen sind diejenigen der Teil formeln II1 bis II23 (L¹, L² und L³: H oder F)

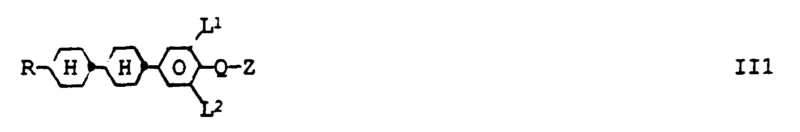

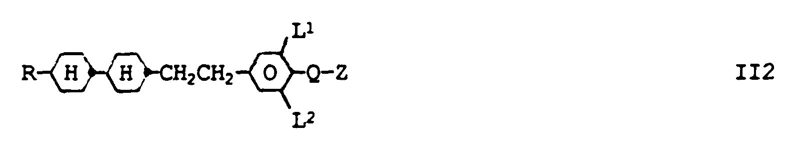

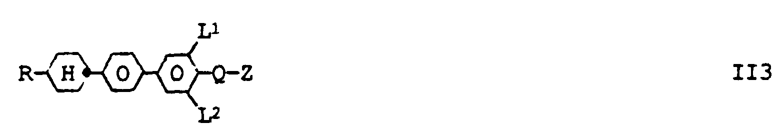

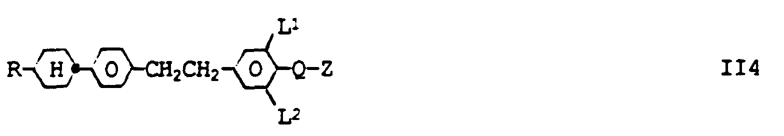

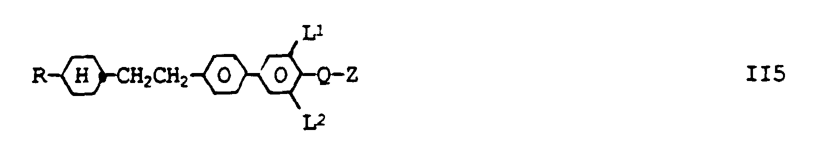

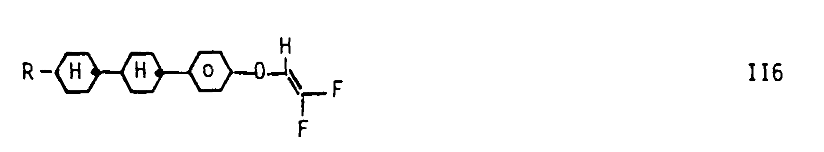

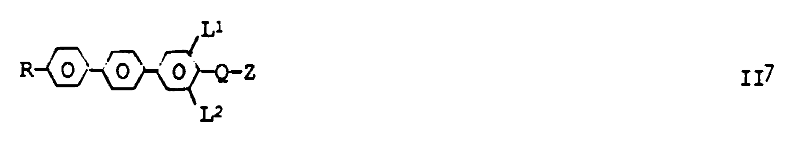

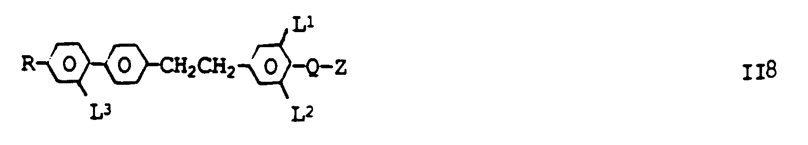

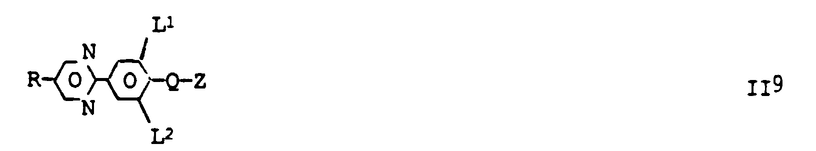

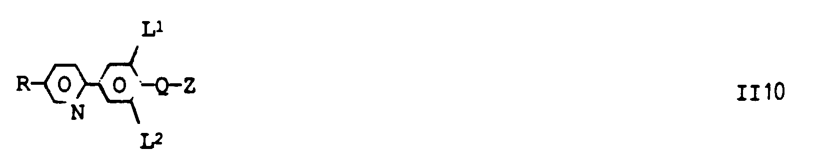

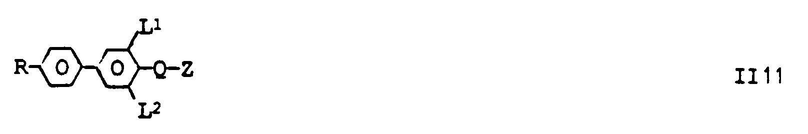

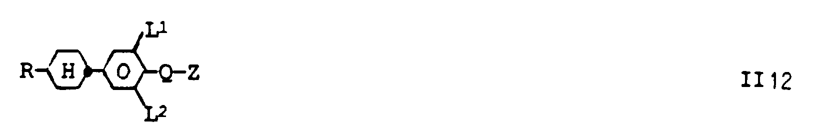

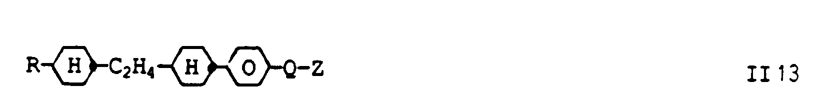

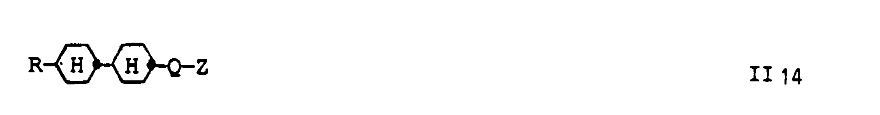

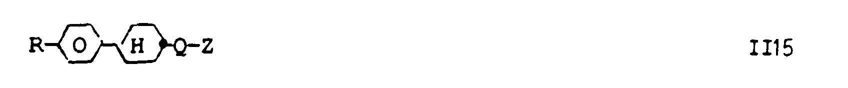

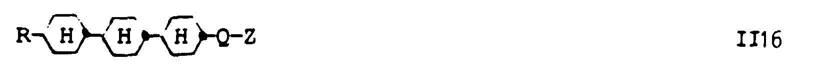

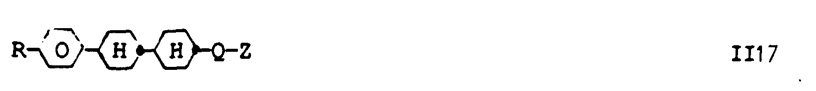

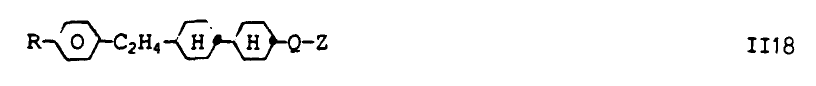

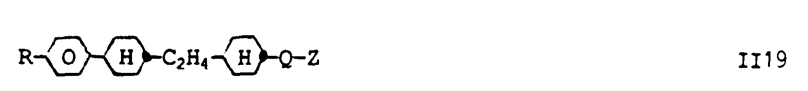

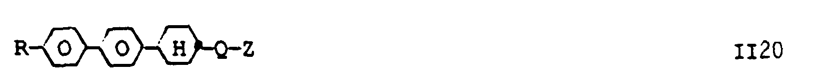

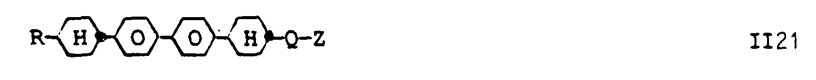

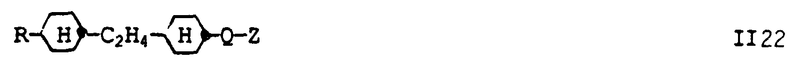

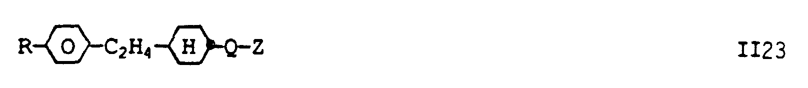

Die 1,4-Cyclohexenylen-Gruppe hat vorzugsweise folgende Strukturen: 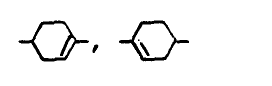 Ebenfalls bevorzugt sind den Teilformeln I1 bis I11 entsprechende Verbindungen mit 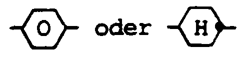 anstelle von

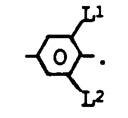

Die Verbindungen der Formel I werden nach an sich bekannten Methoden dargestellt, wie sie in der.Literatur (z.B. in den Standardwerken wie Houben-Weyl, Methoden der Organischen Chemie, Georg-Thieme-Verlag, Stuttgart Bd. IX, S. 867 ff.) beschrieben sind, und zwar unter Reaktionsbedingungen, die für die genannten Umsetzungen bekannt und geeignet sind.

Dabei kann man auch von an sich bekannten, hier nicht näher erwähnten Varianten Gebrauch machen.

Erfindungsgemäße Vinylverbindungen können z.B. hergestellt, indem man eine Verbindung der Formel II, 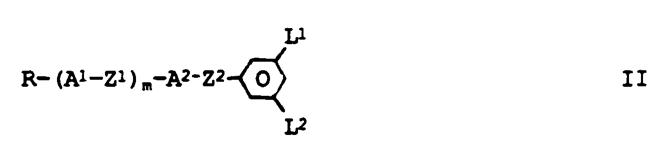 worin L¹ und L² H oder F bedeuten und R, A¹, A², Z¹, Z² und m die angegebene Bedeutung haben, gemäß folgendem Reaktionsschema metalliert und anschließend mit einem geeigneten Elektrophil umsetzt:

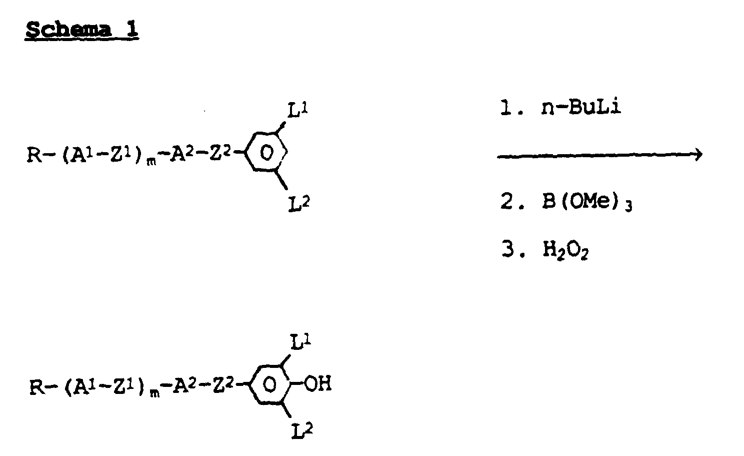

Aus dem erhaltenen Phenol sind die Zielprodukte mit Q = O nach bekannten Methoden, z. B. durch Umsetzung mit Tetrafluorethylen und äquivalenter Menge Base erhältlich (K. Okuhara et al., Bull. Chem. Soc. Jpan. 35, 534 (1962); W.J. Pummer and L.A. Wall, SPE Transactions 3, 220 (1963) und Knunyants/Yakobson, Syntheses of Fluoroorganic Compounds, Springer 1985).

Teilweise treten jedoch durch die als Nebenprodukte entstehenden Tetrafluorethylether Probleme bei der Aufreinigung auf, so daß hier zur Herstellung der Trifluorvinylether die baseninduzierte HF-Abspaltung aus Tetrafluorethylether in Frage kommt:

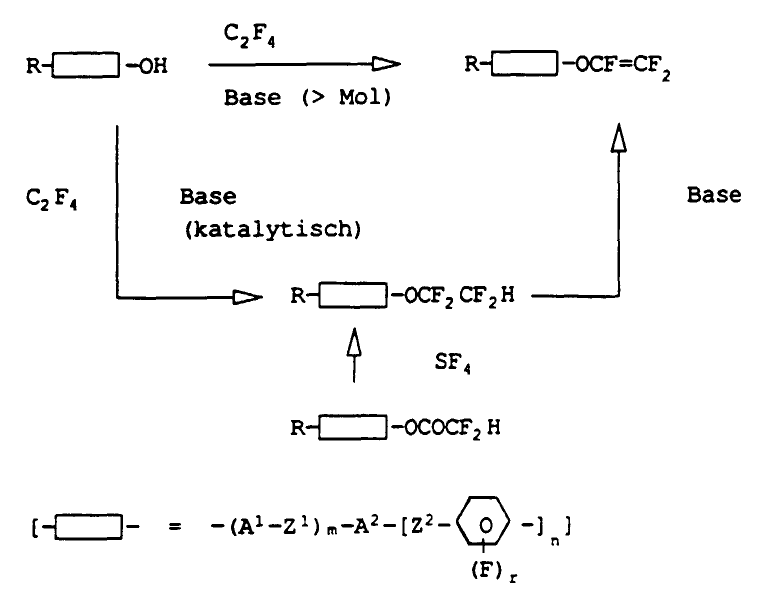

Der indirekte Weg über die Tetrafluorethylether ist vorteilhafter als der direkte Syntheseweg, da letzterer mit Komplikationen behaftet ist und zu Produktgemischen führt.

Weitere Synthesemethoden sind für den Fachmann augenscheinlich. Beispielsweise kann der Rest R-(A¹-Z¹)ₘ-A²-Z² durch in der Flüssigkristallchemie gebräuchliche Reaktionen (z.B. Veresterung, Veretherung oder Kopplungen z.B. gemäß der Artikel E. Poetsch, Kontakte (Darmstadt) 1988 (2), S. 15) eingeführt werden.

Die Verbindungen der Formel II können beispielsweise nach folgenden Syntheseschemata hergestellt werden:

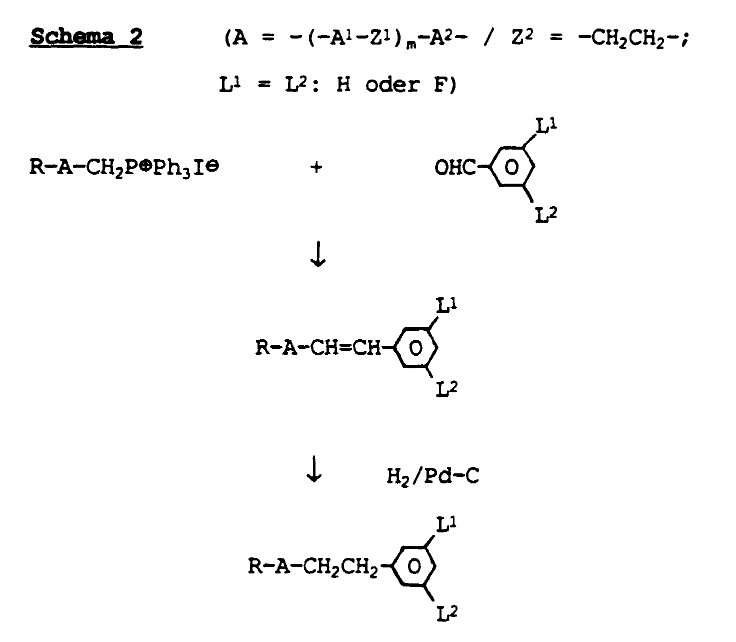

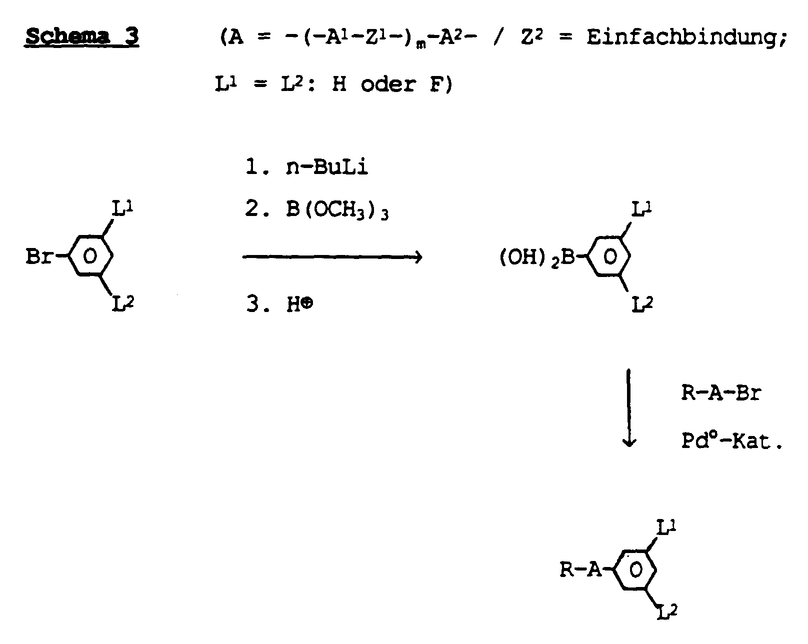

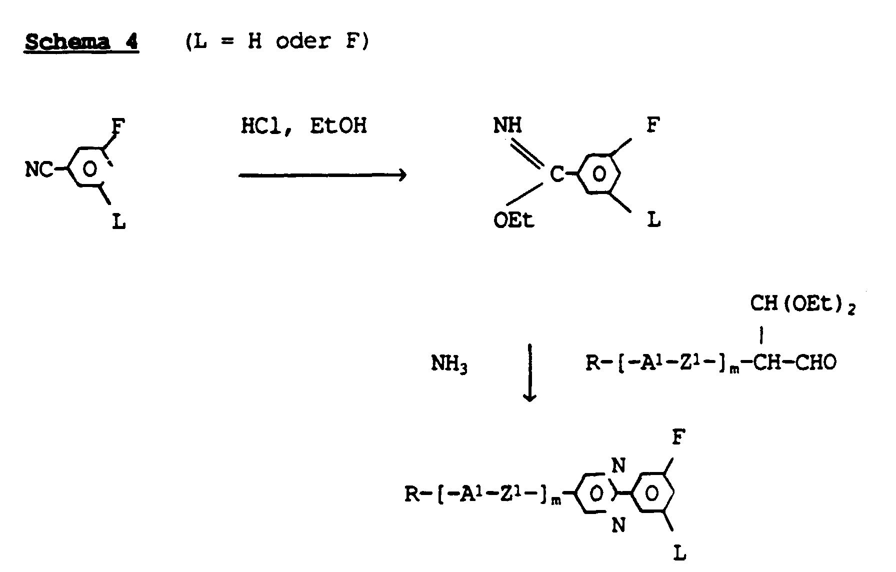

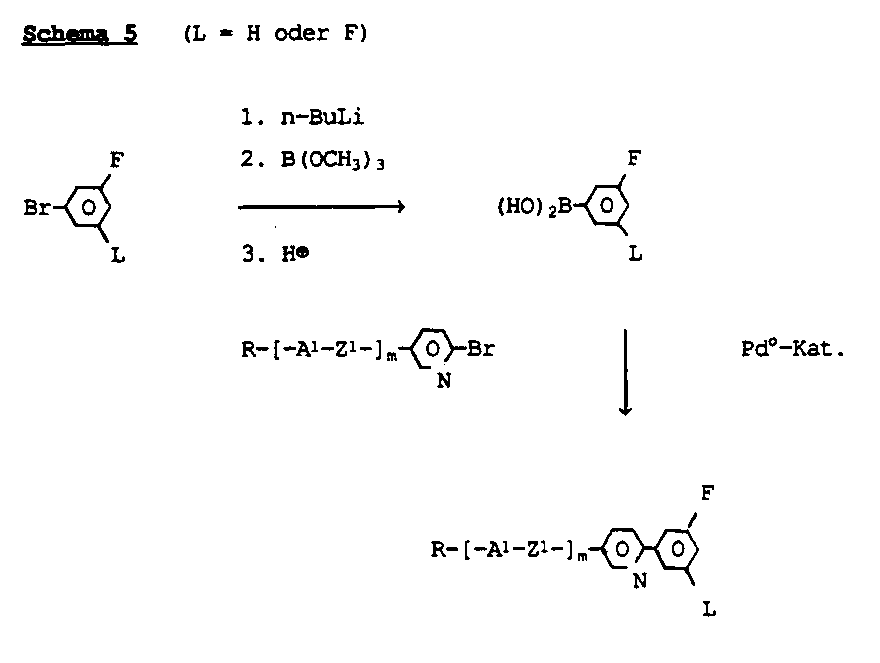

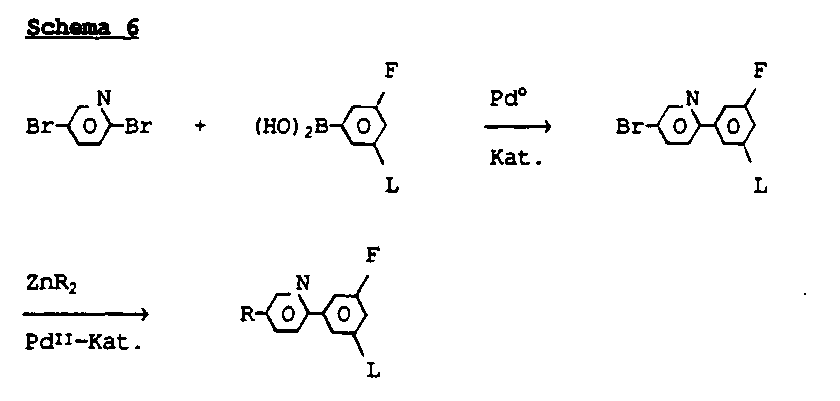

Die Ausgangsmaterialien sind entweder bekannt oder können in Analogie zu bekannten Verbindungen hergestellt werden.

Ester der Formel I können auch durch Veresterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Dicyclohexylcarbodiimid) erhalten werden.

Die entsprechenden Carbonsäuren und Alkohole bzw. Phenole sind bekannt oder können in Analogie zu bekannten Verfahren hergestellt werden.

In einem weiteren Verfahren zur Herstellung der Verbindungen der Formel I setzt man ein Arylhalogenid mit einem Olefin um in Gegenwart eines tertiären Amins und eines Palladiumkatalysators (vgl. R.F. Heck, Acc. Chem. Res. 12 (1979) 146). Geeignete Arylhalogenide sind beispielsweise Chloride, Bromide und Iodide, insbesondere Bromide und Iodide. Die für das Gelingen der Kupplungsreaktion erforderlichen tertiären Amine, wie z.B. Triethylamin, eignen sich auch als Lösungsmittel. Als Palladiumkatalysatoren sind beispielsweise dessen Salze, insbesondere (Pd(II)-acetat, mit organischen Phosphor(III)-Verbindungen wie z.B. Triarylphosphanen geeignet. Man kann dabei in Gegenwart oder Abwesenheit eines inerten Lösungsmittels bei Temperaturen zwischen etwa 0° und 150°, vorzugsweise zwischen 20° und 100°, arbeiten; als Lösungsmittel kommen z.B. Nitrile wie Acetonitril oder Kohlenwasserstoffe wie Benzol oder Toluol in Betracht. Die als Ausgangsstoffe eingesetzten Arylhalogenide und Olefine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Auf diese Weise sind beispielsweise Stilbenderivate herstellbar. Die Stilbene können weiterhin hergestellt werden durch Umsetzung eines 4-substituierten Benzaldehyds mit einem entsprechenden Phoshorylid nach Wittig. Man kann aber auch Tolane der Formel I herstellen, indem man anstelle des Olefins monosubstituiertes Acetylen einsetzt (Synthesis 627 (1980) oder Tetrahedron Lett. 27, 1171 (1986)).

Weiterhin können zur Kopplung von Aromaten Arylhalogenide mit Arylzinnverbindungen umgesetzt werden. Bevorzugt werden diese Reaktionen unter Zusatz eines Katalysators wie z.B. eines Palladium(0)komplexes in inerten Lösungsmitteln wie Kohlenwasserstoffen bei hohen Temperaturen, z.B. in siedendem Xylol, unter Schutzgas durchgeführt.

Kopplungen von Alkinyl-Verbindungen mit Arylhalogeniden können analog dem von A.O. King, E. Negishi, F.J. Villani und A. Silveira in J. Org. Chem. 43, 358 (1978) beschriebenen Verfahren durchgeführt werden.

Tolane der Formel I können auch über die Fritsch-Buttenberg-Wiechell-Umlagerung (Ann. 279, 319, 1984) hergestellt werden, bei der 1,1-Diaryl-2-halogenethylene umgelagert werden zu Diarylacetylenen in Gegenwart starker Basen.

Tolane der Formel I können auch hergestellt werden, indem man die entsprechenden Stilbene bromiert und anschließend einer Dehydrohalogenierung unterwirft. Dabei kann man an sich bekannte, hier nicht näher erwähnte Varianten dieser Umsetzung anwenden.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entsprechenden Alkylhalogenid, -sulfonat oder Dialkylsulfat umgesetzt werden, zweckmäßig in einem inerten Lösungsmittel wie z. B. Aceton, 1,2-Dimethoxyethan, DMF oder Dimethylsulfoxid oder auch mit einem Überschuß an wäßriger oder wäßrig-alkoholischer NaOH oder KOH bei Temperaturen zwischen etwa 20° und 100°.

Die Ausgangsmaterialien sind entweder bekannt oder können in Analogie zu bekannten Verbindungen hergestellt werden.

Die Verbindungen der Formel I' mit Z² = -(CH₂)₄- können nach folgendem Schema hergestellt werden:

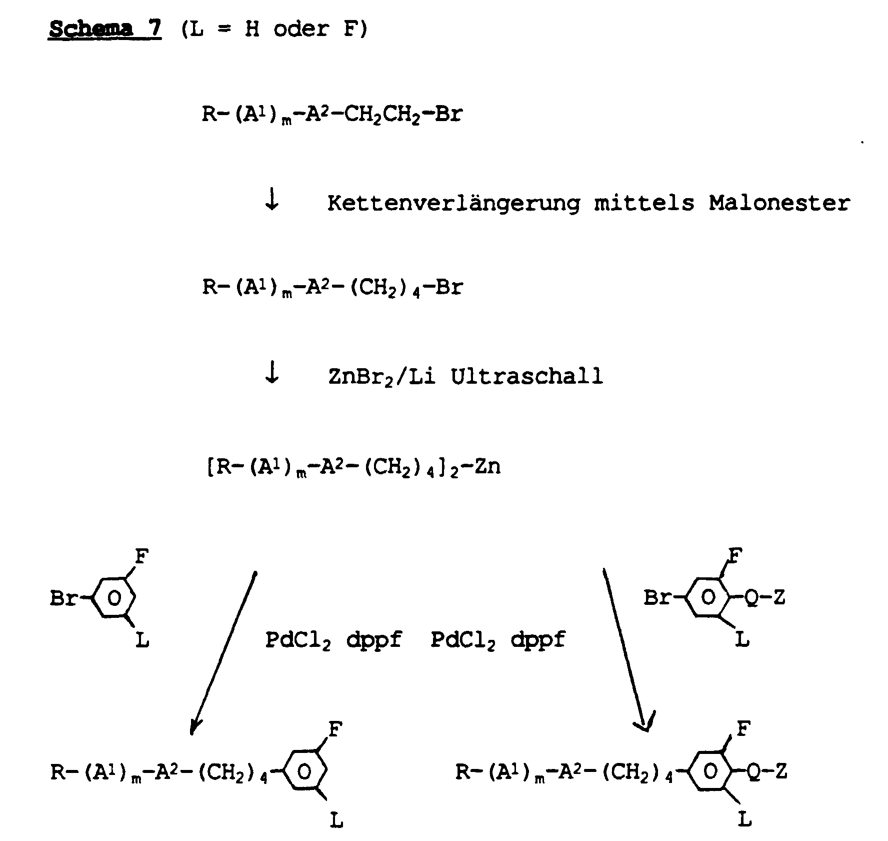

Bei der Pd(II)-katalysierten Kopplungsreaktion wird entweder direkt das Zielprodukt I' gebildet oder ein Vorprodukt, in das völlig analog zu den vorstehenden Methoden für Verbindungen oder Formel I der Rest -Q-Z eingeführt wird.

Die Verbindungen der Formel I' mit Z² = -CH=CH-CH₂CH₂- können noch Wittig gemäß folgendem Schema hergestellt werden:

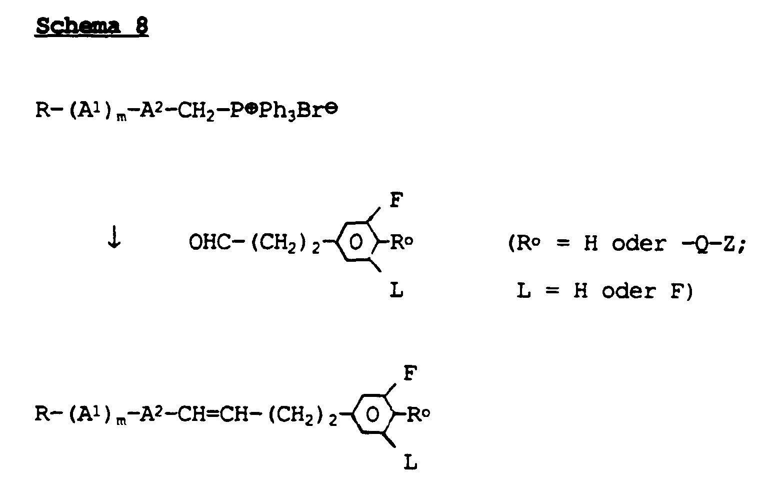

Die bevorzugten trans-Isomeren können nach können nach den literaturbekannten Isomerisierungsmethoden hergestellt werden. Die ggf. erhaltenen Vorprodukte mit R^{o} = H werden völlig analog zu den Vorprodukten der Verbindungen der Formel I durch Einführen des Restes -Q-Z in die Verbindungen der Formel I' übergeführt.

Die Aldehyde können durch Heck-Reaktion von entsprechend substituierten l-Brom-3-fluorbenzolderivaten mit Allylalkohol erhalten werden.

Die erfindungsgemäßen Trifluorvinylverbindungen (Y = F, X¹ = X² = F) mit Q = Einfachbindung können nach bekannten Syntheseverfahren hergestellt werden:

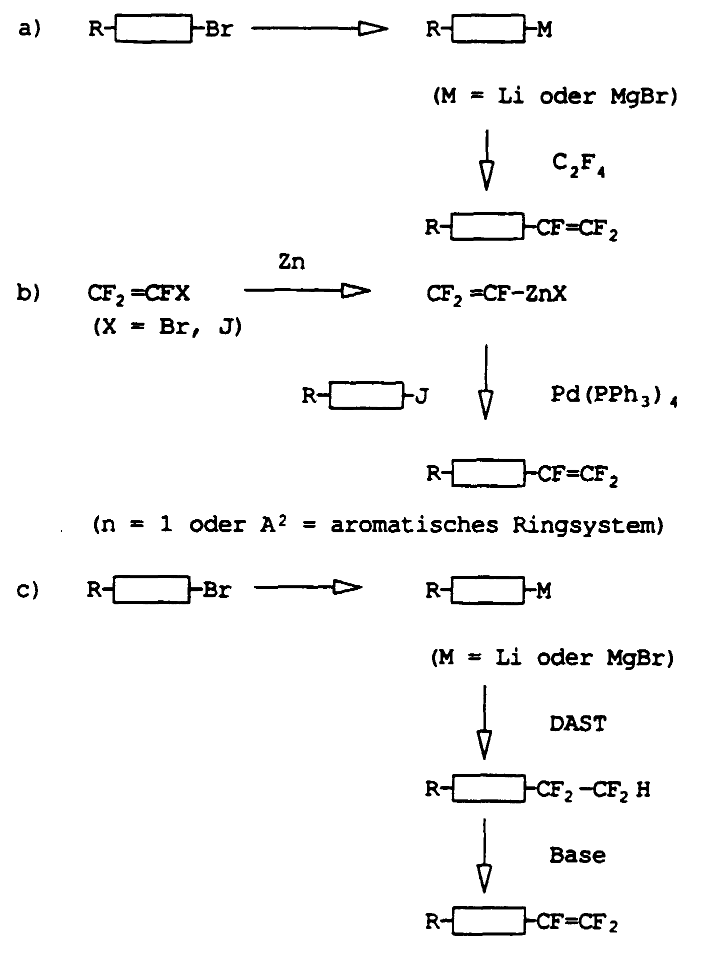

Weitere Details betreffend Reaktionsbedingungen bzw. Ausgangsmaterialien sind zu finden in
Houben-Weyl, Band V/3, S. 424-431;
S. Dixon, J. Org. Chem. 21 (1956) 400;
P.L. Hinze und D.J. Button, ebenda 53 (1988) 2714;
DOS 40 02 374 und DOS 40 06 921.

Die 1,2-Difluorvinylverbindungen (Y = F, X¹ = H, X² = F) lassen sich gemäß folgendem Reaktionsschema darstellen:

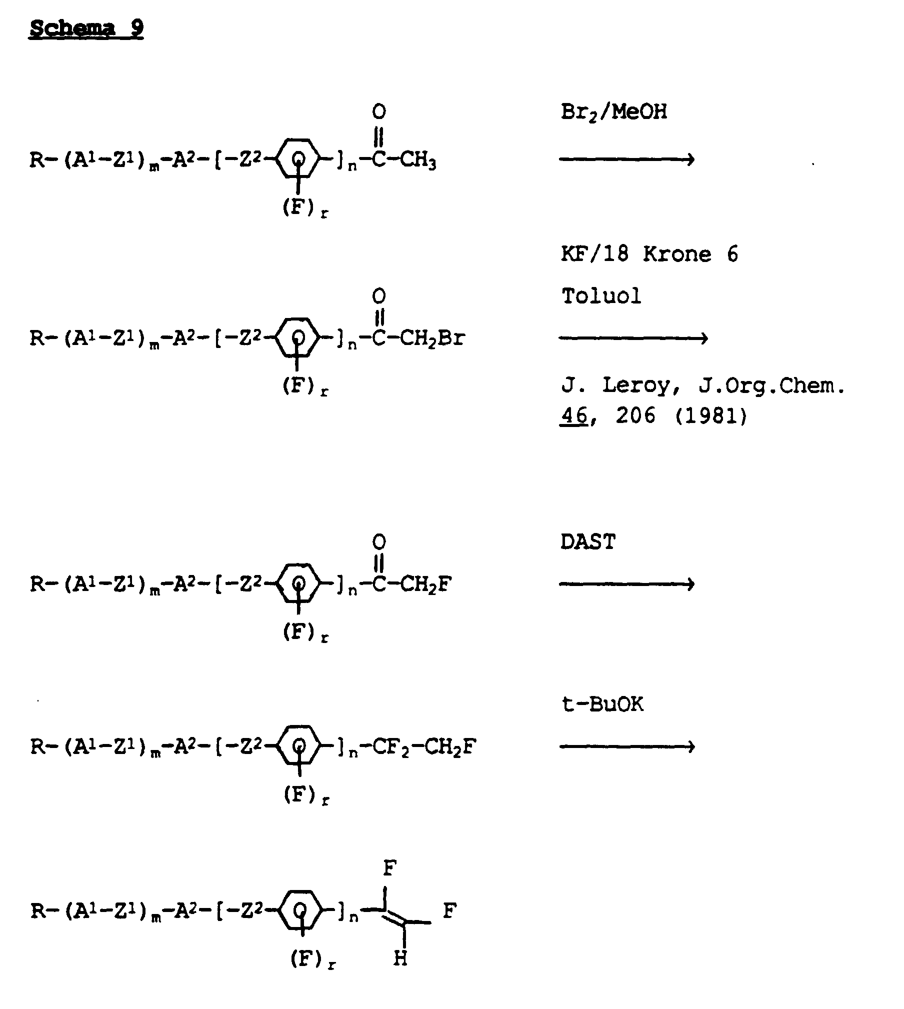

Die 1-Fluor-2-chlor-vinylverbindungen (Y = F, X¹=Cl,X²=H) lassen sich wie folgt herstellen:

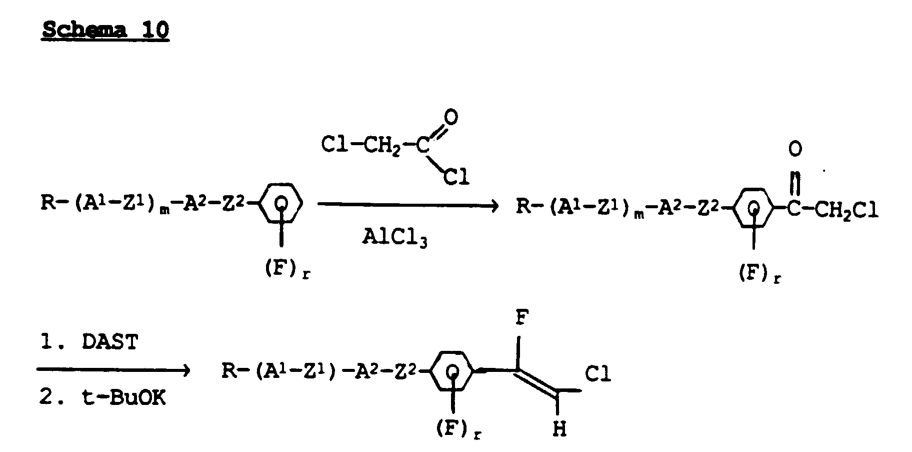

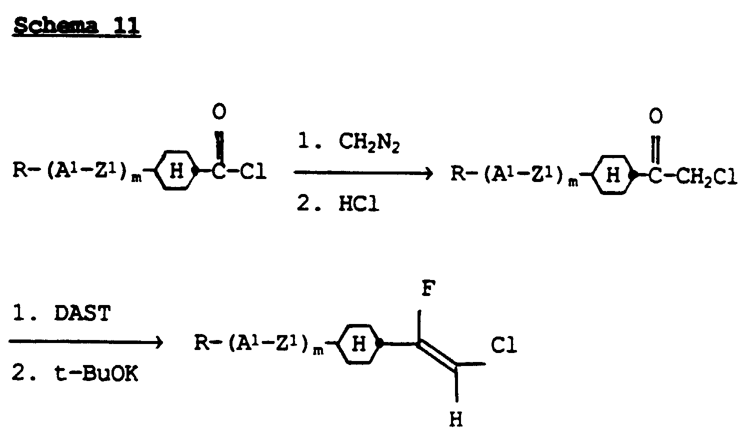

Die Dichlorfluorvinylverbindungen (Y = F, X¹=X²=Cl) sind wie folgt darstellbar:

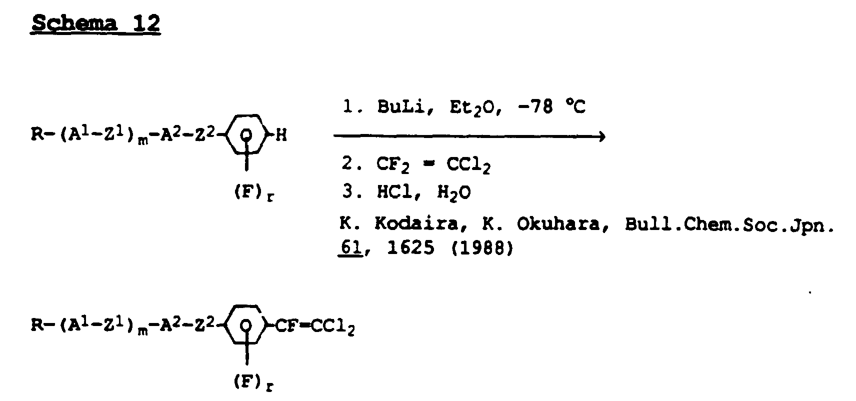

Die 1,2-Difluorvinylverbindungen (Y = F, X¹ = F, X² = F oder SF₅) lassen sich wie folgt herstellen:

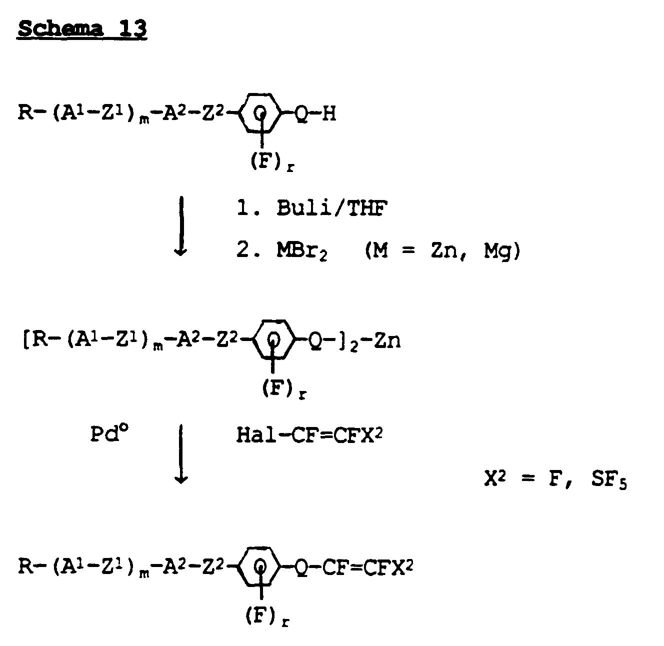

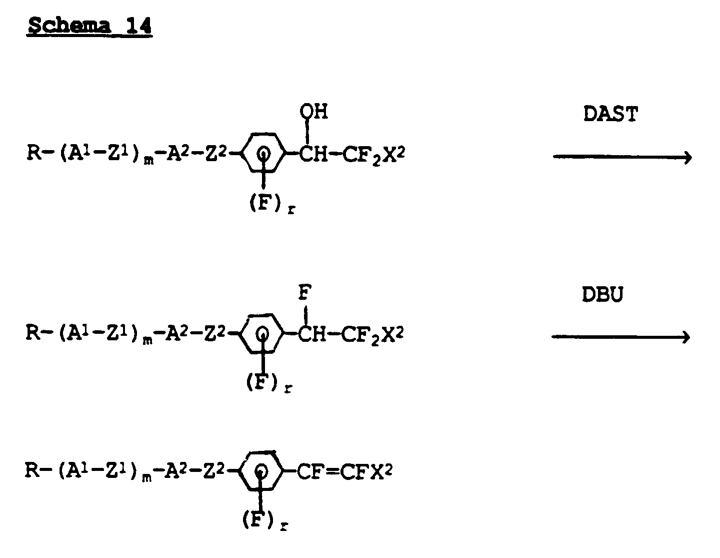

Die 1,1,1,2,3-Pentafluorvinylether (Q = O, Y = F, X¹ = F, X² = CF₃) sind gemäß folgender Schemata herstellbar:

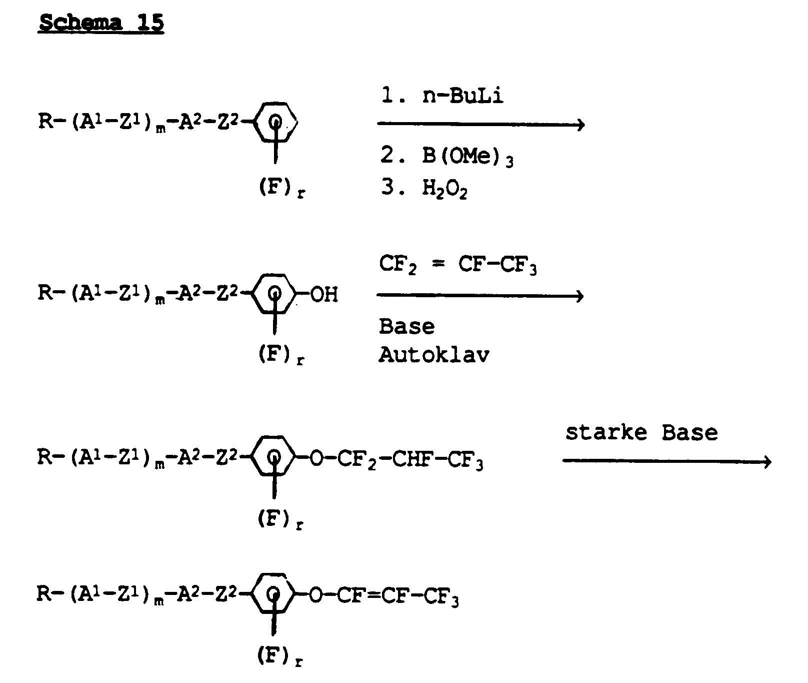

Als starke Basen sind Kalium-t-butylat, Lithiumdiisopropylamid, n-Butyllithium/Kalium-t-butylat sowie t-Butyllithium geeignet. Bevorzugt wird Kalium-t-butylat in einem polar aprotischen Lösungsmittel wie Dimethylsulfoxid verwendet.

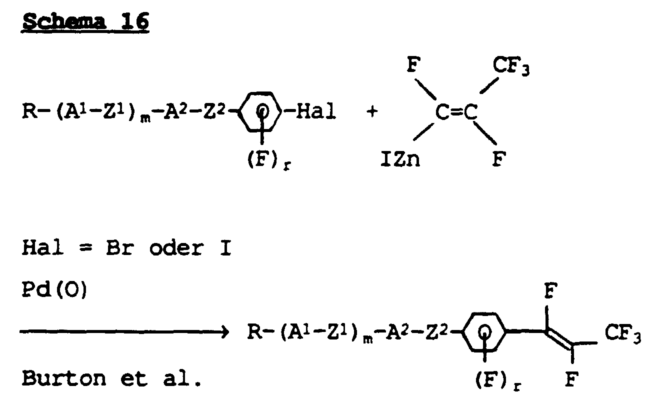

Die 1,2-Dichlorvinylverbindungen (Y = X¹ = Cl, X² = H) werden wie folgt hergestellt:

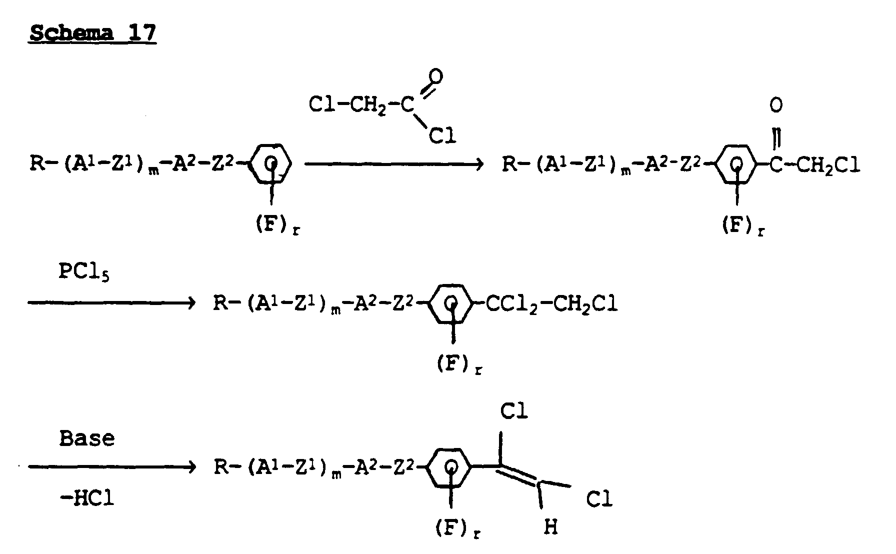

Die 1,1-Dichlorvinylverbindung (Y = H, X¹ = X² = Cl) lassen sich gemäß Schema 18 herstellen.

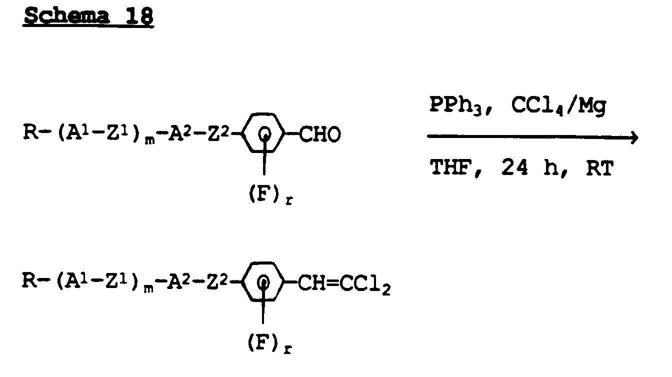

Die Synthese einiger besonders bevorzugter Verbindungen ist im folgenden näher angegeben:

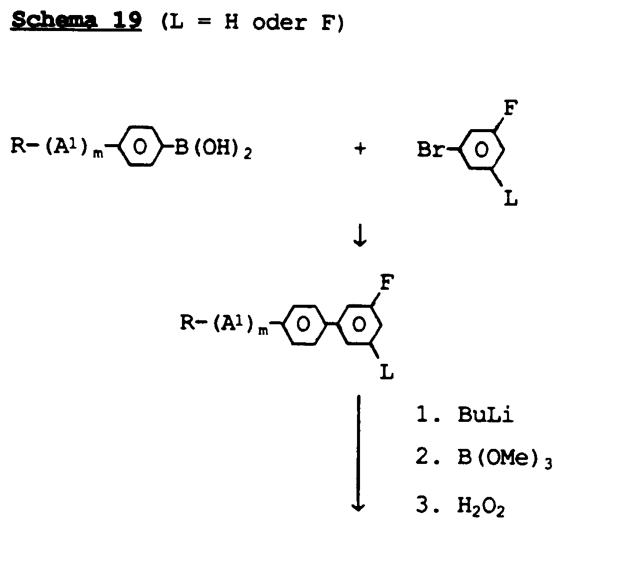

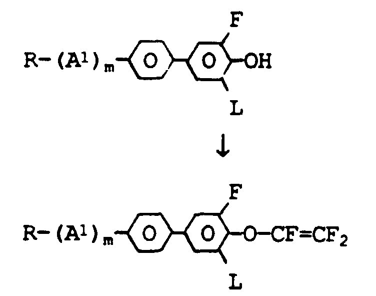

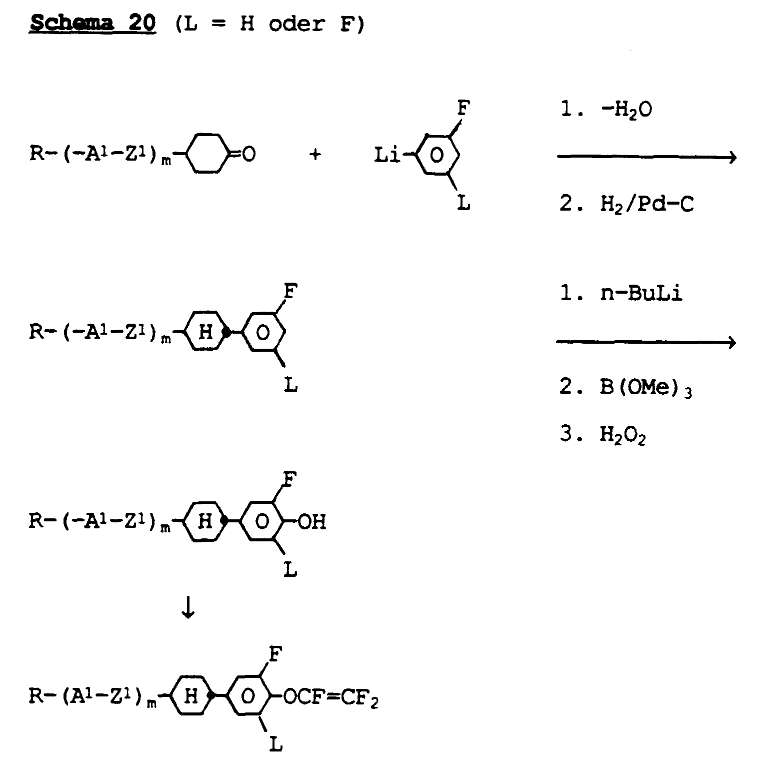

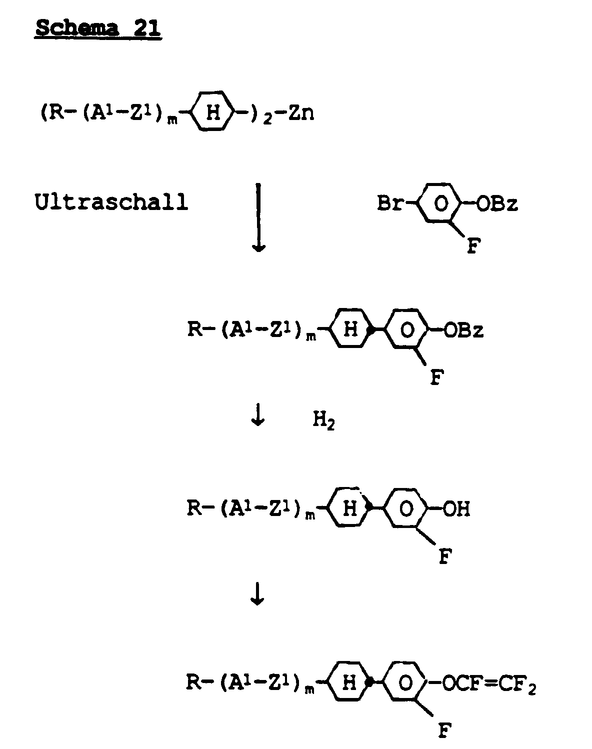

In den Schemata 19, 20, und 21 ist vorzugsweise m 0 oder 1 und -A¹-Z¹ =

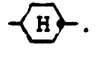

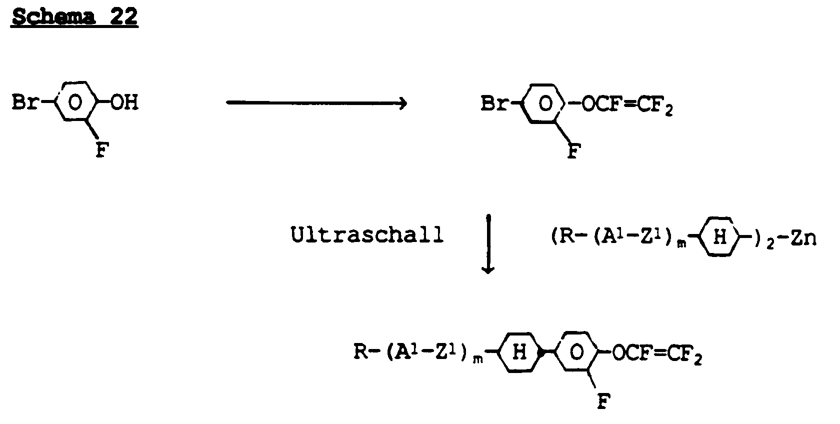

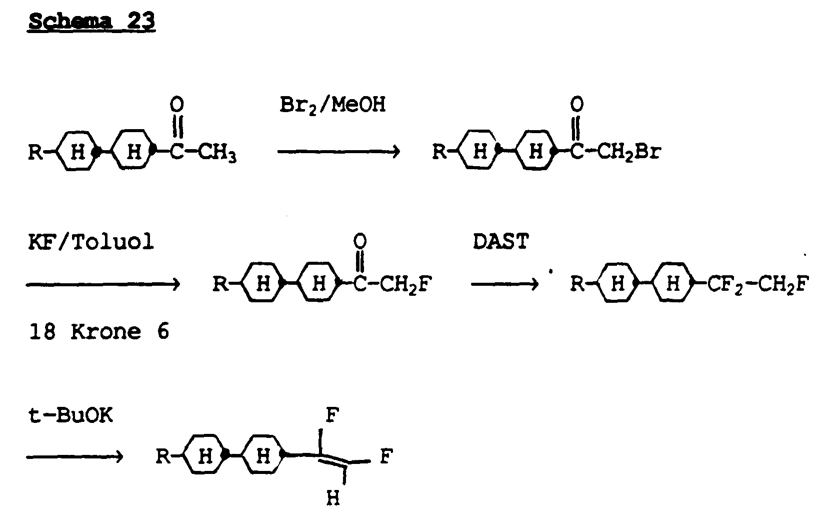

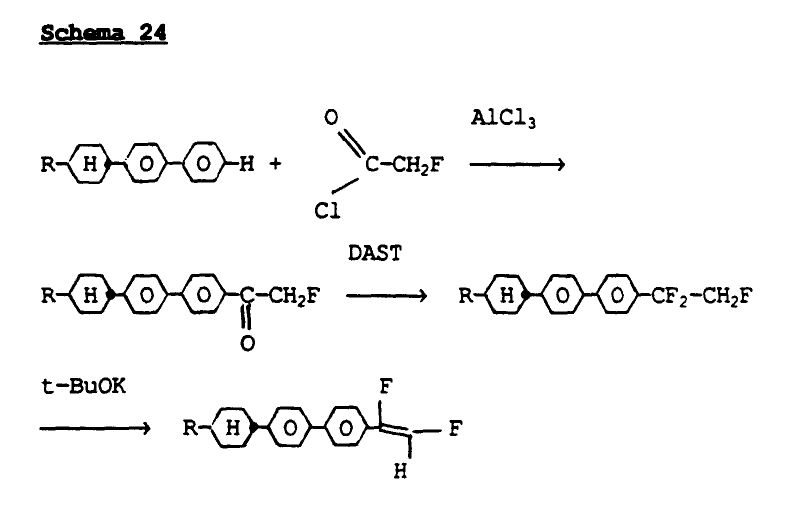

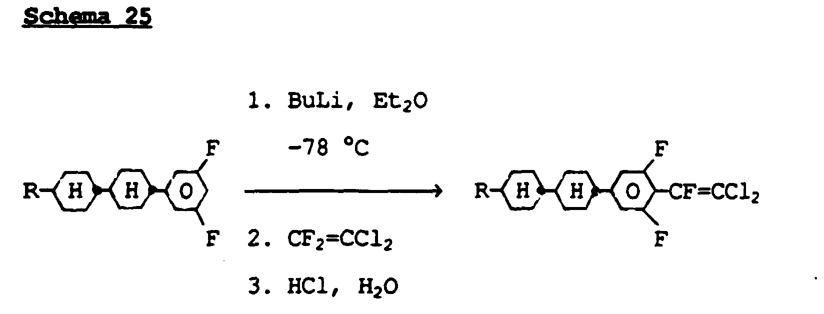

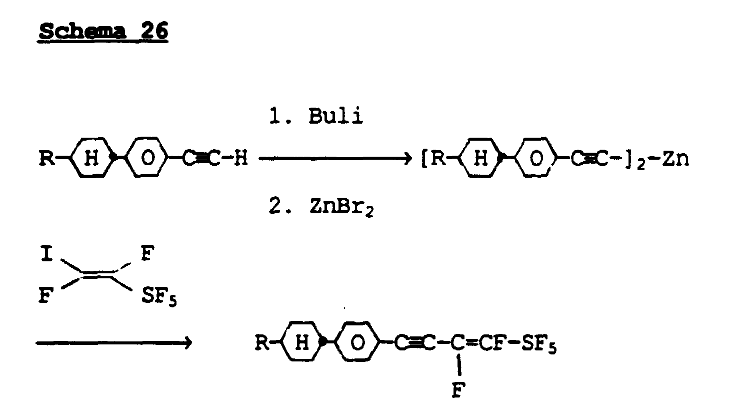

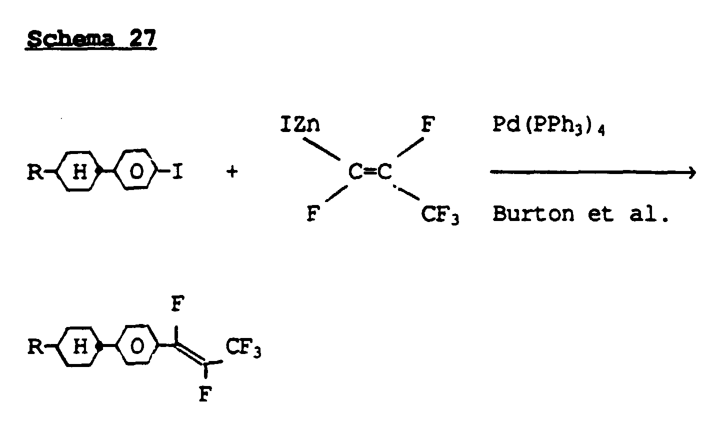

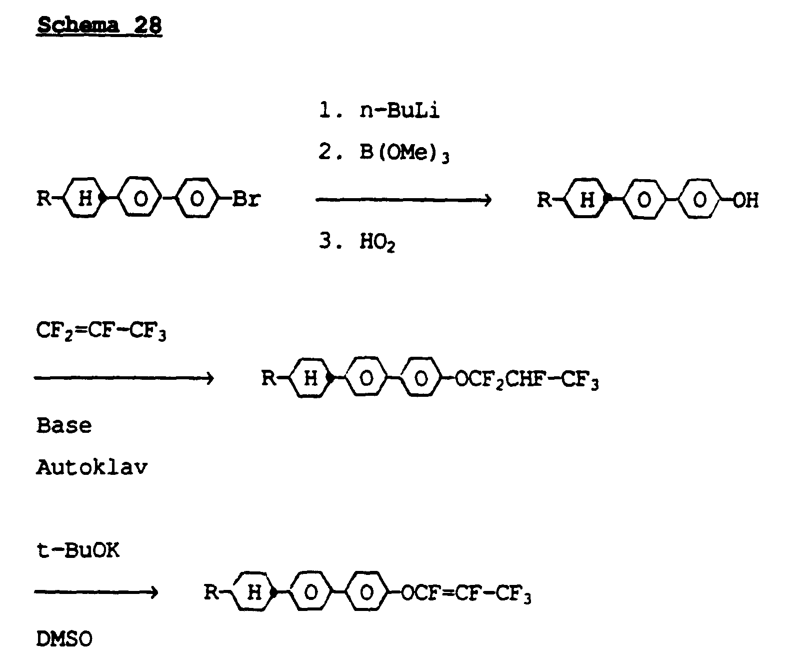

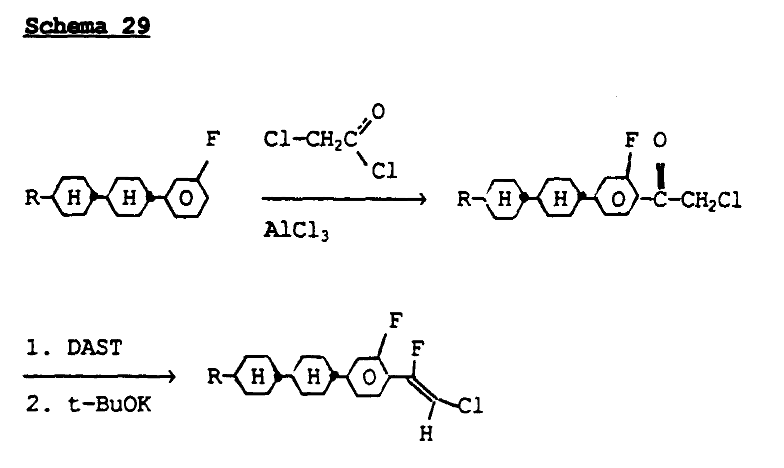

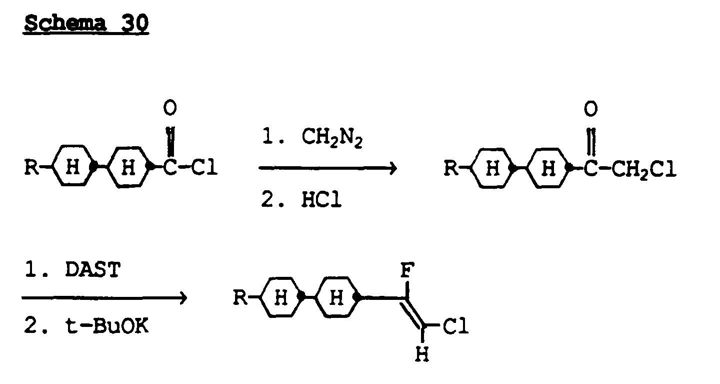

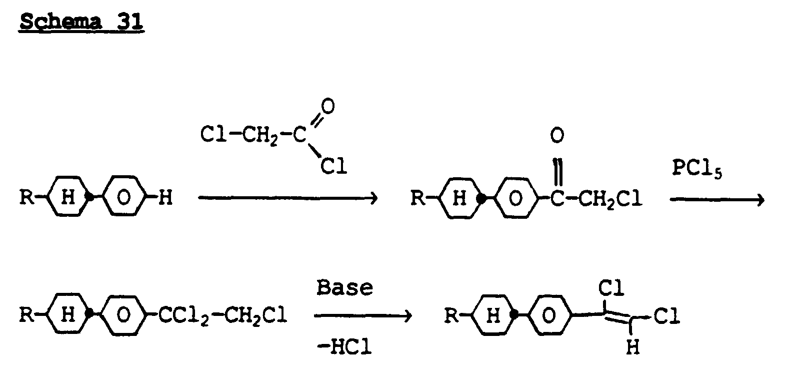

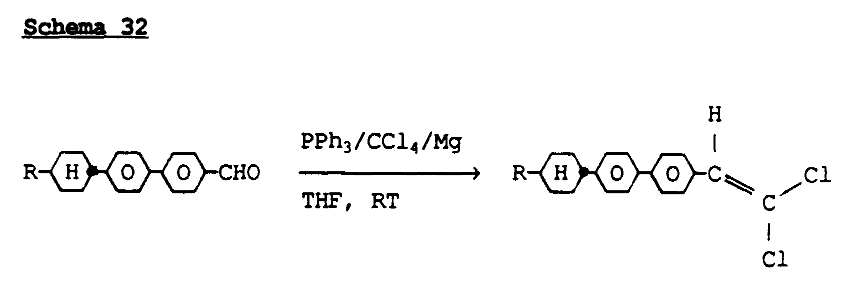

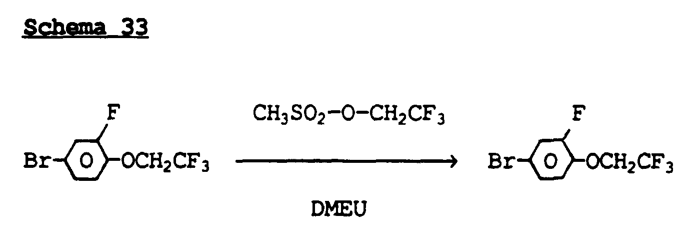

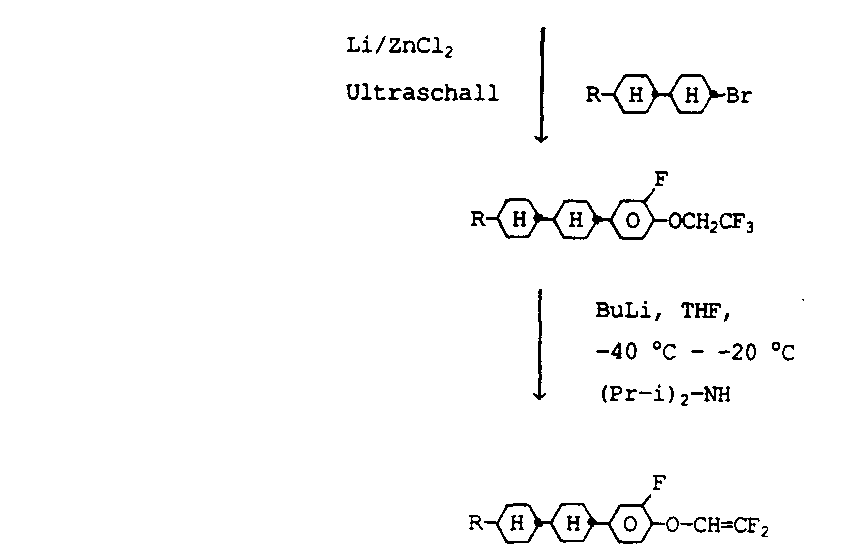

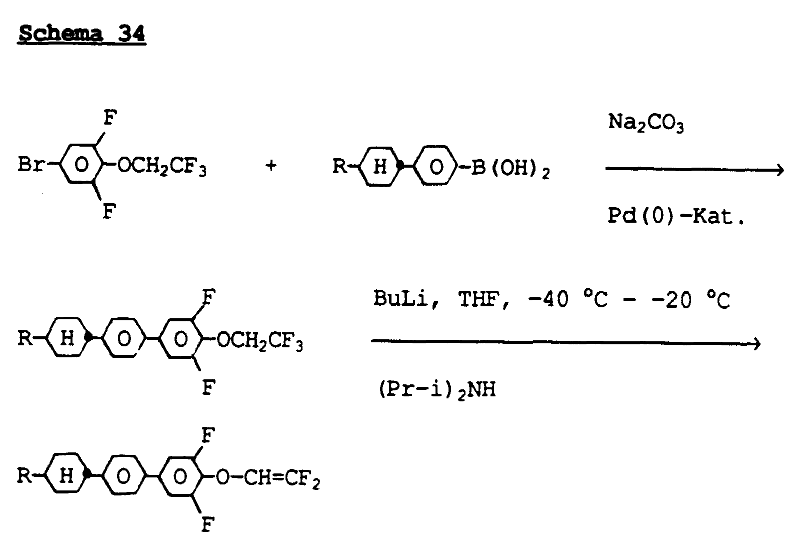

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexan-carbonsäure-phenyl- oder cyclohexyl-ester, Phenyl- oder Cyclohexyl-ester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbidnungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Bio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teil formeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋₍ₖ₊₁₎ FₖCl₁, wobei i 0 oder 1 und k+1 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teil formeln 1b, 2b, 3b, 4b und 5b, in denen R" die Bedeutung -F, -Cl, -NCS, -CF₃, -OCHF₂ oder -OCF₃ hat.

In den Verbindungen der Teil formeln 1b, 2b, 3b, 4b und 5b hat R' die bei den Verbindungen der Teil formeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5 c beschrieben. In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. All diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbidnungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise
- Gruppe A:: 0 bis 90 %, vorzugsweise 20 bis 90 %, insbesondere 30 bis 90 %
- Gruppe B:: 0 bis 80 %, vorzugsweise 10 bis 80 %, insbesondere 10 bis 65 %
- Gruppe C:: 0 bis 80 %, vorzugsweise 5 bis 80 %, insbesondere 5 bis 50 %
wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % an erfindungsgemäßen Verbindungen. Weiterhin bevorzugt sind Medien, enthaltend mehr als 40 %, insbesondere 45 bis 90 % an erfindungsgemäßen Verbindungen. Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder der Orientierung der nematischen Phasen zugesetzt werden.

In der vorliegenden Anmeldung und in den folgenden Beispielen sind die Strukturen der Flüssigkristallverbindungen durch Acronyme angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹ und L²:

| Code für R¹, R², L¹, L², | R¹ | R² | L¹ | L² |
|---|---|---|---|---|
| nm | CₙH₂ₙ₊₁ | CₘH₂ₘ₊₁ | H | H |
| nOm | CₙH₂ₙ₊₁ | OₘH₂ₘ₊₁ | H | H |
| nO.m | OₙH₂ₙ₊₁ | CₘH₂ₘ₊₁ | H | H |
| n | CₙH₂ₙ₊₁ | CN | H | H |
| nN.F | CₙH₂ₙ₊₁ | CN | H | F |
| nF | CₙH₂ₙ₊₁ | F | H | H |
| nOF | OCₙH₂ₙ₊₁ | F | H | H |
| nCl | CₙH₂ₙ₊₁ | Cl | H | H |
| nF.F | CₙH₂ₙ₊₁ | F | H | F |
| nOmFF | CₙH₂ₙ₊₁ | OCₘH₂ₘ₊₁ | F | F |
| nmF | CₙH₂ₙ₊₁ | CₘH₂ₘ₊₁ | F | H |
| nCF₃ | CₙH₂ₙ₊₁ | CF₃ | H | H |
| nOCF₃ | CₙH₂ₙ₊₁ | OCF₃ | H | H |
| nOCF₂ | CₙH₂ₙ₊₁ | OCHF₂ | H | H |
| nS | CₙH₂ₙ₊₁ | NCS | H | H |
| rVsN | CᵣH₂ᵣ₊₁-CH=CH-CₛH₂ₛ- | CN | H | H |
| rEsN | CᵣH₂ᵣ₊₁-O-CₛH₂ₛ- | CN | H | H |
| nNF | CₙH₂ₙ₊₁ | CN | F | H |
| nAm | CₙH₂ₙ₊₁ | COOCₘH₂ₘ₊₁ | H | H |

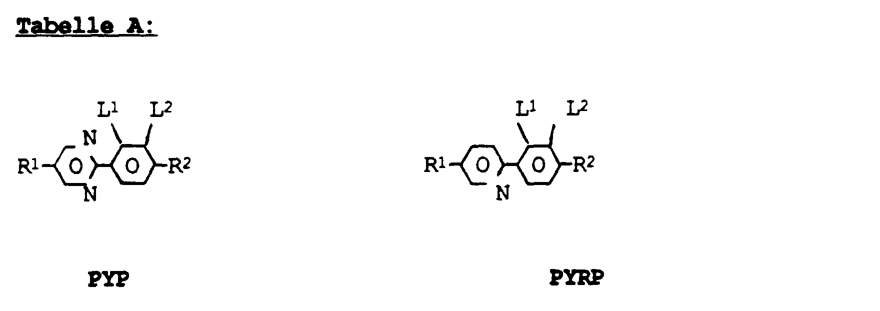

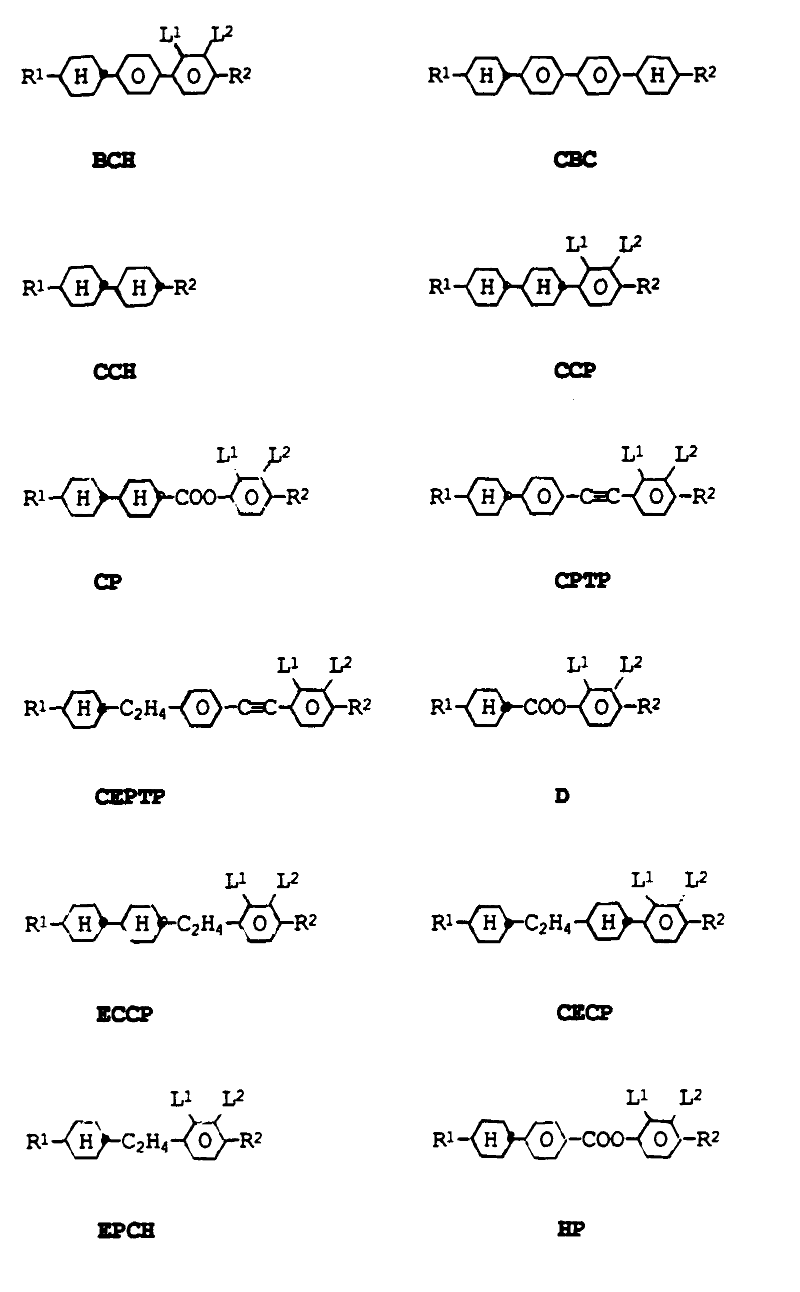

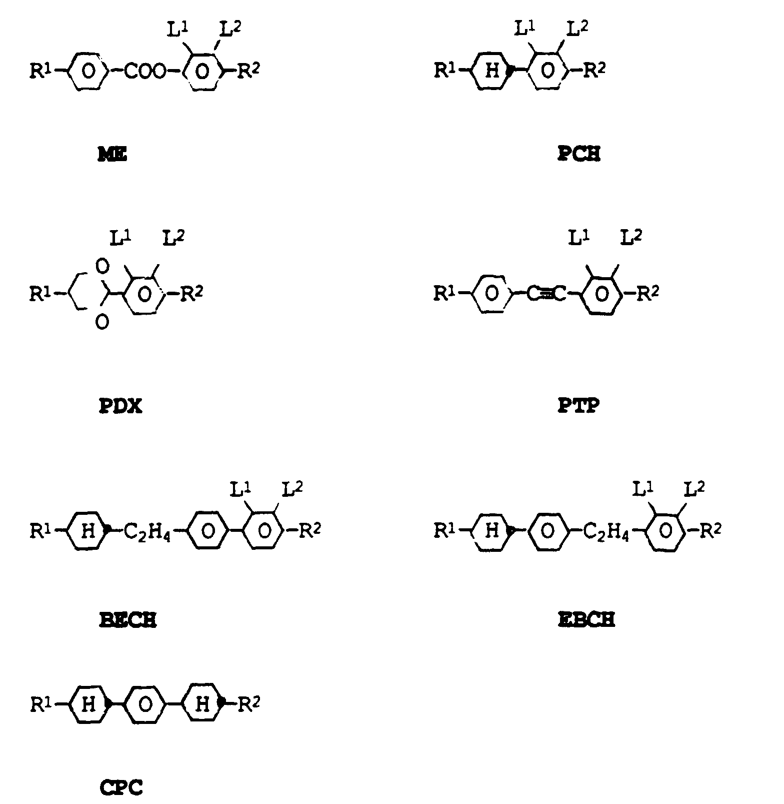

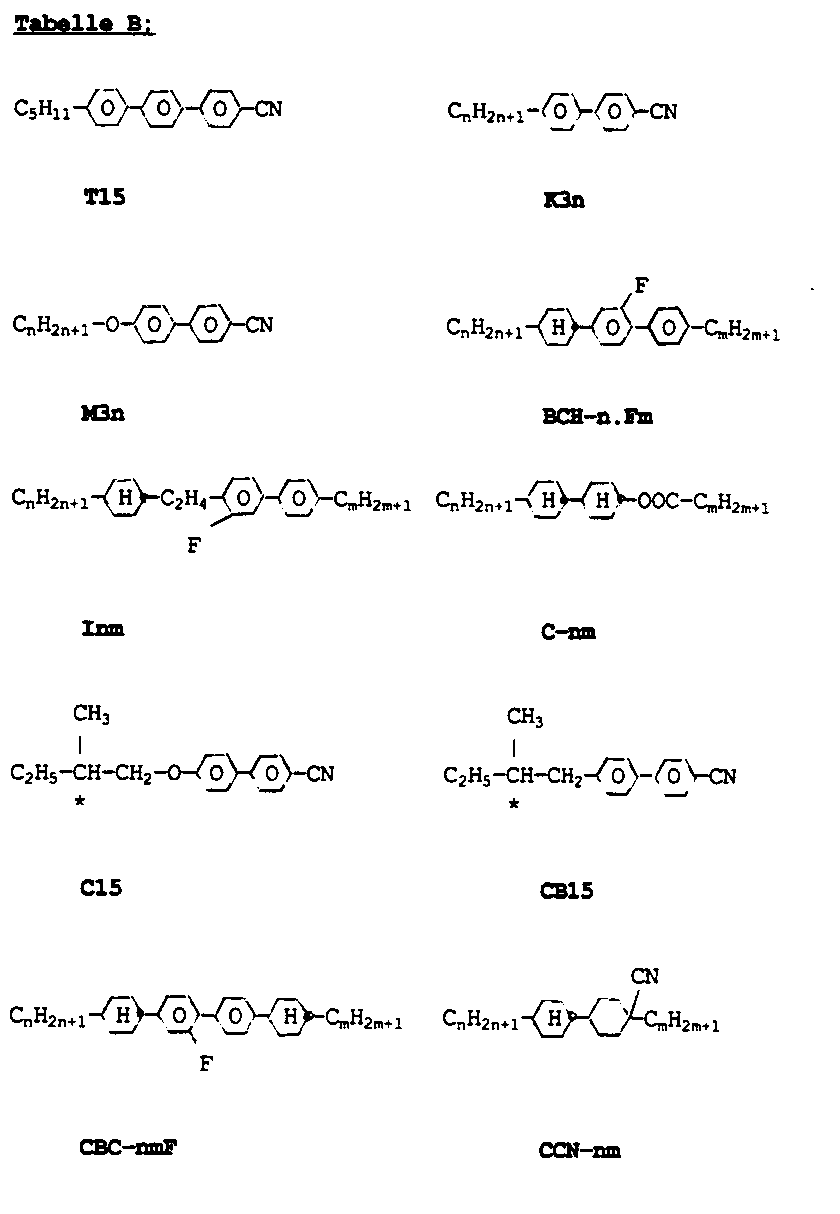

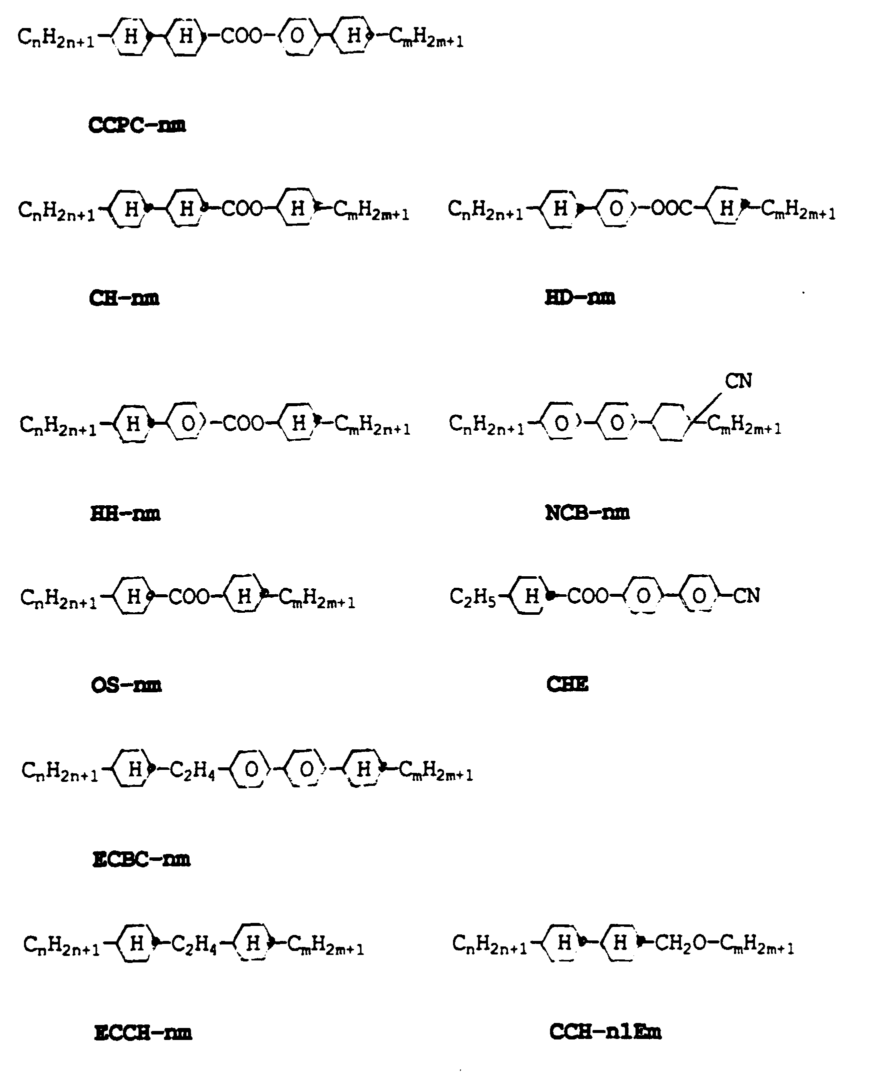

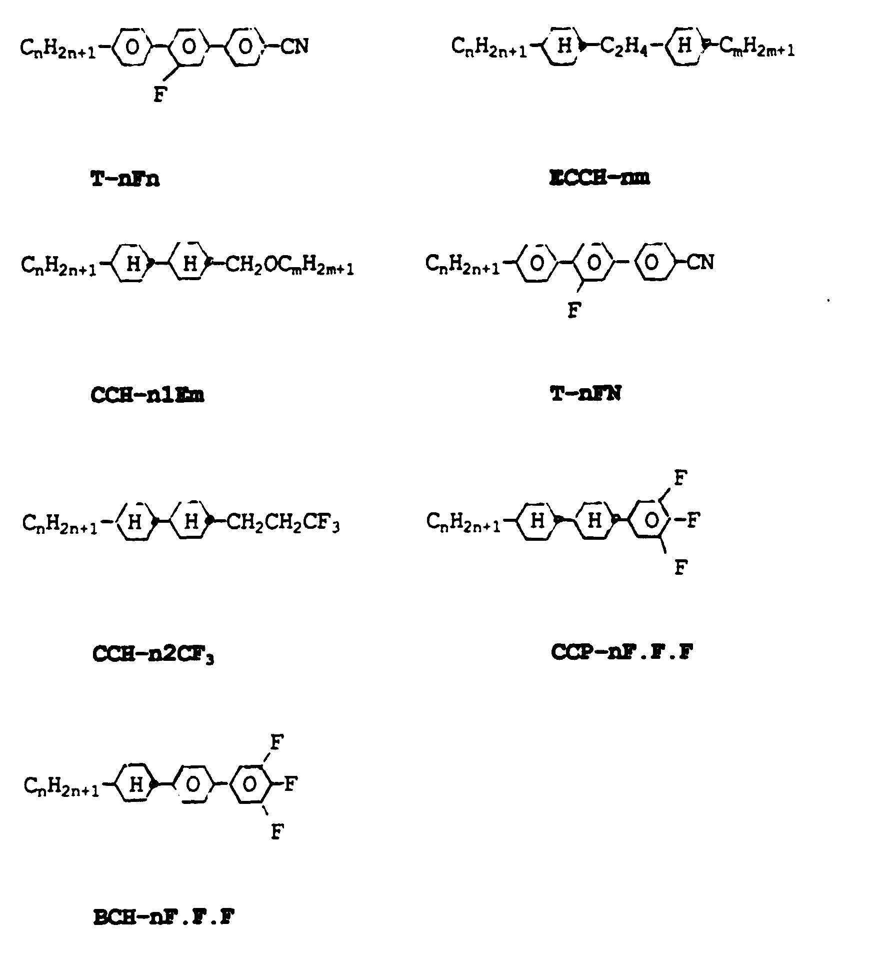

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt, Kp. = Klärpunkt. Ferner bedeuten K = kristalliner Zustand, N = nematische Phase, S = smektische Phase und I = isotrope Phase. Die Angaben zwischen diesen Symbolen stellen die Übergangstemperaturen dar. Δn bedeutet optische Anisotropie (589 nm, 20 °C) und die Viskosität (mm²/sec) wurde bei 20 °C bestimmt.

"Übliche Aufarbeitung" bedeutet: man gibt gegebenenfalls Wasser hinzu, extrahiert mit Dichlormethan, Diethylether oder Toluol, trennt ab, trocknet die organische Phase, dampft ein und reinigt das Produkt durch Destillation unter reduziertem Druck oder Kristallisation und/oder Chromatographie. Folgende Abkürzungen werden verwendet:
- DAST: Diethylaminoschwefeltrifluorid
- DCC: Dicyclohexylcarbodiimid
- DDQ: Dichlordicyanobenzochinon
- DIBALH: Diisobutylaluminiumhydrid
- KOT: Kalium-tertiär-butanolat
- PdCl₂ dppf: 1,1'-Bis(diphenylphosphino)-ferrocen-palladium(II)chlorid
- THF: Tetrahydrofuran
- pTSOH: p-Toluolsulfonsäure
- TMEDA: Tetramethylethylendiamin

### Beispiel 1

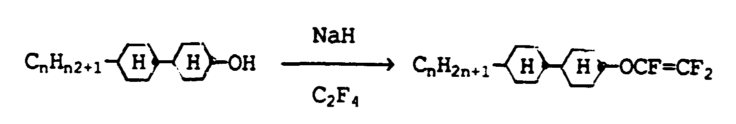

In einem 200 ml fassenden Autoklaven werden unter einer Atmosphäre von trockenem Stickstoff zu einer Lösung von 12,5 g des bekannten 5-Pentyl-trans,trans-bicyclohexanols-1 und 0,5 g Hydrochinon in 50 ml 1,4-Dioxan 1,2 g Natriumhydrid gegeben. Der Autoklav wird geschlossen und sein Inhalt zunächst 2 h bei Raumtemperatur und dann 2 h bei 120 °C mit einem Magnetrührer gerührt. Das Druckgefäß wird daraufhin zunächst mit einem Eisbad und dann mit flüssigem Stickstoff gekühlt. Nach Evakuierung auf einen Druck von < 0,1 mmHg werden unter Ausschluß von Luft 15 g Tetrafluorethen einkondensiert. Es wird 20 h bei 50 bis 60 °C gerührt. Nach Abkühlen auf 0 °C wird das überschüssige Tetrafluorethen abgelassen. Das Reaktionsgemisch wird auf 20 ml Wasser gegeben. Es wird 3mal mit je 100 ml Diethylether extrahiert, die vereinigten organischen Phasen mit Na₂SO₄ getrocknet und eingedampft. Der Rückstand wird bei Raumtemperatur mit 100 ml Toluol ausgerührt, wobei die Hauptmenge des Ausgangsalkohols zurückbleibt. Die Lösung wird eingeengt und einer säulenchromatographischen Trennung (Kieselgel/Petrolether) unterzogen. Umkristallisation der Hauptfraktion aus einem Gemisch gleicher Volumenteile aus Toluol und Ethanol ergibt 5-Pentyl-1-trifluorvinyloxy-trans,trans-bicyclohexyl.

Analog werden die Verbindungen 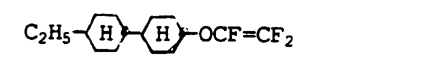 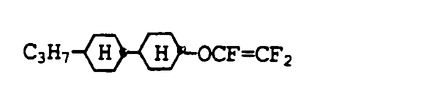 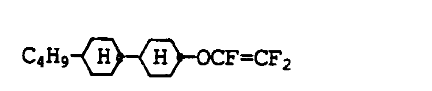 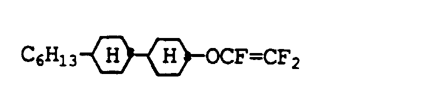 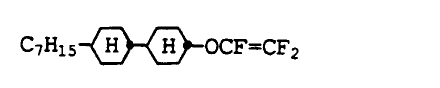 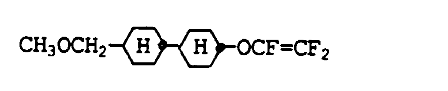 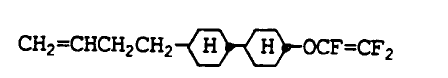 hergestellt.

### Beispiel 2

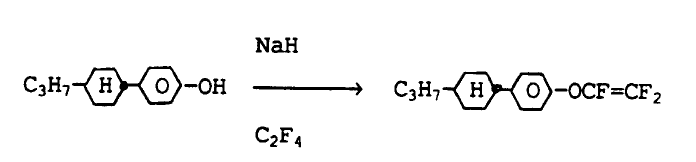

Analog Beispiel 1 wird eine Lösung von 10 g p-(trans-4-n-Propylcyclohexyl)-phenol in einem Gemisch aus 40 ml Benzol und 10 ml Tetrahydrofuran mit Natriumhydrid umgesetzt. Nach Einkondensieren von Tetrafluorethen wird für ca. 20 h auf 140 °C erhitzt und wie in Beispiel 1 beschrieben bearbeitet. Man erhält p-(trans-4-n-Propylcyclohexyl)-trifluorvinyloxy-benzol.

Analog werden hergestellt:
p-(trans-4-Ethylcyclohexyl)-trifluorvinyloxybenzol p-(trans-4-n-Butylcyclohexyl)-trifluorvinyloxybenzol p-(trans-4-n-Pentylcyclohexyl)-trifluorvinyloxybenzol p-(trans-4-n-Hexylcyclohexyl)-trifluorvinyloxybenzol p-(trans-4-n-Heptylcyclohexyl)-trifluorvinyloxybenzol p-(trans-4-n-Ethylcyclohexylcyclohexyl)-trifluorvinyloxybenzol p-(trans-4-n-Propylcyclohexylcyclohexyl)-trifluorvinyloxybenzol p-(trans-4-n-Butylcyclohexylcyclohexyl)-trifluorvinyloxybenzol p-(trans-4-n-Pentylcyclohexylcyclohexyl)-trifluorvinyloxybenzol, K 47 S_{B} 103 I p-(trans-4-n-Hexylcyclohexylcyclohexyl)-trifluorvinyloxybenzol p-(trans-4-n-Heptylcyclohexylcyclohexyl)-trifluorvinyloxybenzol p-(5-Ethylpyrimidin-2-yl)-trifluorvinyloxybenzol p-(5-n-Propylpyrimidin-2-yl)-trifluorvinyloxybenzol p-(5-n-Butylpyrimidin-2-yl)-trifluorvinyloxybenzol p-(5-n-Pentylpyrimidin-2-yl)-trifluorvinyloxybenzol p-(5-n-Hexylpyrimidin-2-yl)-trifluorvinyloxybenzol p-(5-n-Heptylpyrimidin-2-yl)-trifluorvinyloxybenzol

### Beispiel 3

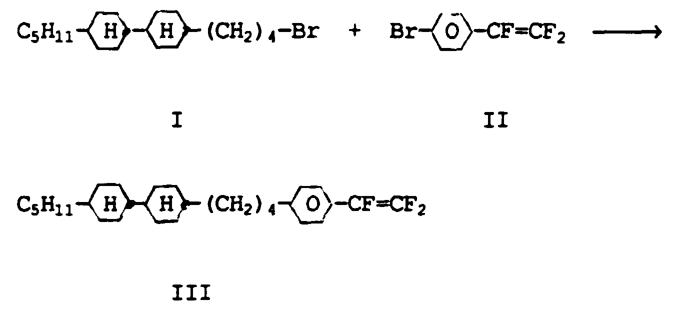

37,1 g I (0,1 Mol) werden in 150 ml eines Lösungsmittelgemisches von THF/Toluol (1 : 4 - Volumenverhältnis) vorgelegt, dann 11,5 g Zinkbromid wasserfrei und darauf 1,4 g Lithiumgranulat hinzugefügt. Das Gemisch wird unter Argon und Rühren 4 Std. zwischen 0 °C und 10 °C mit Ultraschall behandelt, um I in die entsprechende Dialkylzinkverbindung zu überführen. Die zinkorganische Verbindung wird mit 21,1 g II (0,1 Mol) und 1,5 g (2 mol %) 1,1'-Bis(diphenylphosphino)-ferrocen-palladium (II) dichlorid (PdCl₂ dppf) versetzt und nach Entfernung des Ultraschallbades und der Kühlung 24 h bei Zimmertemperatur gerührt. Es wird mit 100 ml gesättigter NH₄Cl-Lösung unter Rühren zersetzt, die organische Phase abgetrennt, die wäßrige Phase 2 x mit Toluol extrahiert. Die vereinigten organischen Extrakte liefern nach dem Trocknen, Einengen und Chromatografieren über Kieselgel mit Hexan III. (I ist herstellbar durch Kettenverlängerung von 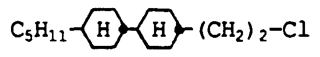 mittels Malonester.) Mit II lassen sich analog I die nachfolgend aufgeführten Alkylbromide umsetzen: 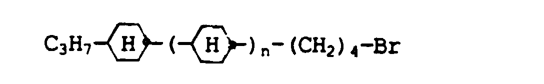 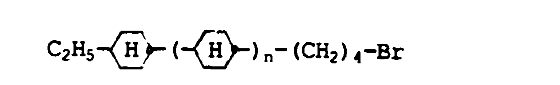 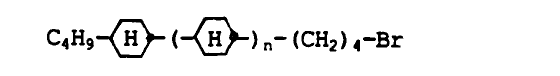 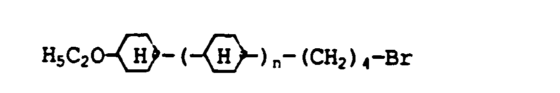 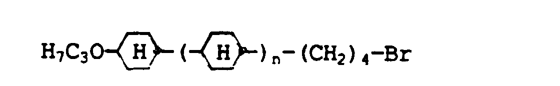 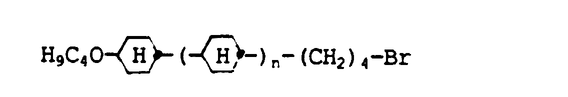 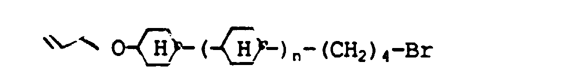 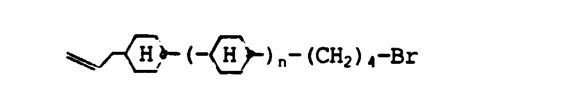 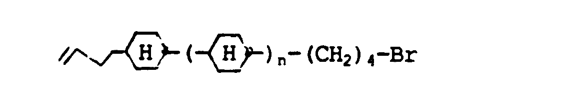 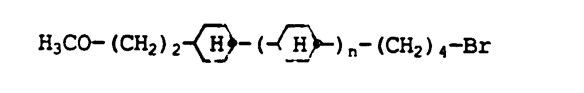 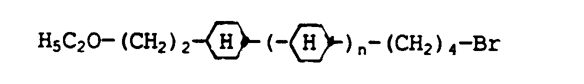 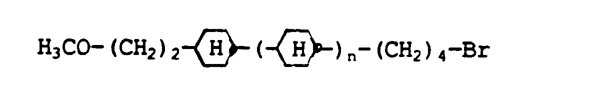 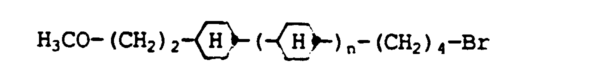 n = 0 und 1 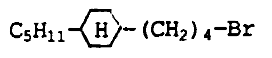 auch

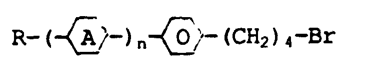

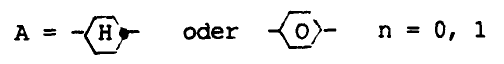

### Beispiel 4

### 4-(4-Heptyl-cyclohexyl)-1-(1,2,3-trifluorvinyl)-benzol

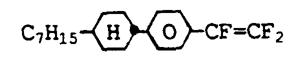

Zu 16,87 g (50 mmol) 1-Brom-4(4-heptylcyclohexyl)benzol in 300 ml Diethylether/THF 1 : 1 werden bei -70° 36 ml einer 15%igen Lösung von Butyllithium in Hexan zugetropft. Nach 1 Std. Rühren wird, weiterhin bei -70°, Tetrafluoroethylen in starkem Strom durchgeleitet (insgesamt ca. 20 g). Nach Erwärmen auf Raumtemperatur wird bei 100 ml 1N-Salzsäure angesäuert; die organische Phase wird abgetrennt und im Vakuum eingedampft. Der Rückstand wird im Vakuum (ca. 0,1 Torr) bei ca. 180° destilliert.

Analog erhält man

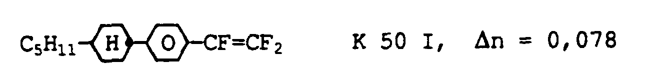

### Beispiel 5

### 4-[4-(4-Propyl-cyclohexyl)cyclohexyl]-1-(1,2,2-trifluorvinyl)benzol

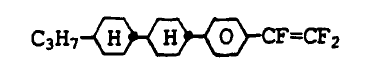

Eine Mischung aus 5,0 g (77 mmol) Zinkpulver, 50 ml THF und 13,5 g (65 mmol) Trifluoriodethylen wird über Nacht, anfänglich unter Eiskühlung, später bei Raumtemperatur gerührt. Dann werden 20,5 g (50 mmol) 1-Jod-4-[4-(4-propylcyclohexyl)cyclohexyl]-benzol und 1,1 g Tetrakis-(triphenylphosphin)-palladium gegeben.

Nach Rückflußkochen über Nacht wird eingedampft, der Rückstand in Toluol aufgenommen und über eine Kieselgelsäule mit Toluol als Eluens chromatographiert. Das Eluat wird im Vakuum eingedampft, der Rückstand aus Hexan/Essigester umkristallisiert.

### Analog erhält man

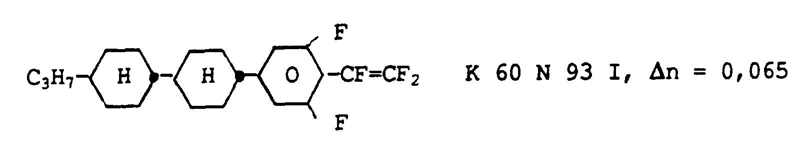

### Beispiel 6

### 4(4-Pentyl-cyclohexyl)cyclohexyl-4'-(1,2,2-trifluorvinyl)biphenyl

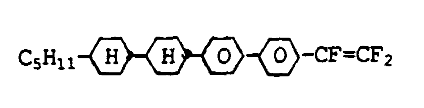

Zu einer Lösung von 5,05 g (50 mmol) Diisopropylamin in 25 ml THF werden bei -20° 36 ml (58 mmol) einer 15%igen Lösung von Butyllithium in Hexan zugetropft. Nach 1/2 Std. Rühren bei dieser Temperatur werden bei -71° eine Lösung von 18,8 g 4(4-Pentylcyclohexyl)cyclohexyl-4'-(1,1,2,2-tetrafluorethyl)-biphenyl (DOS 40 02 374) in 100 ml THF zugetropft. Man läßt langsam auf Raumtemperatur erwärmen und kocht dann noch 10 min am Rückfluß. Nach Abkühlen wird mit 1N-Salzsäure angsäuert; die organische Phase wird abgetrennt, mit 2 x 50 ml gewaschen und dann im Vakuum eingedampft. Der Rückstand wird über eine Kieselgelsäule mit Toluol als Laufmittel chromatographiert, das Eluat wird im Vakuum eingedampft, der Rückstand aus Cyclohexan/Essigester umkristallisiert.

### Beispiel 7

### 4-(4-Pentyl-cyclohexyl)cyclohexyl)-(1,2,2,-trifluorvinyl)ether

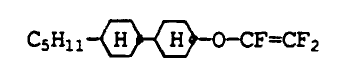

Mit 16,0 g (50 mmol) 4(4-Pentyl-cyclohexyl)cyclohexyl(1,1,2,2-tetrafluorethyl)ether wird analog verfahren wie in Beispiel 6 beschrieben (Aufreinigung durch Tieftemperatur-Kristallisation aus Pentan).

### Analog werden die folgenden Verbindungen der Formel

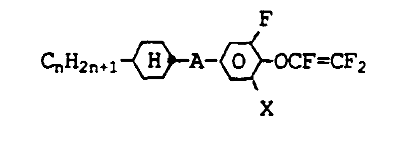

hergestellt:

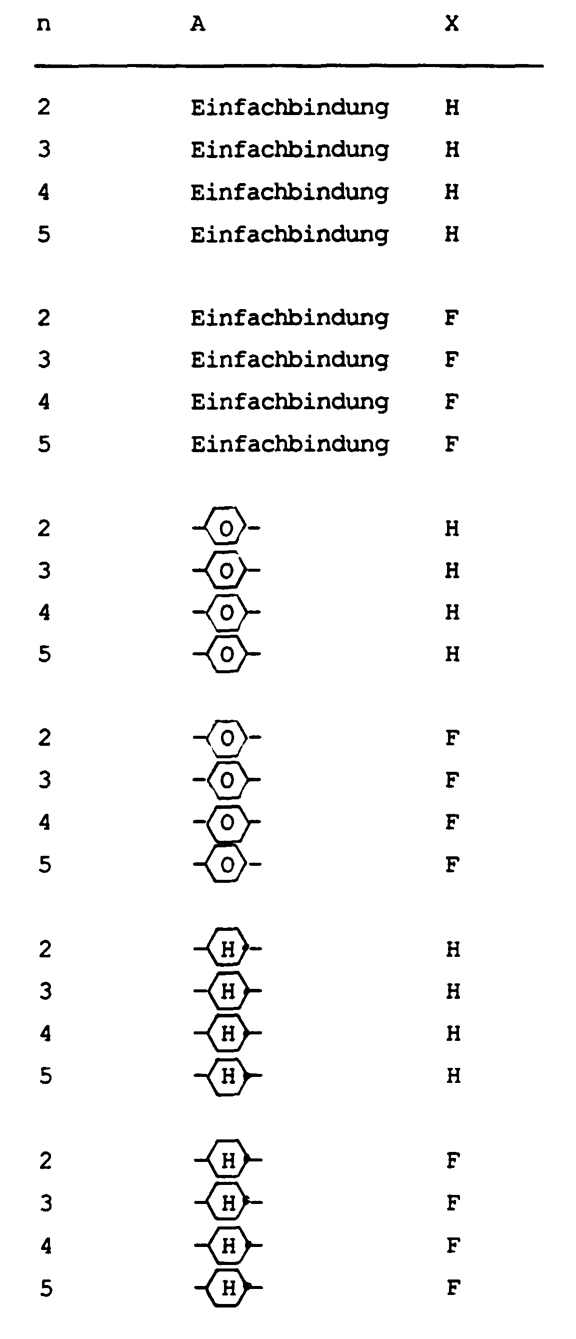

### Beispiel 8

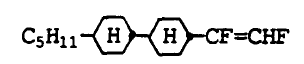

### Schritt 8.1

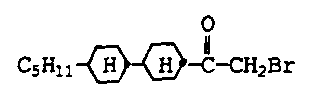

Zu 0,5 mol n-Pentylcyclohexylcyclohexylmethylketon gelöst in 300 ml abs. Methanol werden bei 0 bis 5 °C 0,5 mol Brom langsam zugetropft. Nach ca. 1 h wird die Lösung mit 150 ml Wasser versetzt und anschließend über Nacht gerührt. Nach Zugabe von weiteren 450 ml Wasser wird wie üblich aufgearbeitet.

### Schritt 8.2

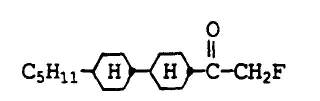

0,45 mmol 18-Krone-6 in 25 ml Toluol werden mit 0,05 mol wasserfreiem Kaliumfluorid versetzt. Das Gemisch wird 15 Minuten bei Raumtemperatur gerührt, mit 0,025 mol Bromketon aus Schritt 8.1 versetzt und 24 h bei 80 °C gerührt. Anschließend wird wie üblich aufgearbeitet.

### Schritt 8.3

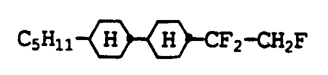

Zu 26 mmol DAST werden unter Stickstoffatmosphäre 17 mmol Fluormethylketon (Schritt 8.2) in 10 ml abs. Toluol zugegeben. Anschließend wird das Gemisch 17 h bei 50 °C gerührt. Dann wird auf 0 °C abgekühlt, vorsichtig mit Wasser versetzt und mit Natriumhydrogencarbonat-Lösung neutral gewaschen. Anschließend wird wie üblich aufgearbeitet.

### Schritt 8.4

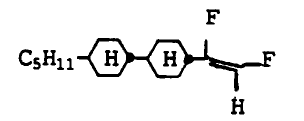

Zu einer Lösung von 50 mmol Kalium-tert.butylat in 20 ml tert.-Butanol gibt man 25 mmol der Trifluorverbindung (Schritt 8.3) und erwärmt 18 h auf 100 °C. Nach dem Abkühlen gießt man auf Wasser und arbeitet wie gewöhnlich auf. K 25 N 73,8 I; Δn = +0,039

Analog werden die folgenden Verbindungen der Formel 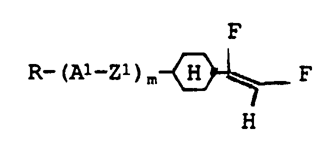 hergestellt:

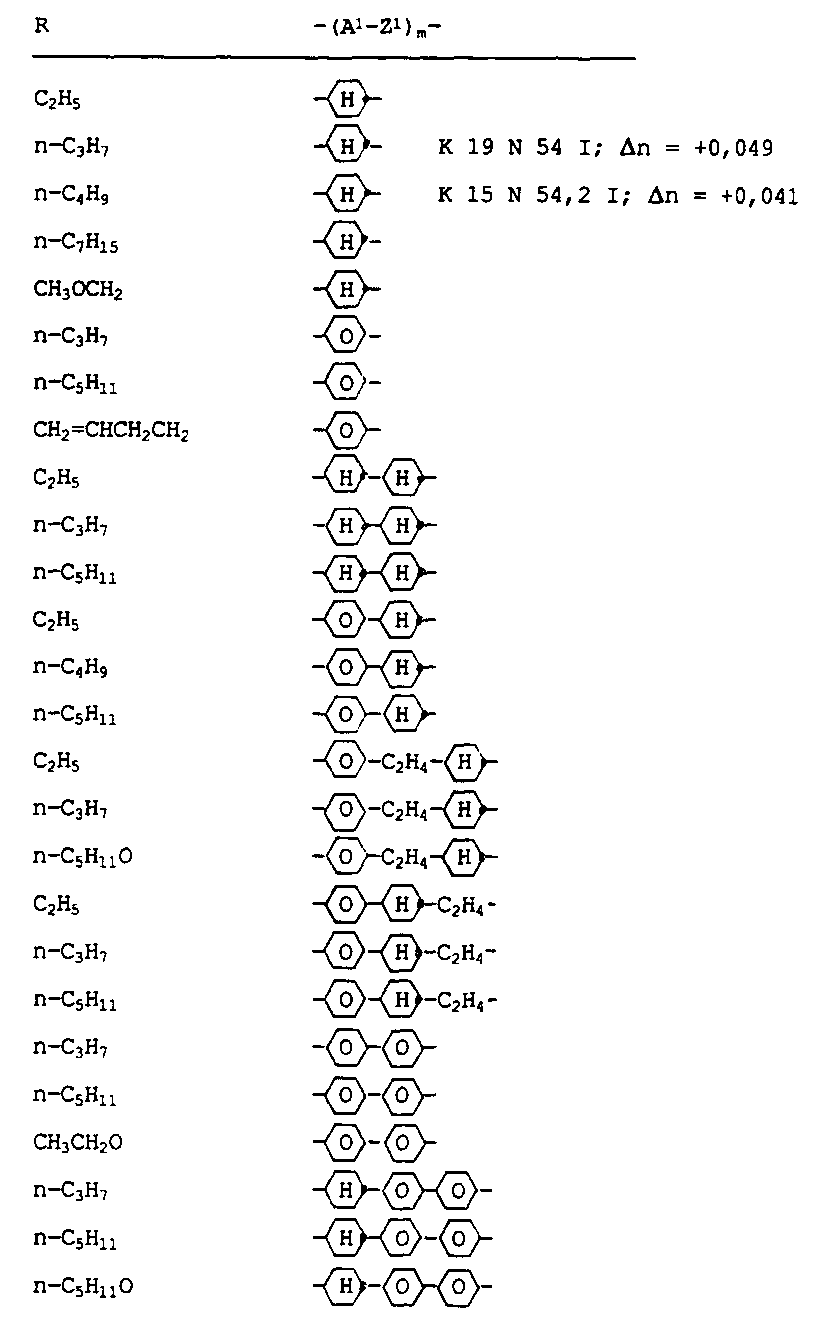

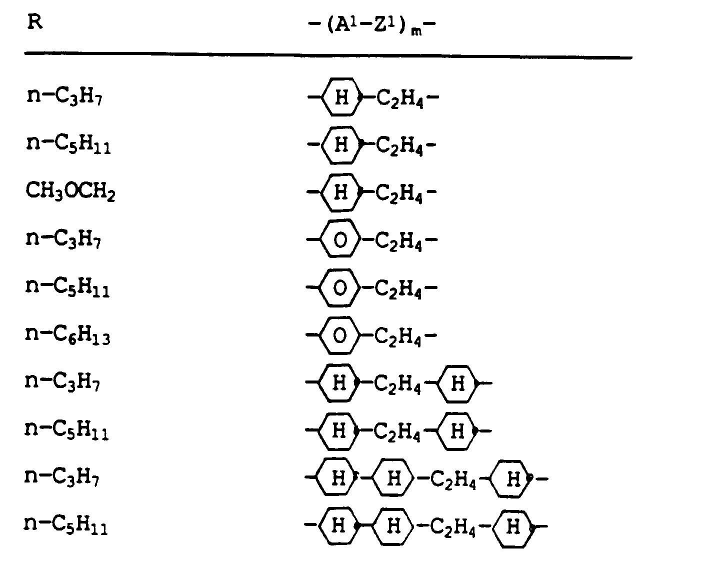

### Beispiel 9

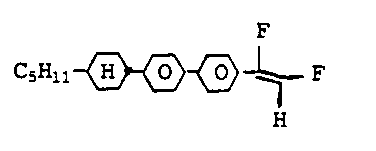

Zu 0,6 mol gepulvertem Aluminiumchlorid und 200 ml Dichlormethan werden unter Rühren bei 0 °C 0,52 mol Fluoressigsäurechlorid zugetropft. Anschließend werden zu dem Gemisch 0,5 mol n-Pentylcyclohexylbiphenyl unter Kühlung zugegeben. Dann wird eine weitere Stunde gerührt und über Nacht stehengelassen. Es wird auf Eis gegossen und wie üblich aufgearbeitet. Das erhaltene Fluorketon wird analog 8.3 und 8.4 zunächst mit DAST, dann mit Kalium-tert.-butylat zur Difluorvinylverbindung umgesetzt.

Analog werden die folgenden Verbindungen der Formel 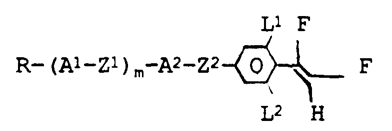 hergestellt.

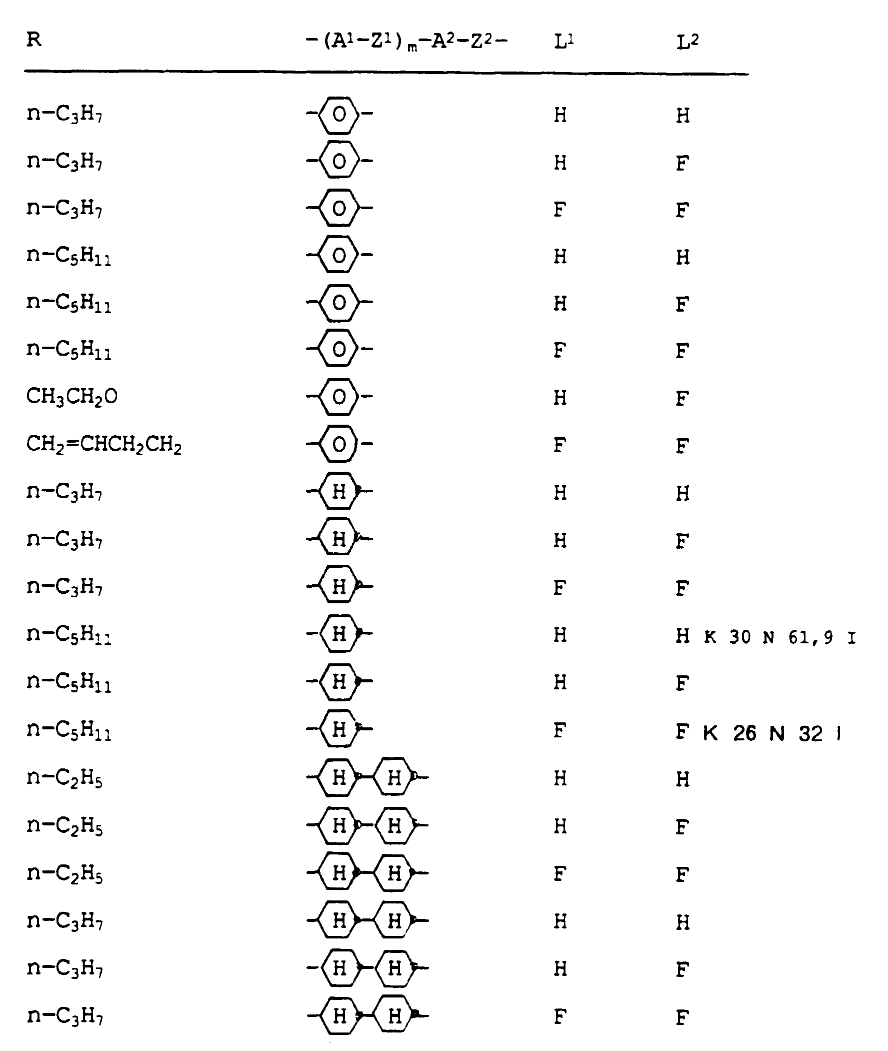

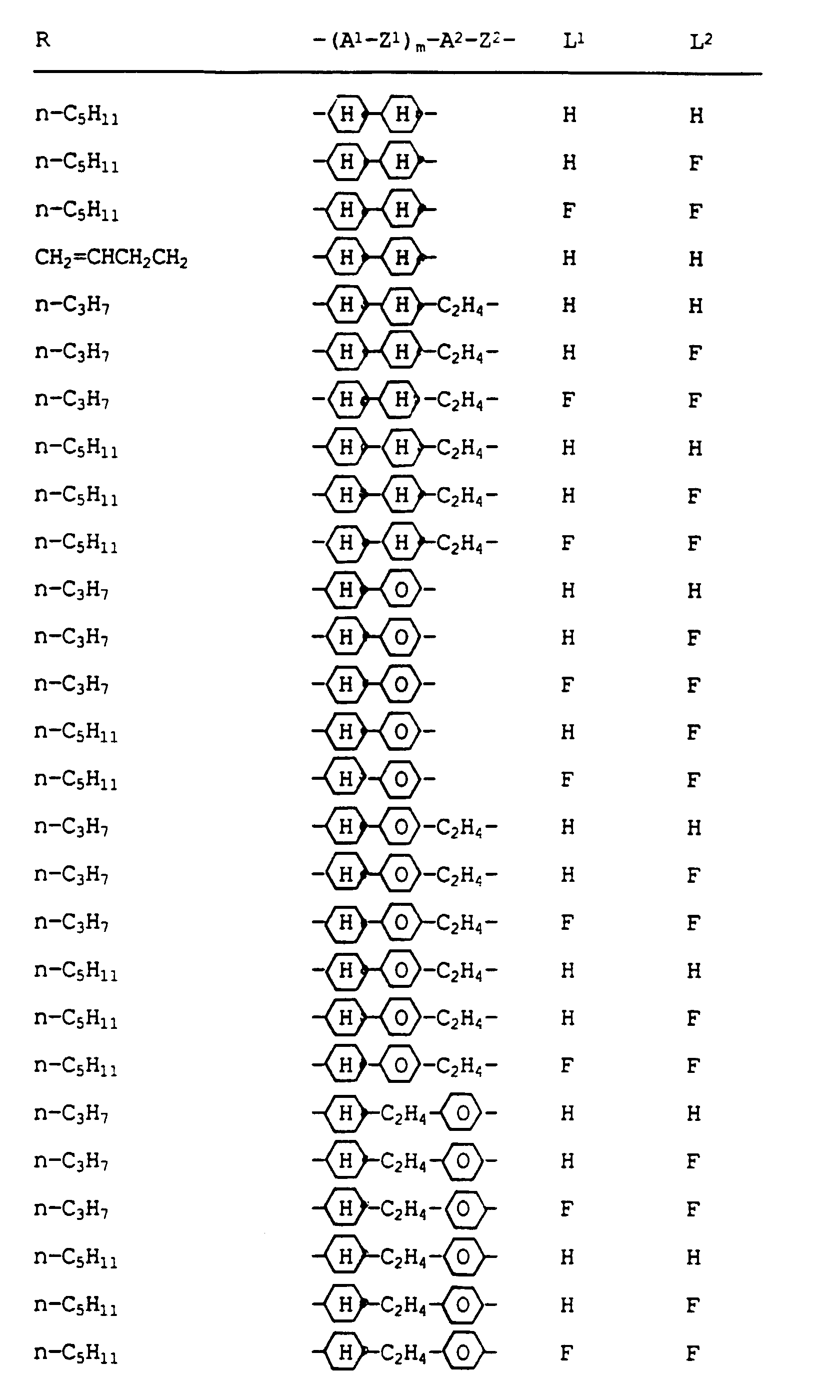

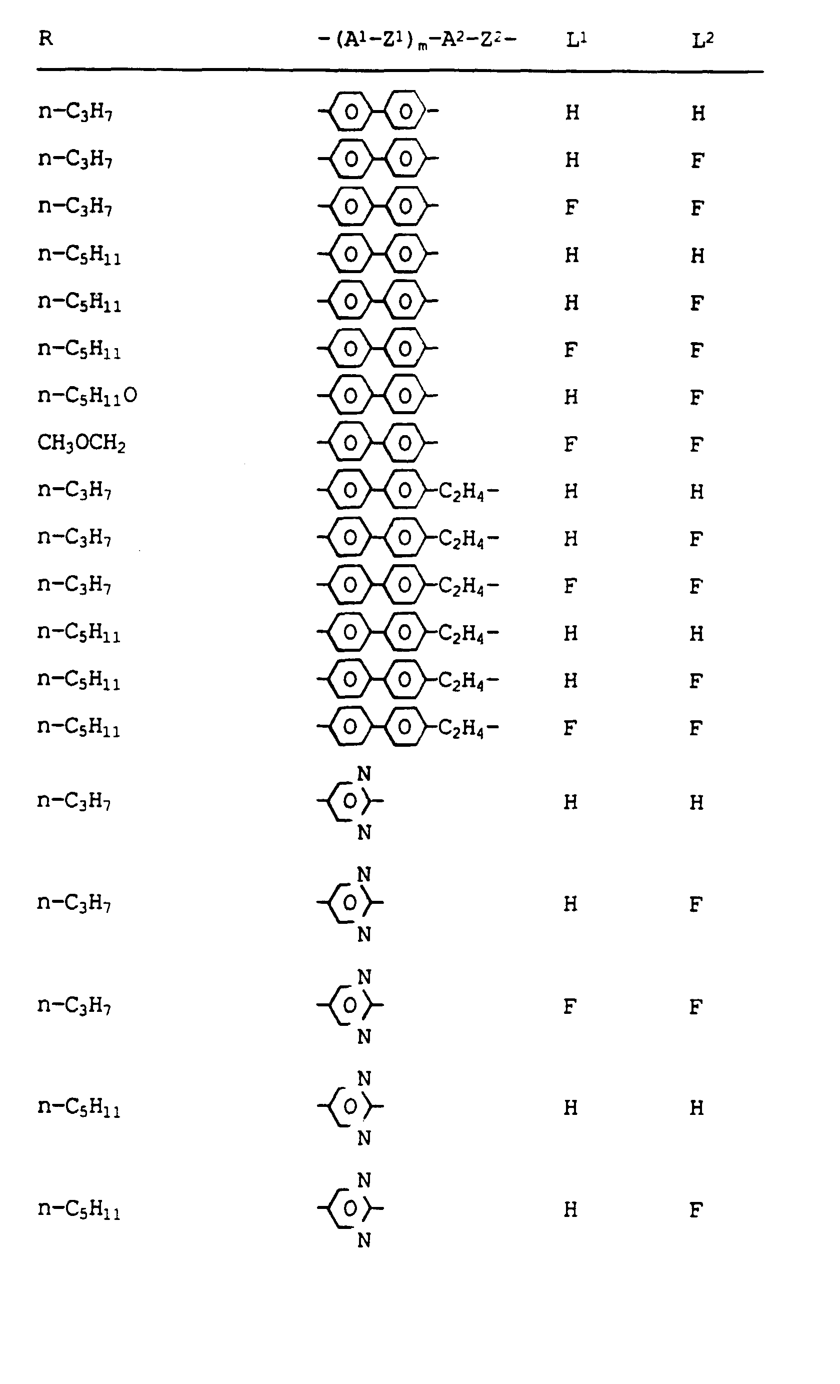

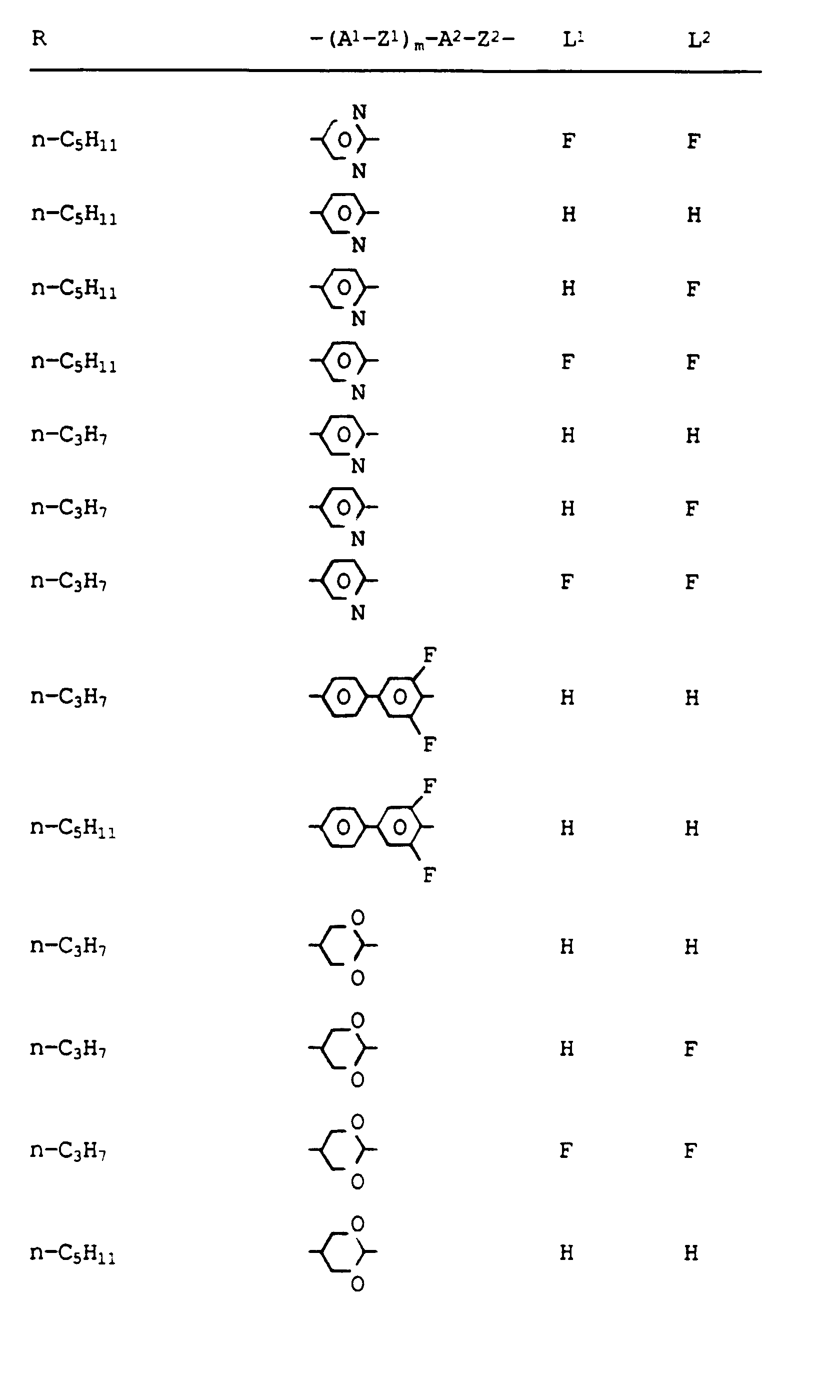

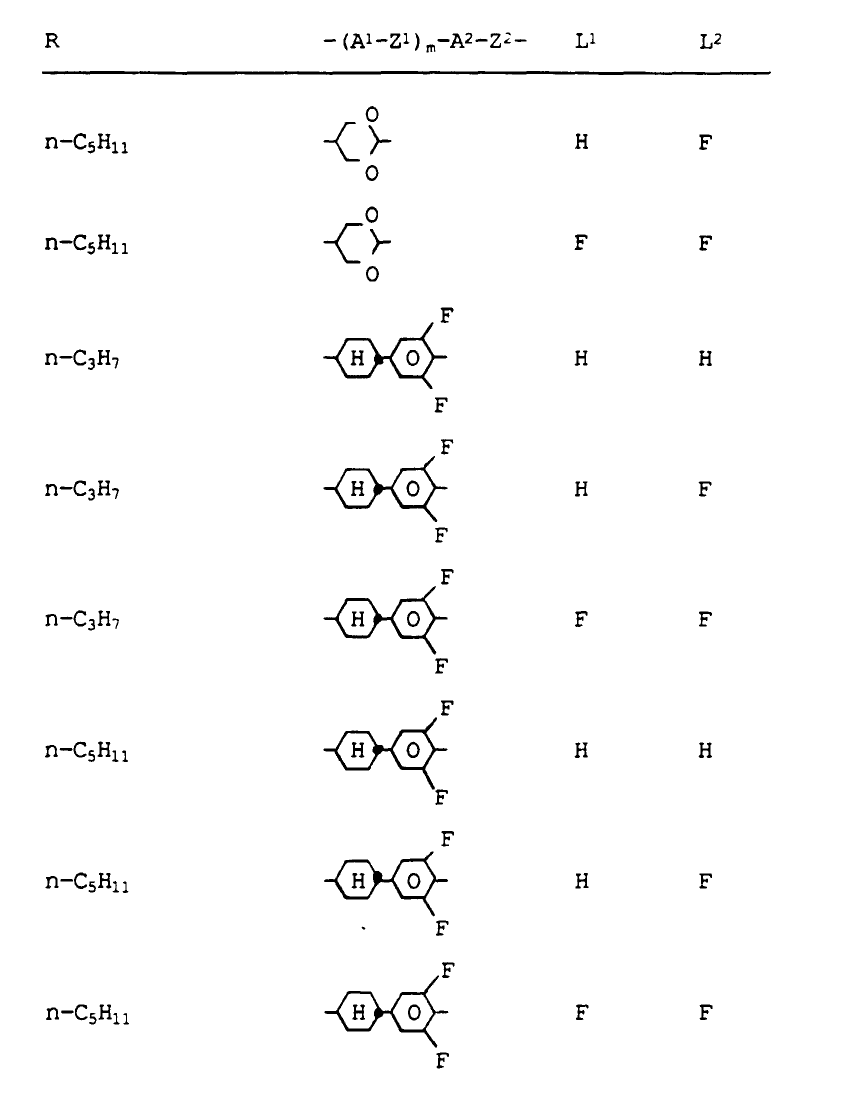

**Beispiel 10**

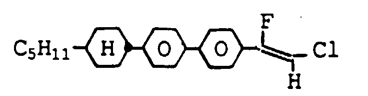

Analog Beispiel 9 werden 0,5 mol n-Pentylcyclohexylbiphenyl zunächst mit 0,52 mol Chloressigsäurechlorid, dann mit DAST und Kalium-tert.-butylat behandelt. K 44 N 93,8 I

Analog werden die folgenden Verbindungen der Formel 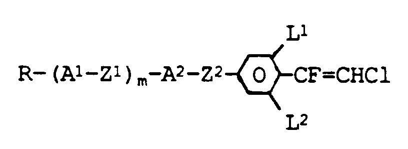 hergestellt.

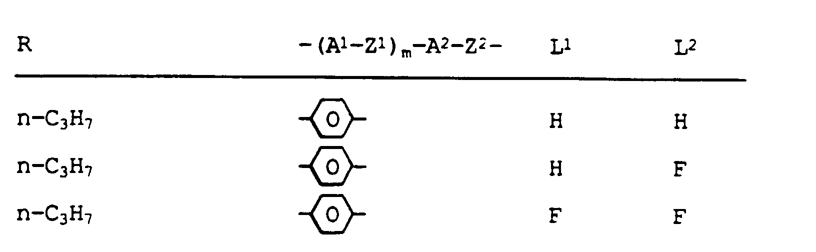

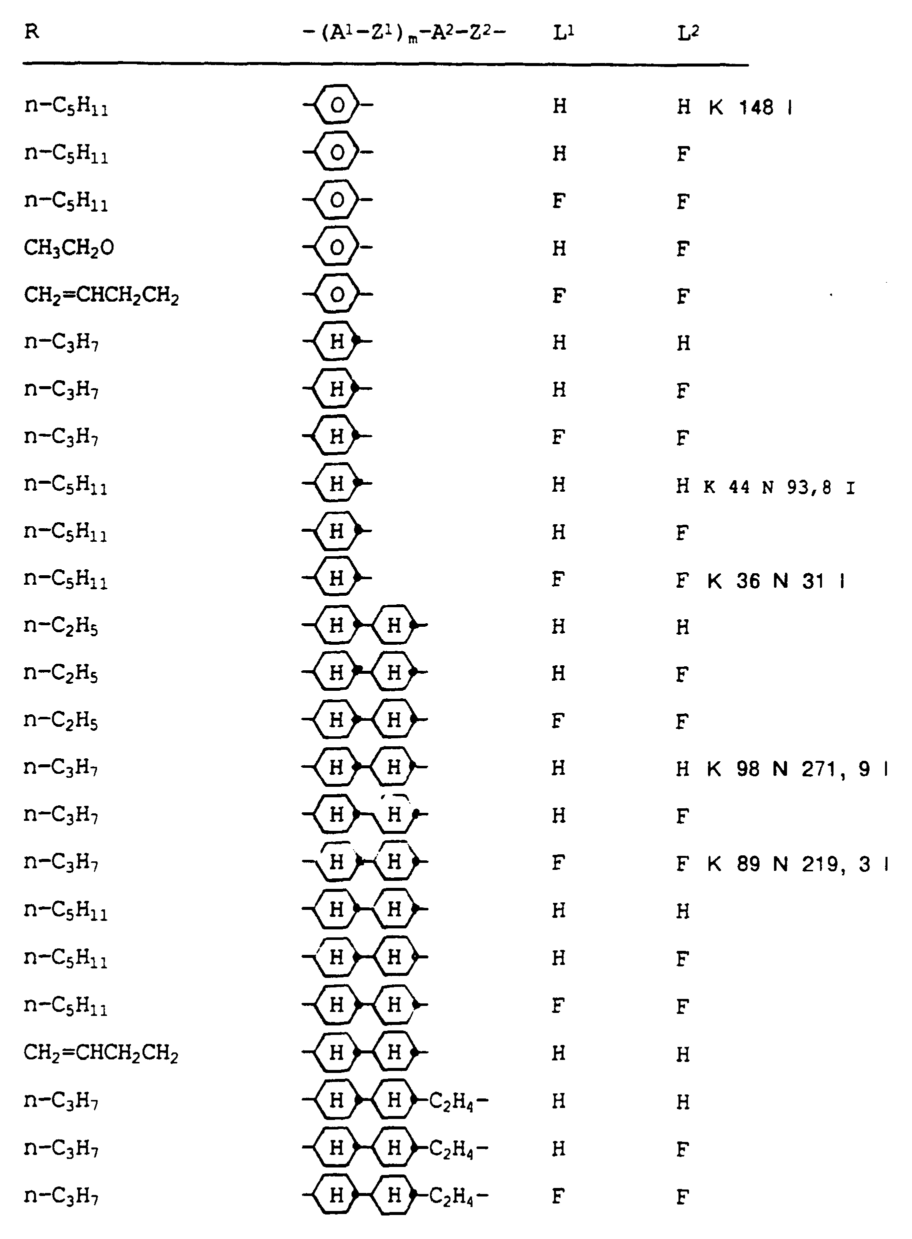

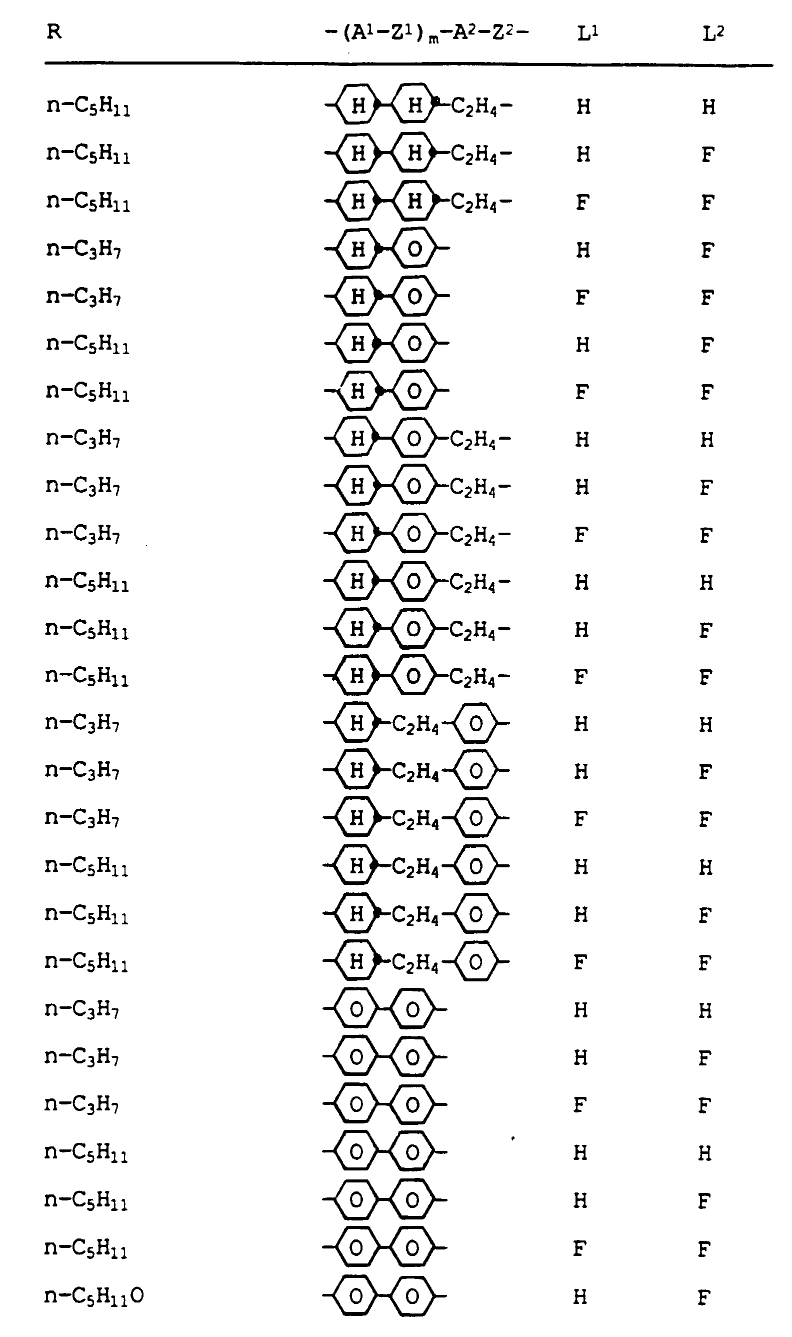

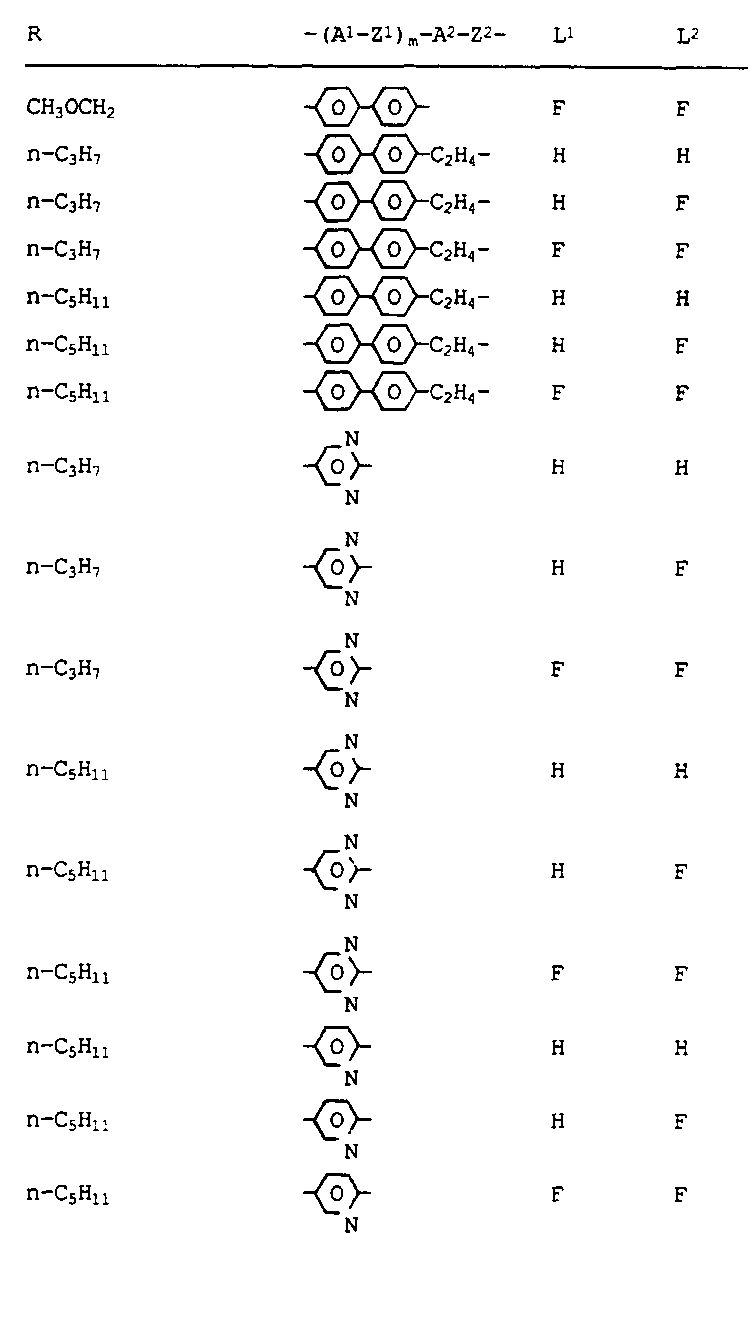

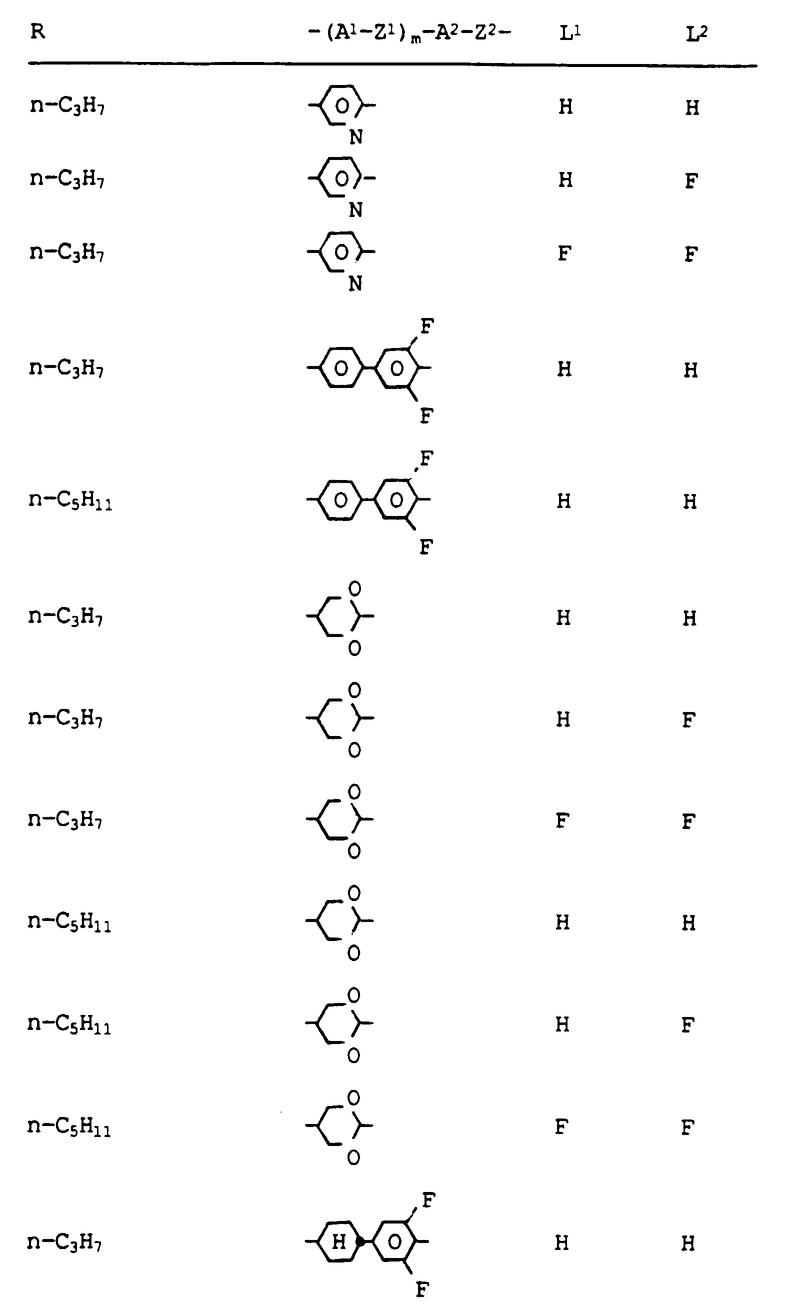

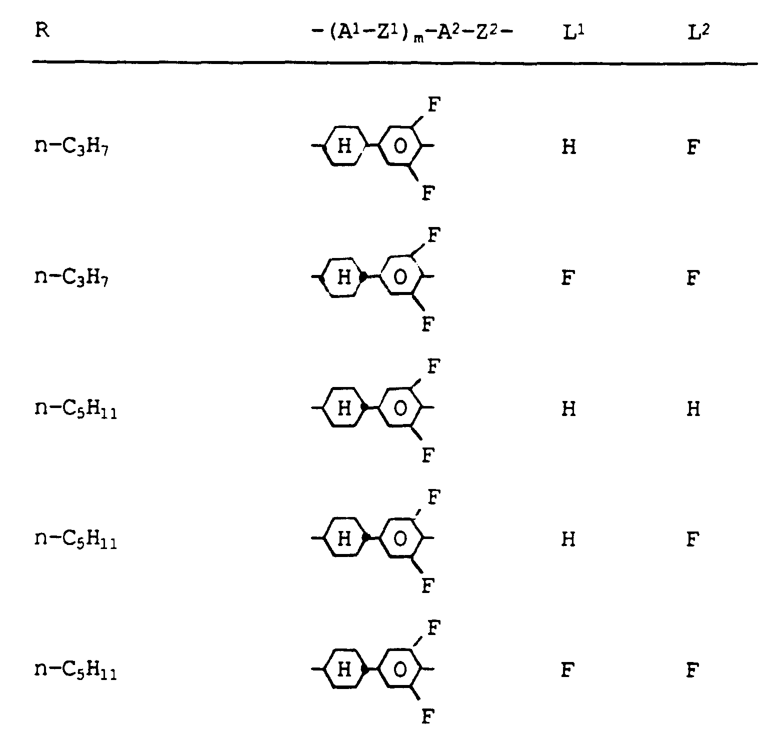

### Beispiel 11

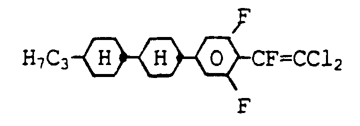

Zu einem Gemisch aus 76 mmol n-Propylcyclohexylcyclohexyl-2,6-difluorbenzol und 100 ml Ether wird bei -78 °C eine Lösung von 54 ml BuLi in Ether (89 mmol) gegeben. Man rührt 0,5 h nach, kühlt dann auf -90 °C und gibt 0,147 mol Dichlordifluorethylen zum Reaktionsgemisch. Man rührt 1 h bei -78 °C nach und läßt dann langsam auf -30 °C erwärmen. Anschließend wird wie üblich aufgearbeitet.

Analog werden die folgenden Verbindungen der Formel 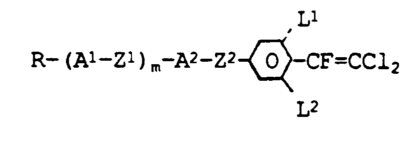 hergestellt:

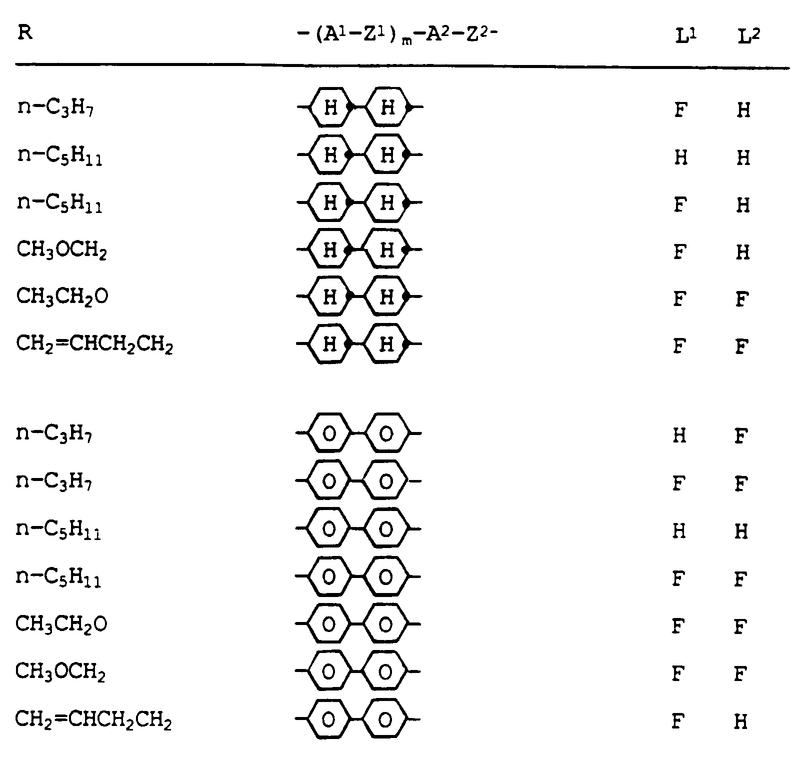

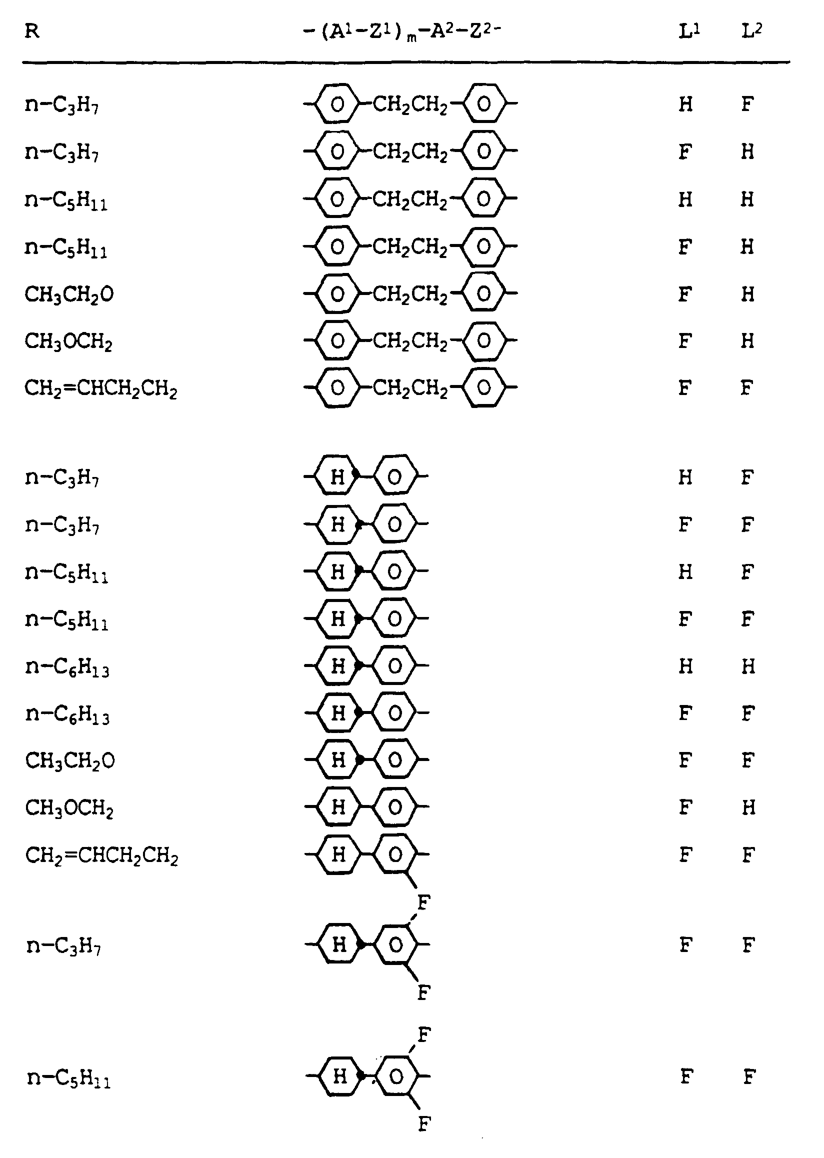

### Beispiel 12

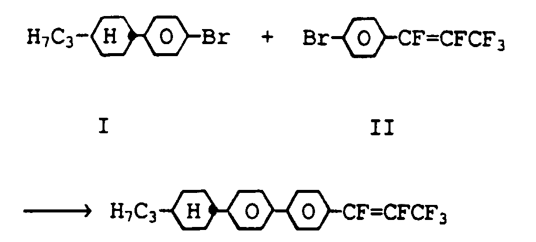

Zu 0,48 mol 4-(4-trans-Propylcyclohexyl)-1-brombenzol in 800 ml THF werden bei -75 °C 0,48 mol Butyllithium (15%ig in Hexan) innerhalb von 45 min zugetropft. Anschließend wird zunächst 0,5 h gerührt, danach eine gekühlte Lösung von 0,24 mol Zinkbromid in 200 ml THF zugetropft und weitere 0,5 h bei -75 °C gerührt. Nach Zugabe von 0,4 mol II und 7 g PdCl₂ dppf bei -75 °C werden weitere 72 h bei Raumtemperatur gerührt. Nach Zugabe von 500 ml gesättigter Ammoniumchlorid-Lösung wird die organische Phase abgetrennt und wie üblich aufgearbeitet.

Analog werden die folgenden Verbindungen der Formel I 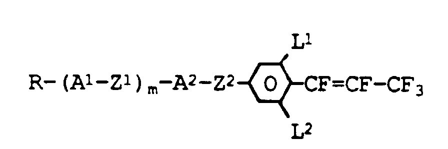 hergestellt:

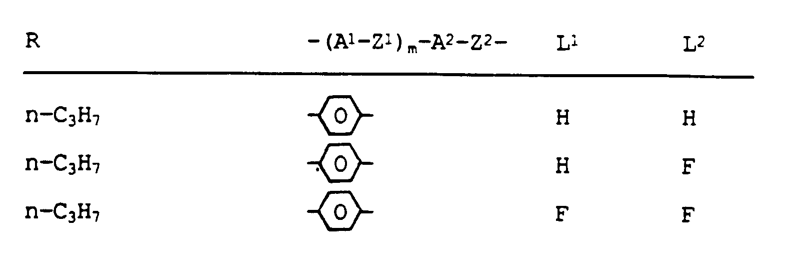

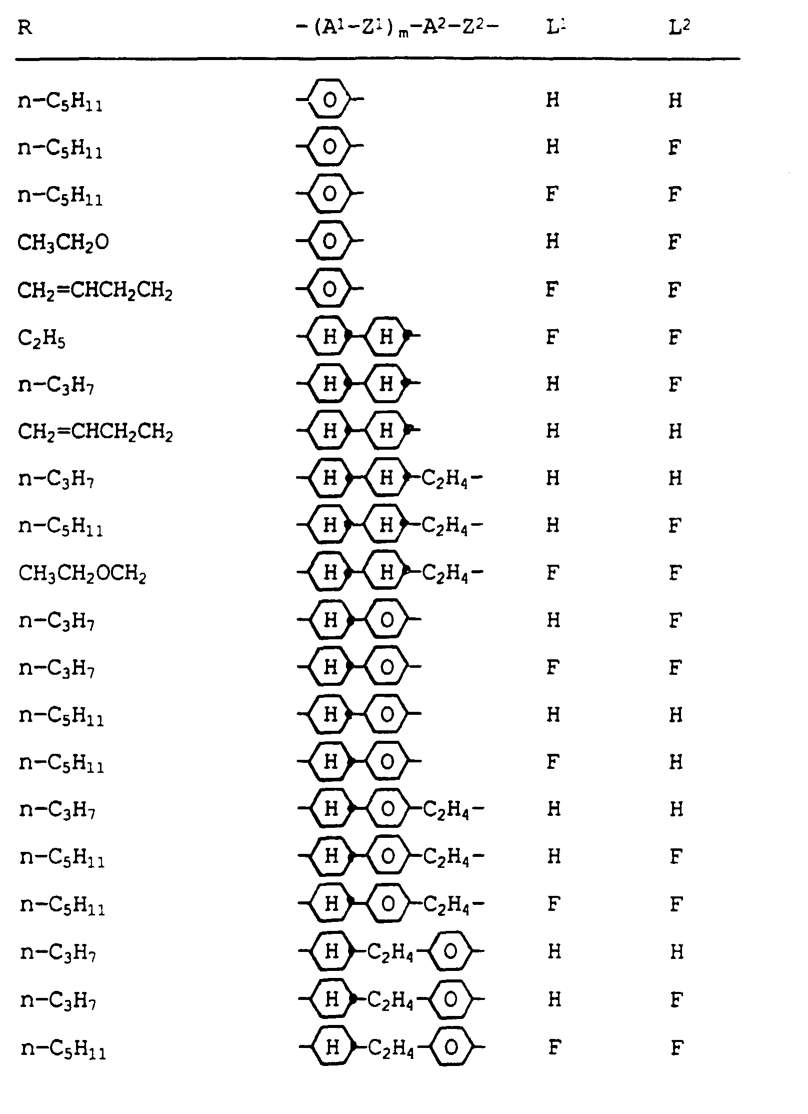

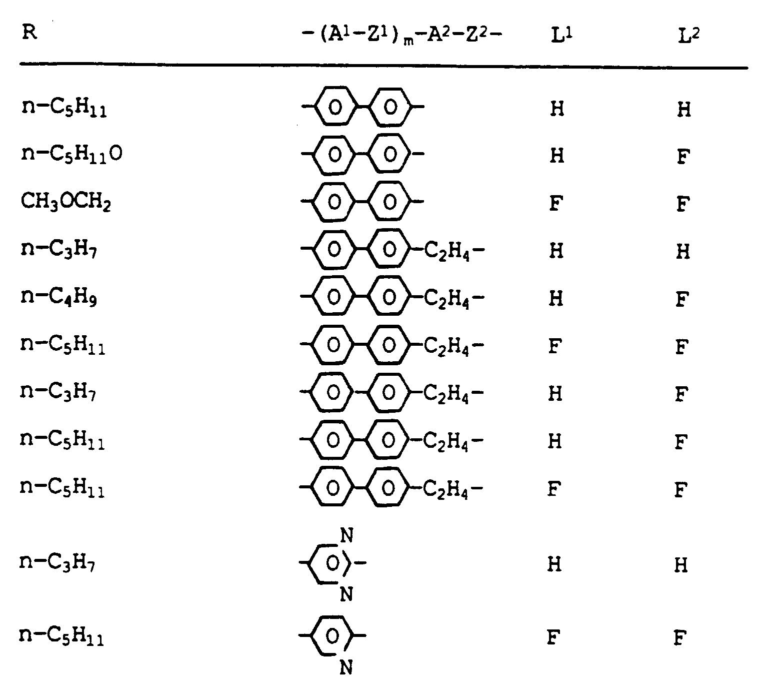

### Beispiel 13

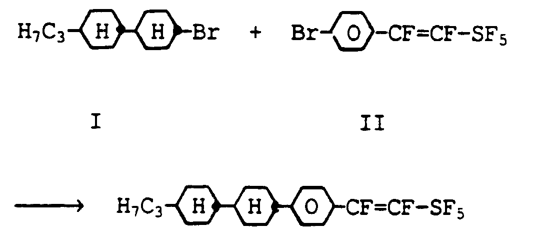

Zu 1,12 mol I in 1500 ml THF: Toluol = 1:4 werden bei 0 °C 0,56 mol Zinkbromid und danach 2,24 mol Lithium zugegeben. Anschließend wird unter Rühren bei 0-10 °C das Gemisch 4 h mit Ultraschall behandelt. Nach Zugabe von 0,56 mol II und 12,3 g PdCl₂-dppf wird die Kühlung entfernt und weitere 72 h bei Raumtemperatur gerührt. Das Gemisch wird in 1000 ml Ammoniumchlorid-Lösung gegossen und 15 min gerührt. Die organische Phase wird abgetrennt und wie üblich aufgearbeitet.

Analog werden die folgenden Verbindungen der Formel 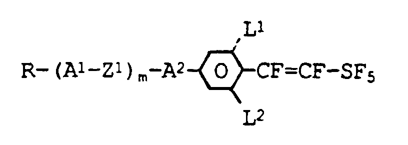 hergestellt:

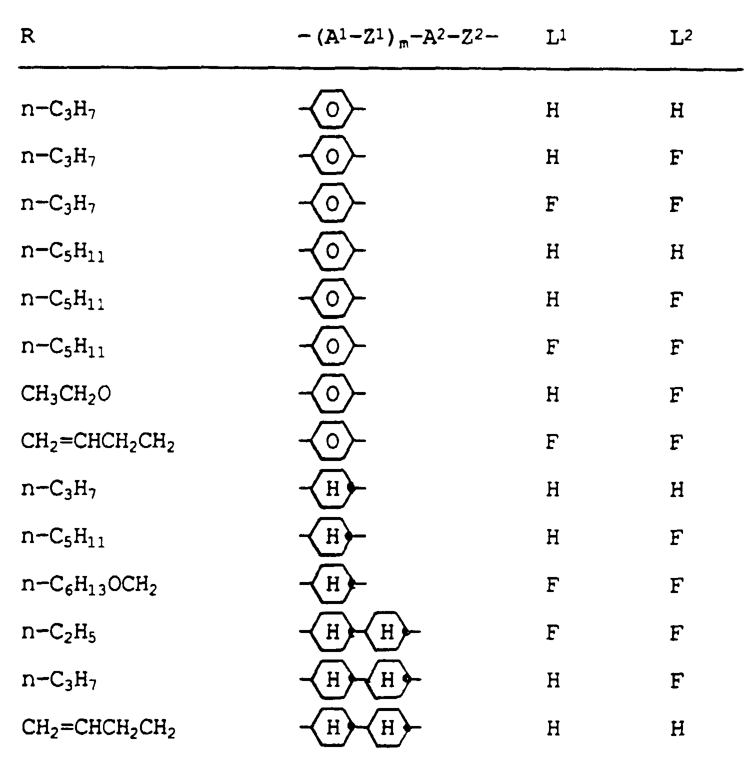

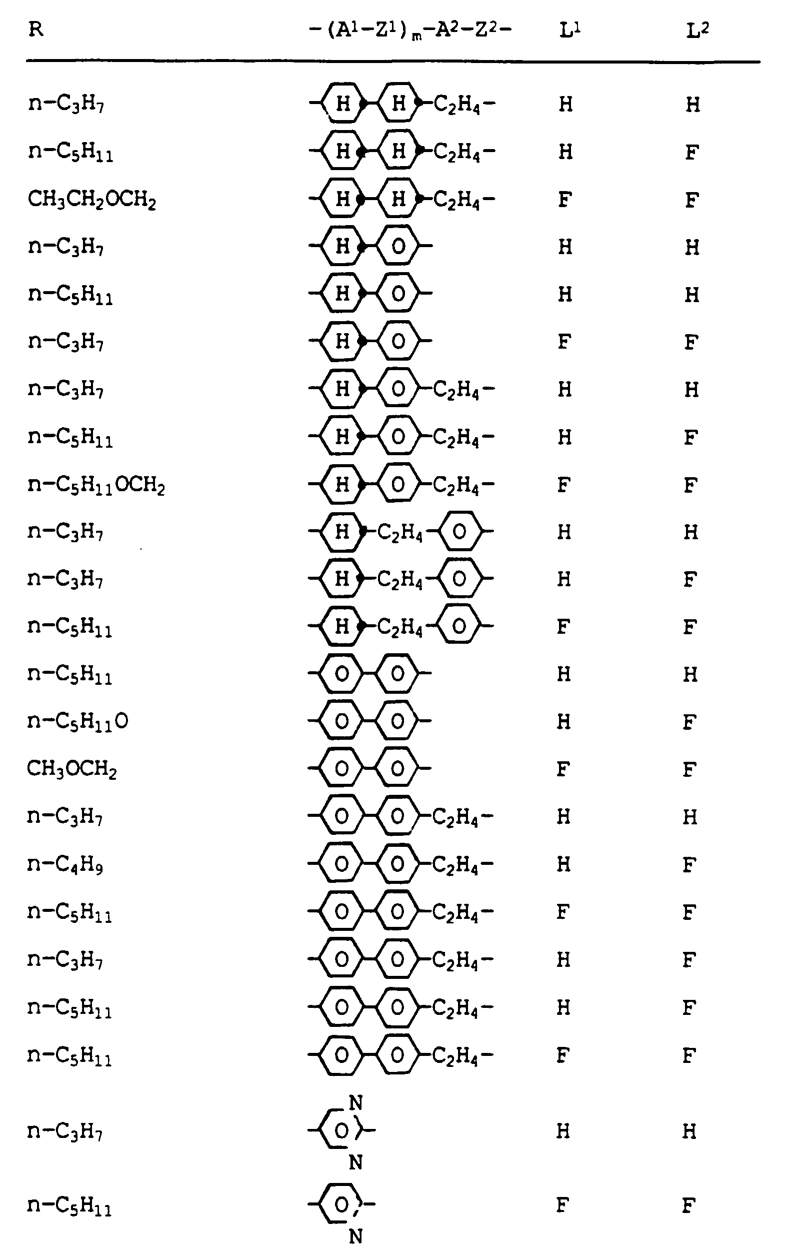

### Beispiel 14

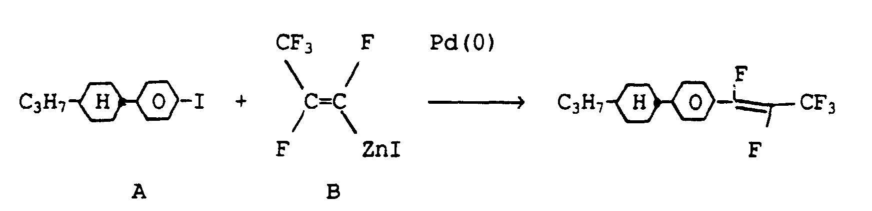

Nach dem von Burton et al. beschriebenen Verfahren wird aus 60 mmol (Z)-CF₃CF=CF-I mit 5,0 g aktiviertem Zink das entsprechende Zinkderivat B in Triethylenglykoldimethylether (Triglyme) bereitet (Dolliest et al. J. Am. Chem. Soc. 109, 219-225, 1987). Nach Zugabe von 0,05 1-(4-Propyl-trans-cyclohexyl)-4-iodbenzol A und 1,4 g Pd(PPh₃)₄ zu der Lösung des Zinkderivats wird 48 h bei 40 °C erwärmt. Danach wird auf Wasser gegossen und wie üblich aufgearbeitet.

Analog werden die folgenden Verbindungen der Formel 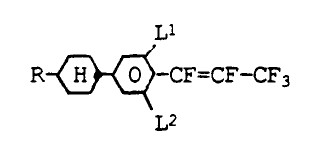 hergestellt:

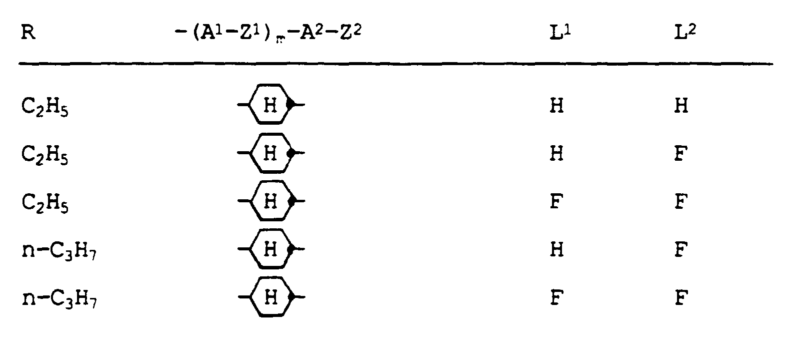

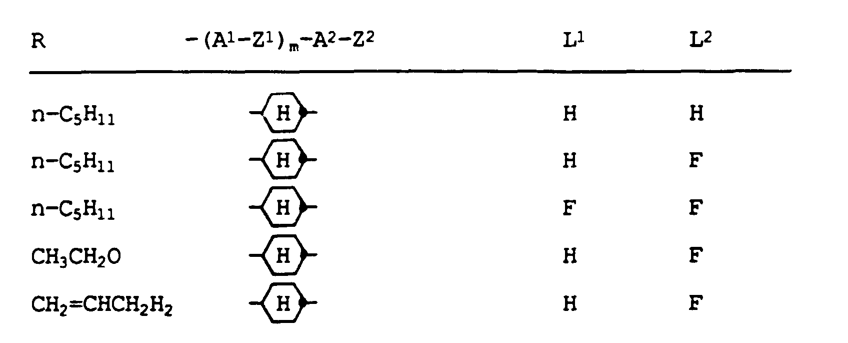

### Beispiel 15

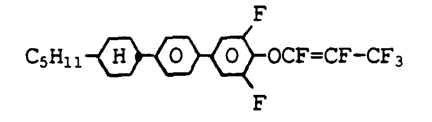

19,07 mmol {2,6-Difluor-4-[4-(4-pentylcyclohexyl)-phenyl]phenyl}-(1,1,1,2,3,4-hexafluorpropyl)-ether werden in 100 ml THF gelöst. Unter Kühlung (-78 °C) wird hierzu eine Lösung von 20 mmol Lithium-tetramethylpiperidin (hergestellt bei -20 °C aus 20 mmol 2,2,6,6-Tetramethylpiperidin in 10 ml THF und 12,5 ml einer 1,6-molaren Lösung von Butyllithium in Hexan) getropft. Die Mischung läßt man langsam auf Raumtemperatur erwärmen. Anschließend wird 0,5 h unter Rückfluß gekocht. Danach wird die Lösung bei Raumtemperatur unter vermindertem Druck zur Trockne eingedampft. Der Rückstand wird mit Dichlormethan extrahiert und wie üblich aufgearbeitet.

Analog werden die folgenden Verbindungen der Formel 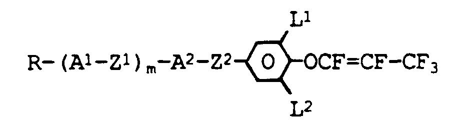 hergestellt:

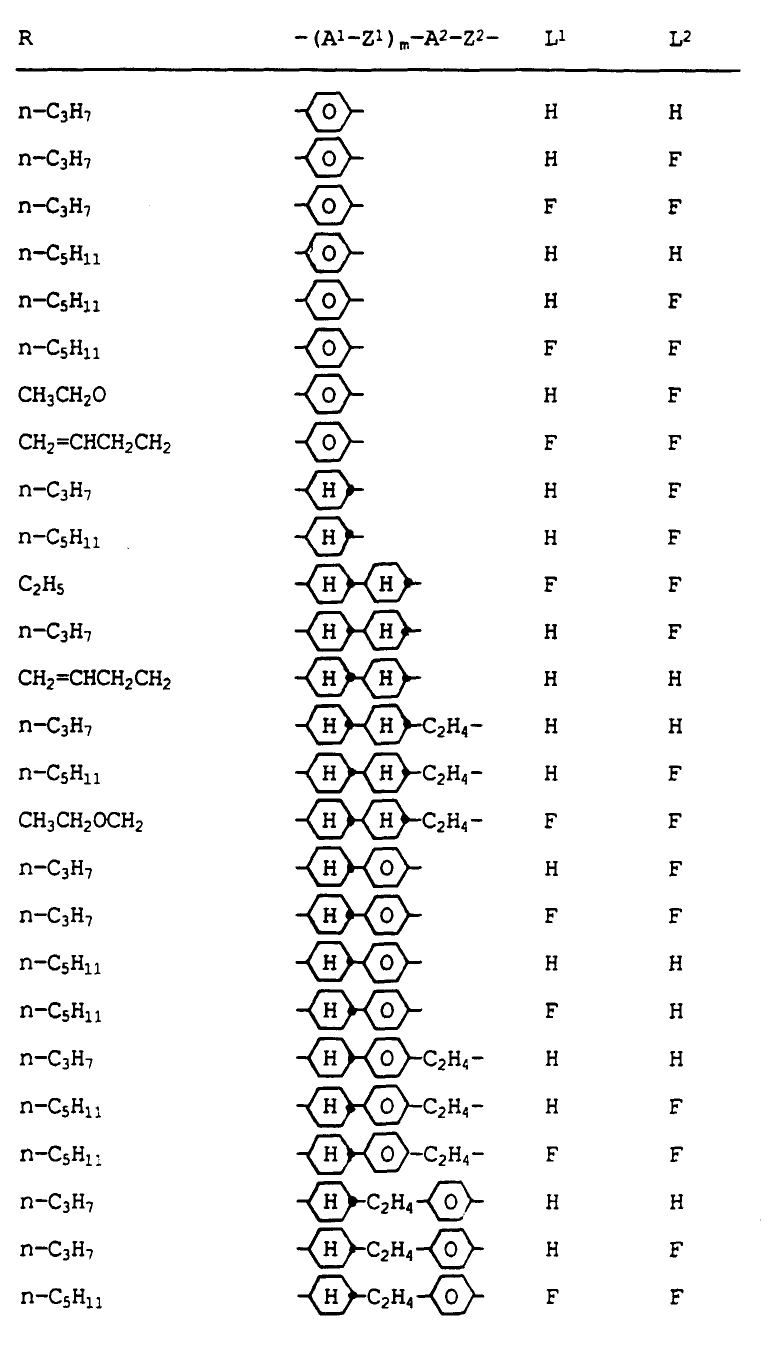

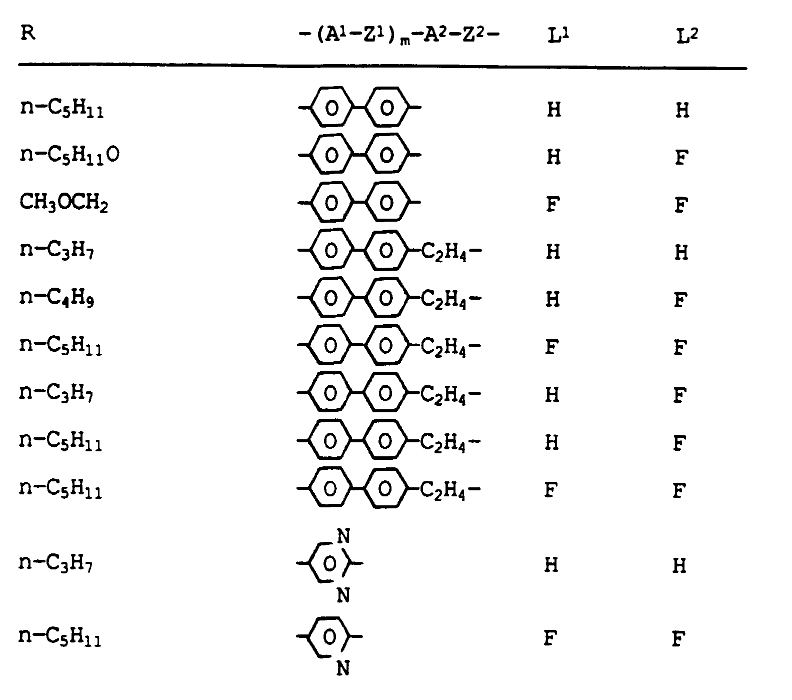

### Beispiel 16

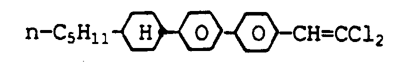

3 eq. Triphenylphosphin werden in abs. THF (5,0 ml/g PPh₃) gelöst und bei Raumtemperatur mit 1,5 eq. CCl₄ versetzt. Das entstehende Gemisch wird 2 h gerührt und anschließend werden 1,5 eq. aktiviertes Magnesium zugegeben. Dann werden weitere 0,5 h gerührt. Nach Zugabe von 1,0 eq n-Pentylcyclohexylphenyl-4-benzaldehyd wird das Gemisch 12 h gerührt, und über Kieselgel abfiltriert. Das Filtrat wird wie üblich aufgearbeitet.

Analog werden die folgenden Verbindungen der Formel 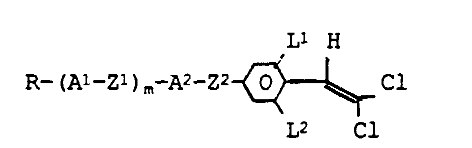 hergestellt:

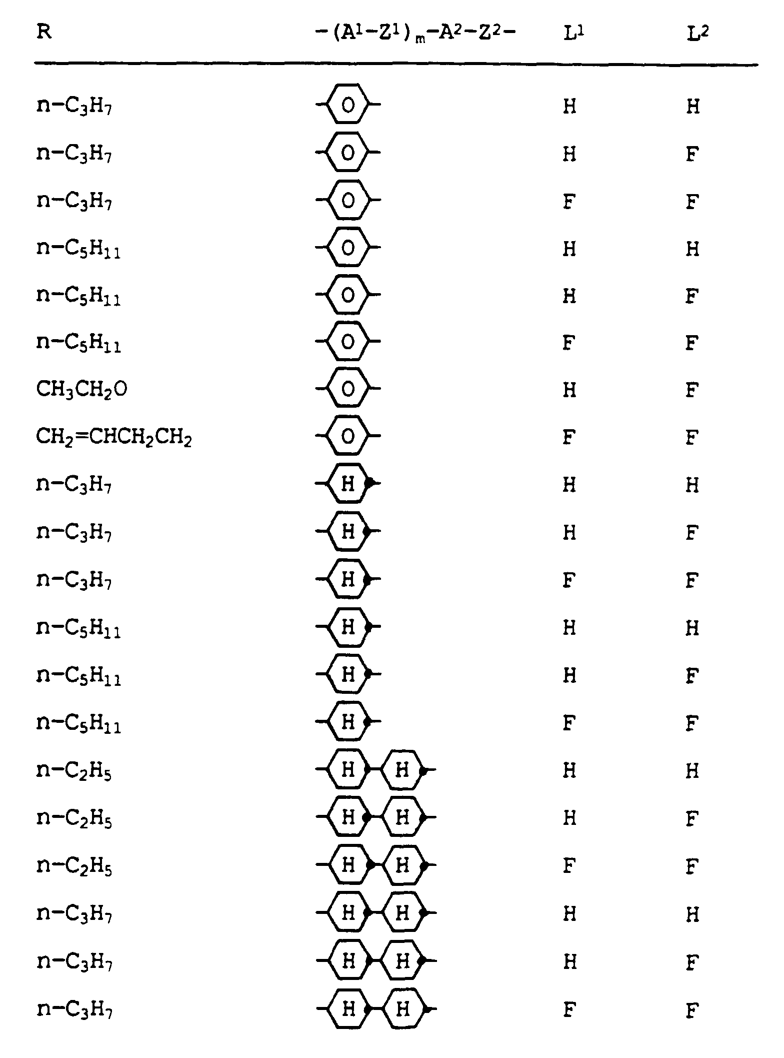

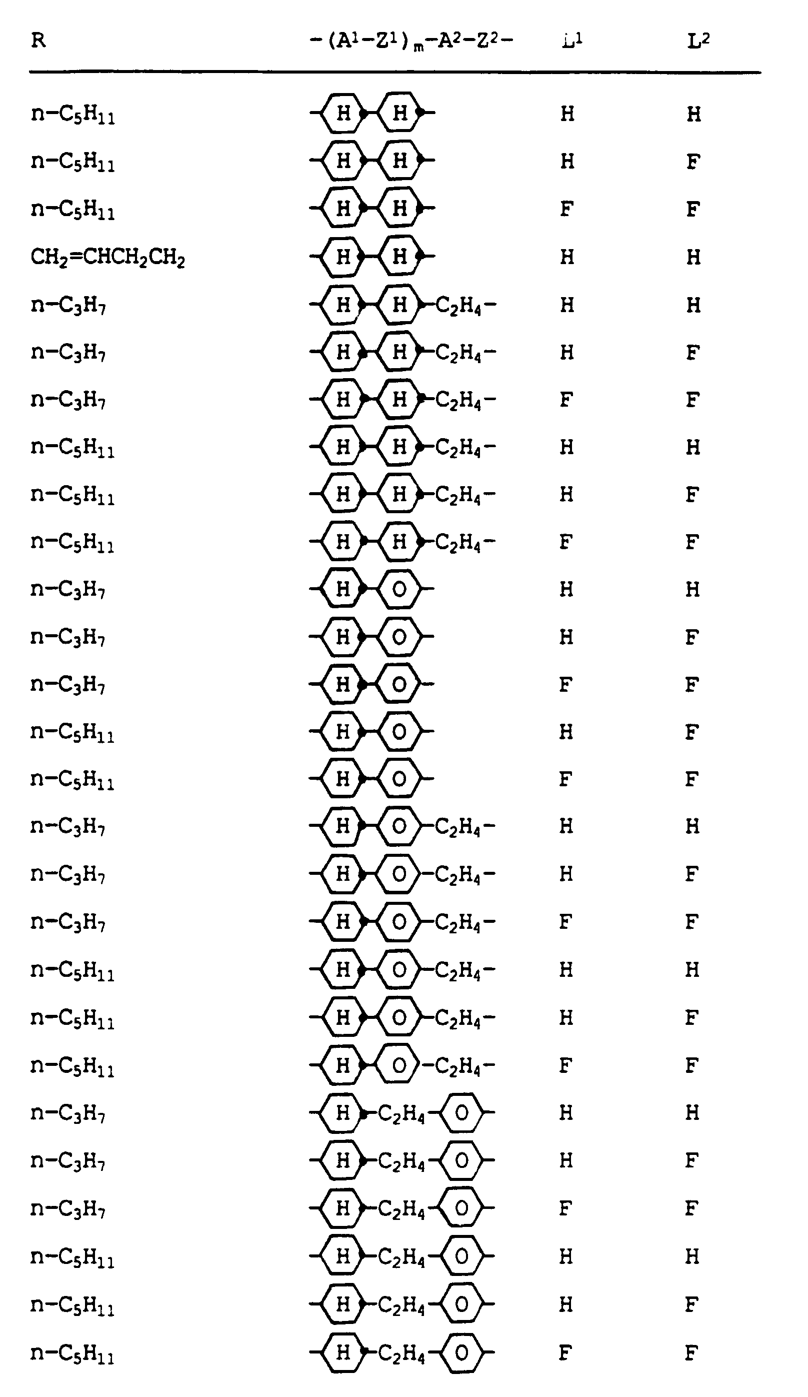

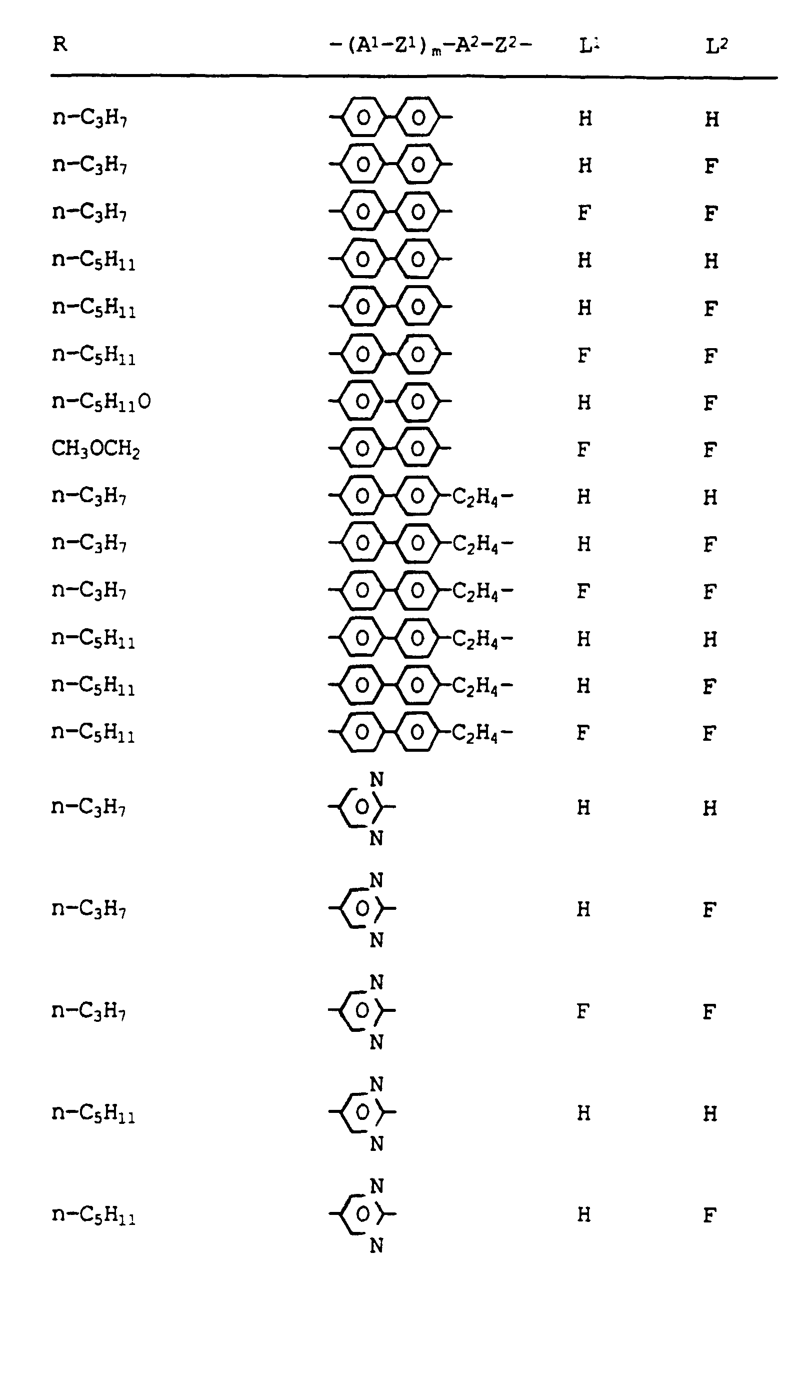

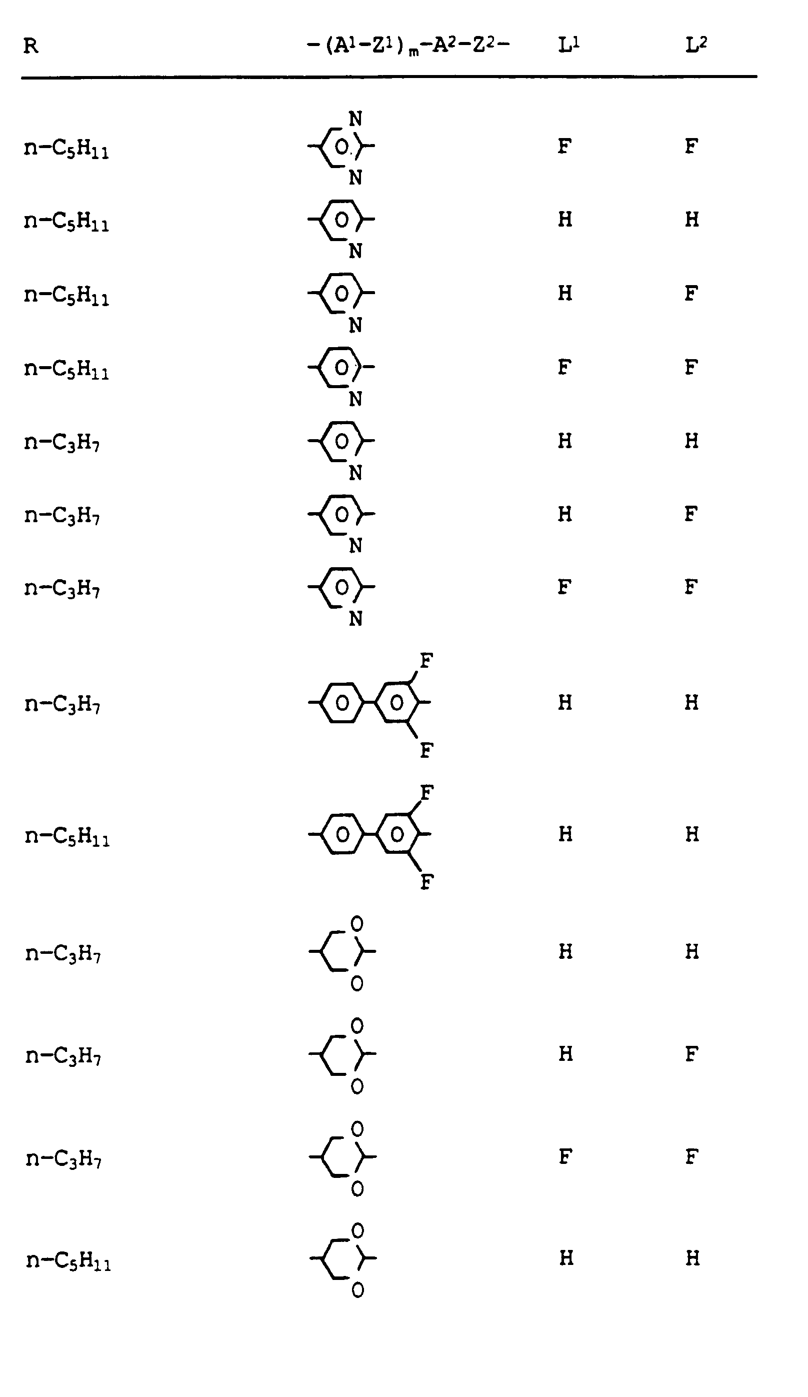

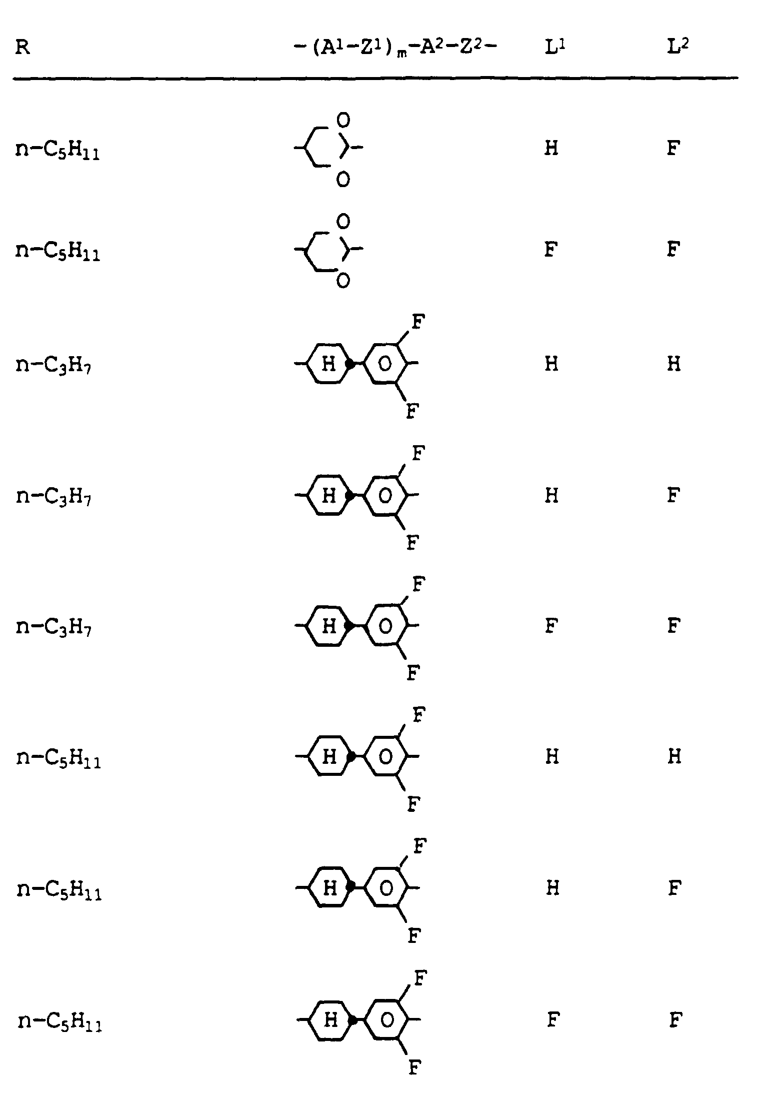

### Beispiel 17

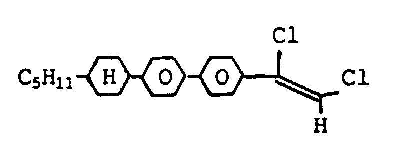

Zu 0,6 mol gepulvertem Aluminiumchlorid und 200 ml Dichlormethan werden unter Rühren bei 0 °C 0,52 mol Chloressigsäure zugetropft. Anschließend werden zu dem Gemisch 0,5 mol n-Pentylcyclohexylbiphenyl gegeben. Dann wird eine weitere Stunde gerührt und über Nacht stehen gelassen. Es wird auf Eis gegossen und wie üblich aufgearbeitet. Das erhaltene Chlorketon wird zunächst mit PCl₅ behandelt und danach mit KOH. Zuletzt wird wie üblich aufgearbeitet.

Analog werden die folgenden Verbindungen der Formel 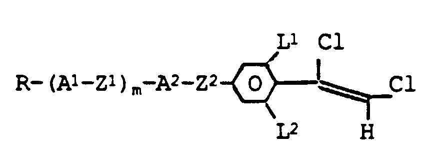 hergestellt:

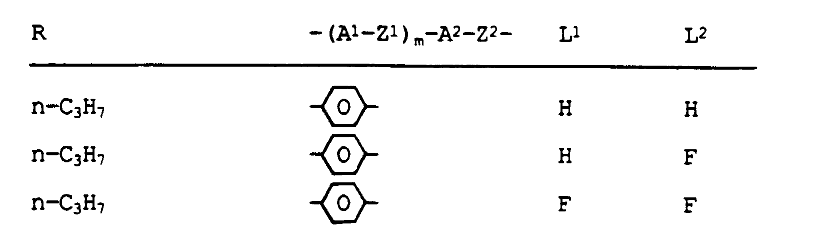

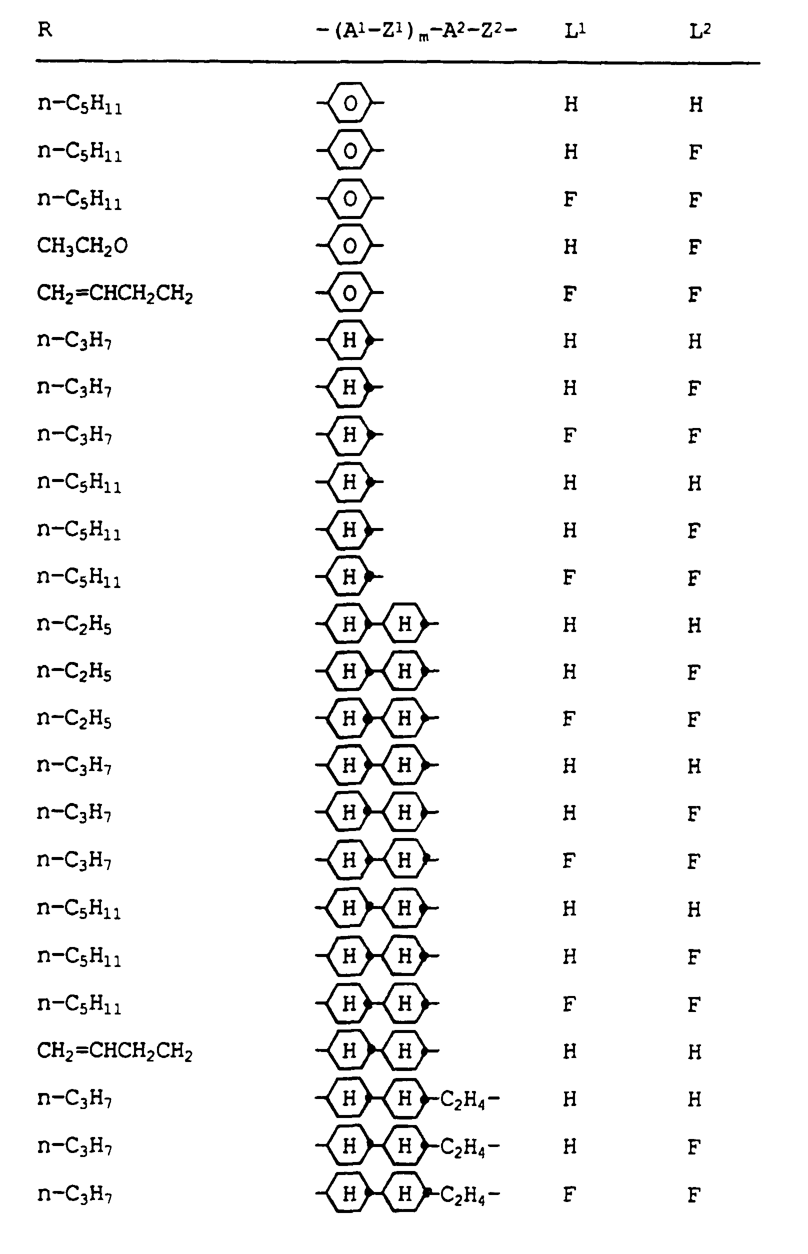

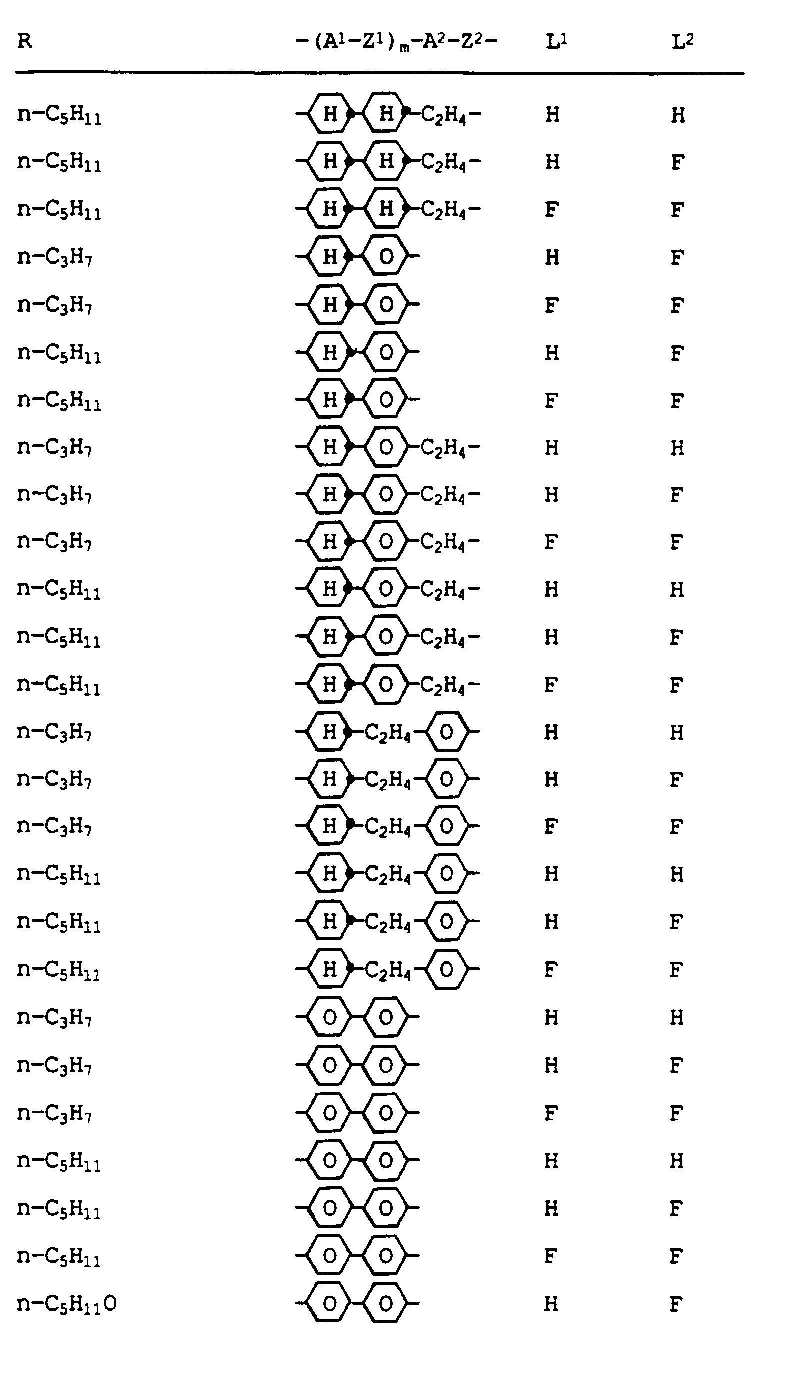

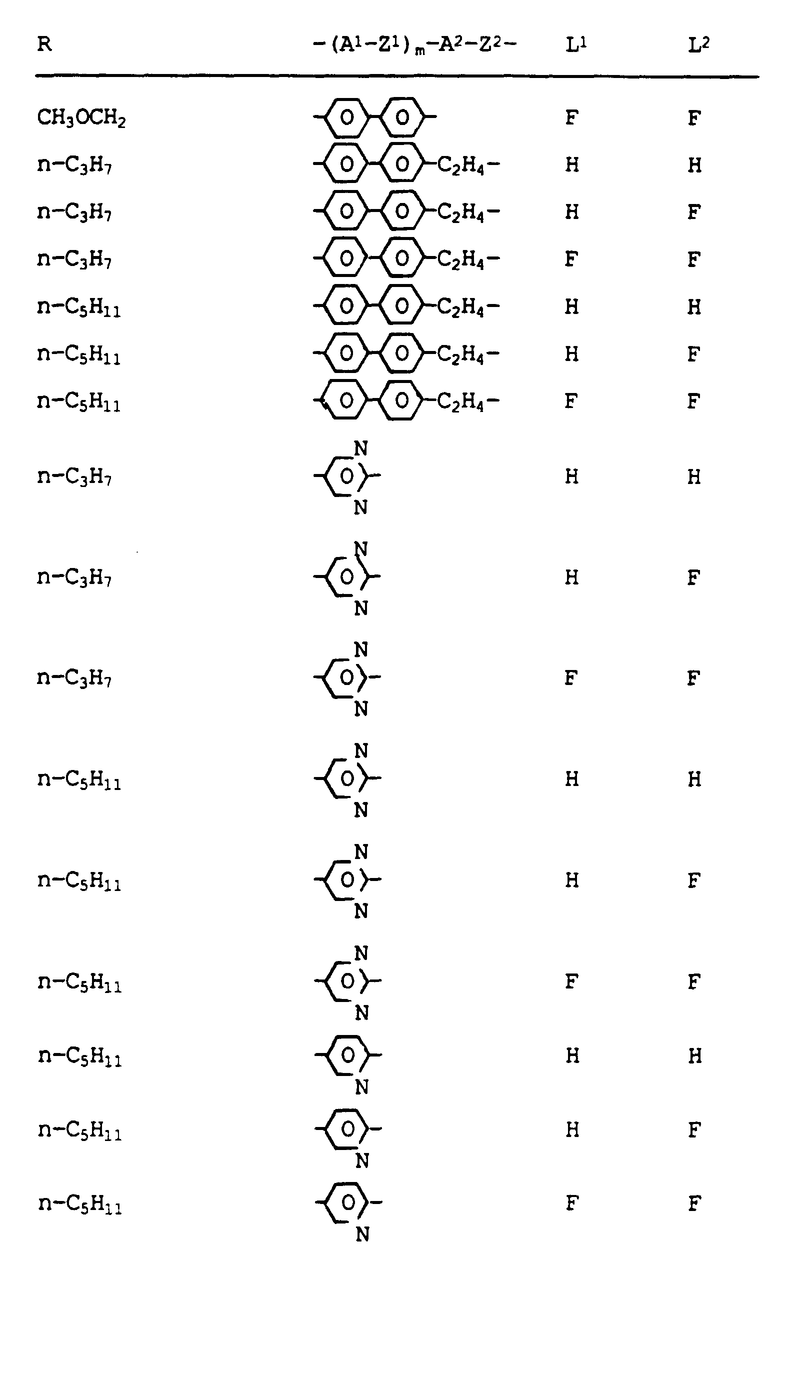

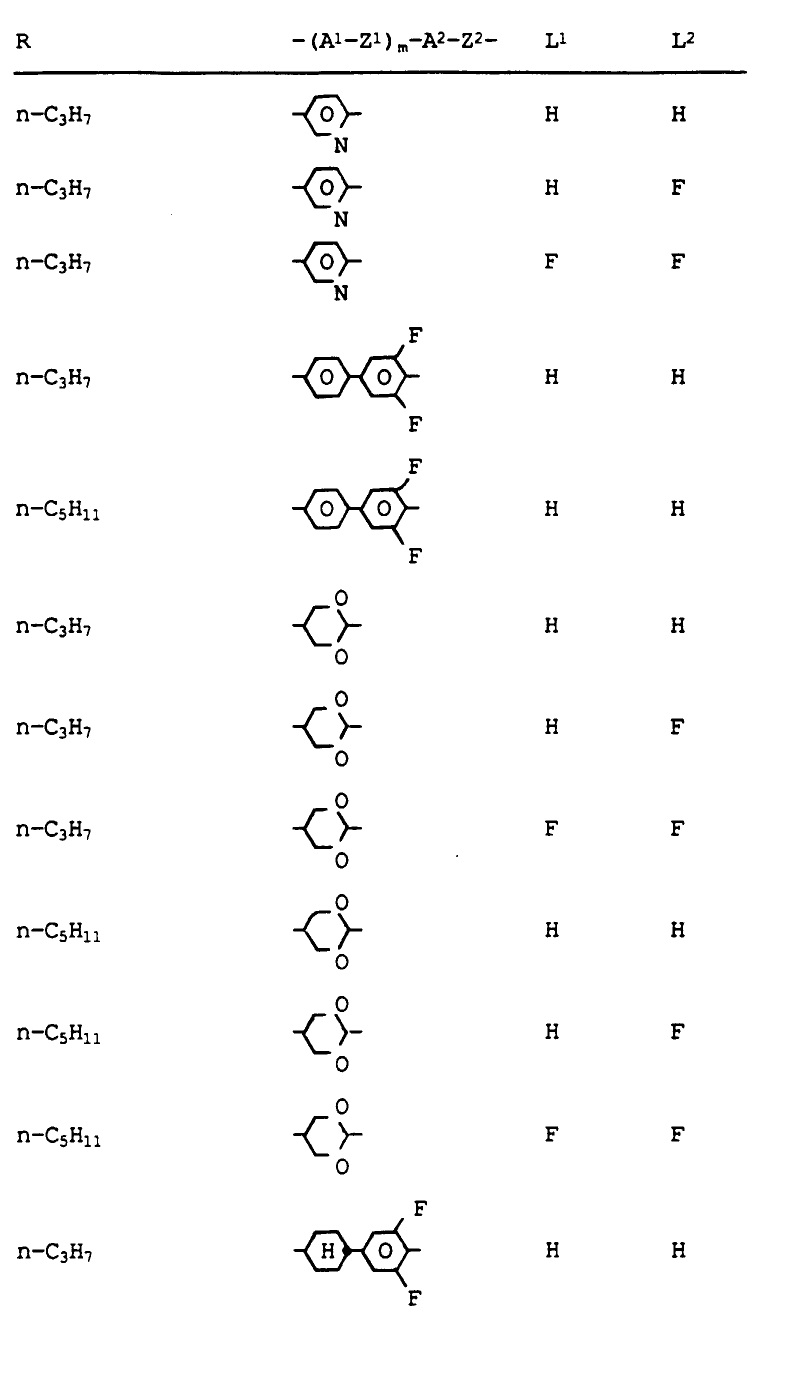

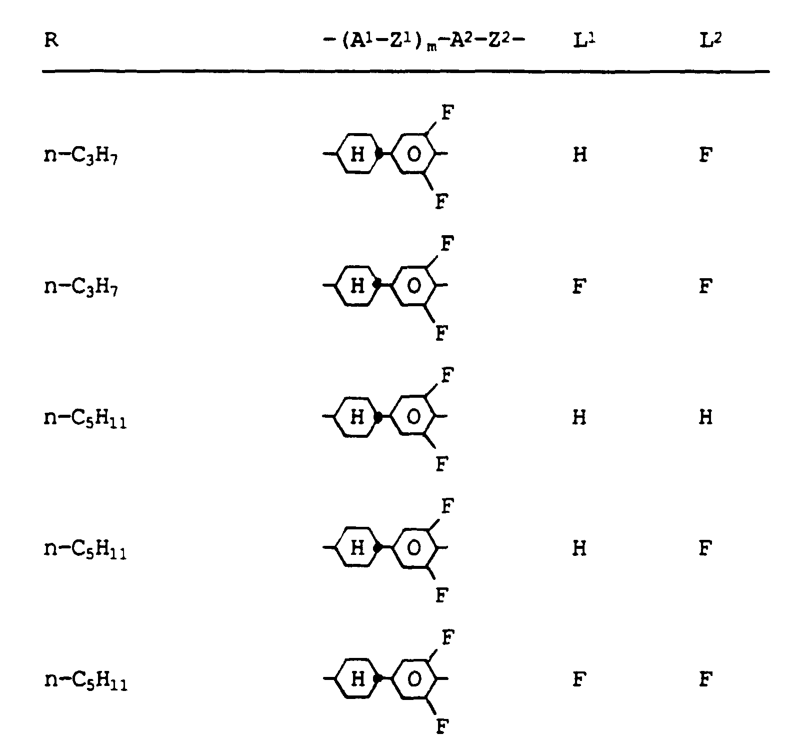

### Beispiel 18

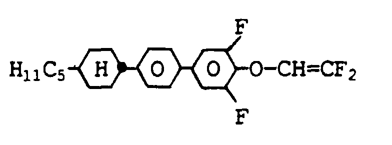

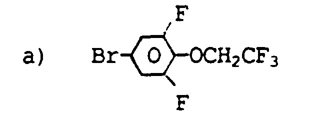

0,085 mol 4-Brom-2,6-difluorphenolat werden in 100 ml 1,3-Dimethyl-2-imidazolidinon (DMEU) gelöst, auf 140 °C erhitzt und tropfenweise mit 0,09 ml 2,2,2-Trifluorethylmethylsulfonat versetzt. Die Lösung wird 24 h bei 140 °C gerührt. Anschließend wird mit 500 ml Eiswasser versetzt und wie üblich aufgearbeitet.

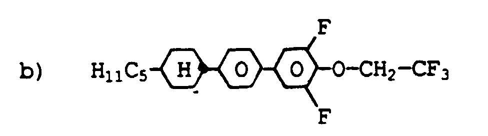

0,015 mol 4-Brom-2,6-difluorphenol-(2,2,2-trifluorethyl)ether werden mit 0,05 mol trans-n-Pentylcyclohexylphenylboronsäure, 20 ml Toluol, 10 ml Ethanol, 0,030 mol Natriumcarbonat und 0,86 mmol Tetrakis(triphenylphosphin)-palladium(0) versetzt und 2 h unter Rückfluß gekocht. Nach Zugabe von 100 ml Petrolether (40-80°) wird wie üblich aufgearbeitet.

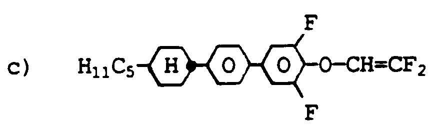

Zu 13,5 mmol BuLi (15 % in n-Hexan) in 10 ml THF werden bei -20 °C 13,5 mmol Diisopropylamin zugetropft. Anschließend wird die Lösung 10 min gerührt und zu einem Gemisch bestehend aus 10 ml THF, 13,5 mmol trans-n-Pentylcyclohexylphenyl-2,6-difluorphenol-(2,2,2-trifluorethyl)ether unter Schutzgas bei -40 °C zugetropft. Das Gemisch wird 0,5 h zunächst bei -40 °C und anschließend über Nacht bei Raumtemperatur gerührt. Nach der Hydrolyse wird wie üblich aufgearbeitet. K 31 N 103 I

Analog werden die folgneden Verbindungen der Formel 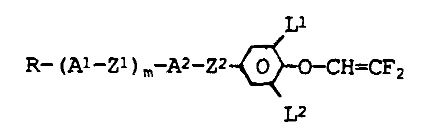 hergestellt.

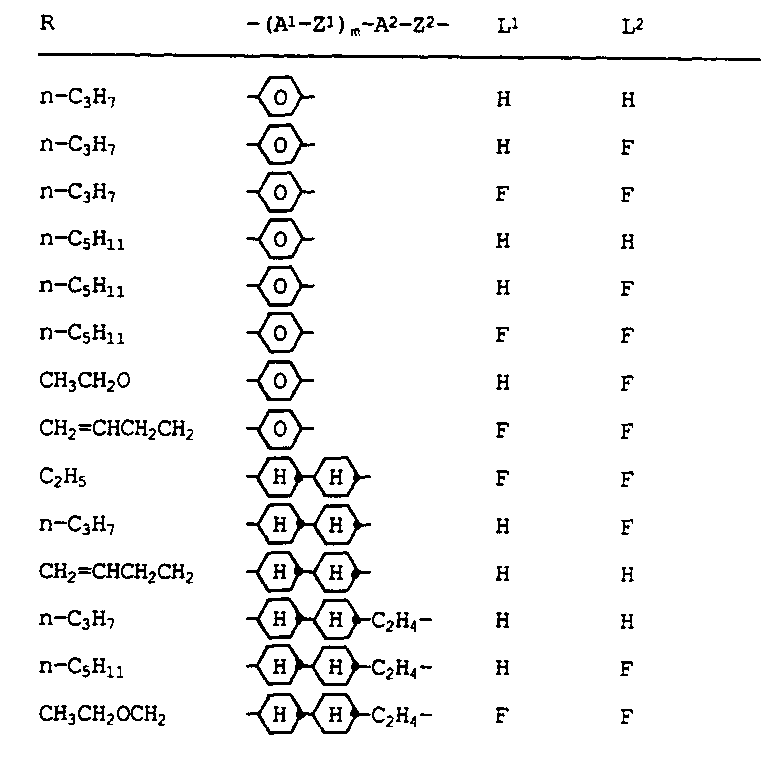

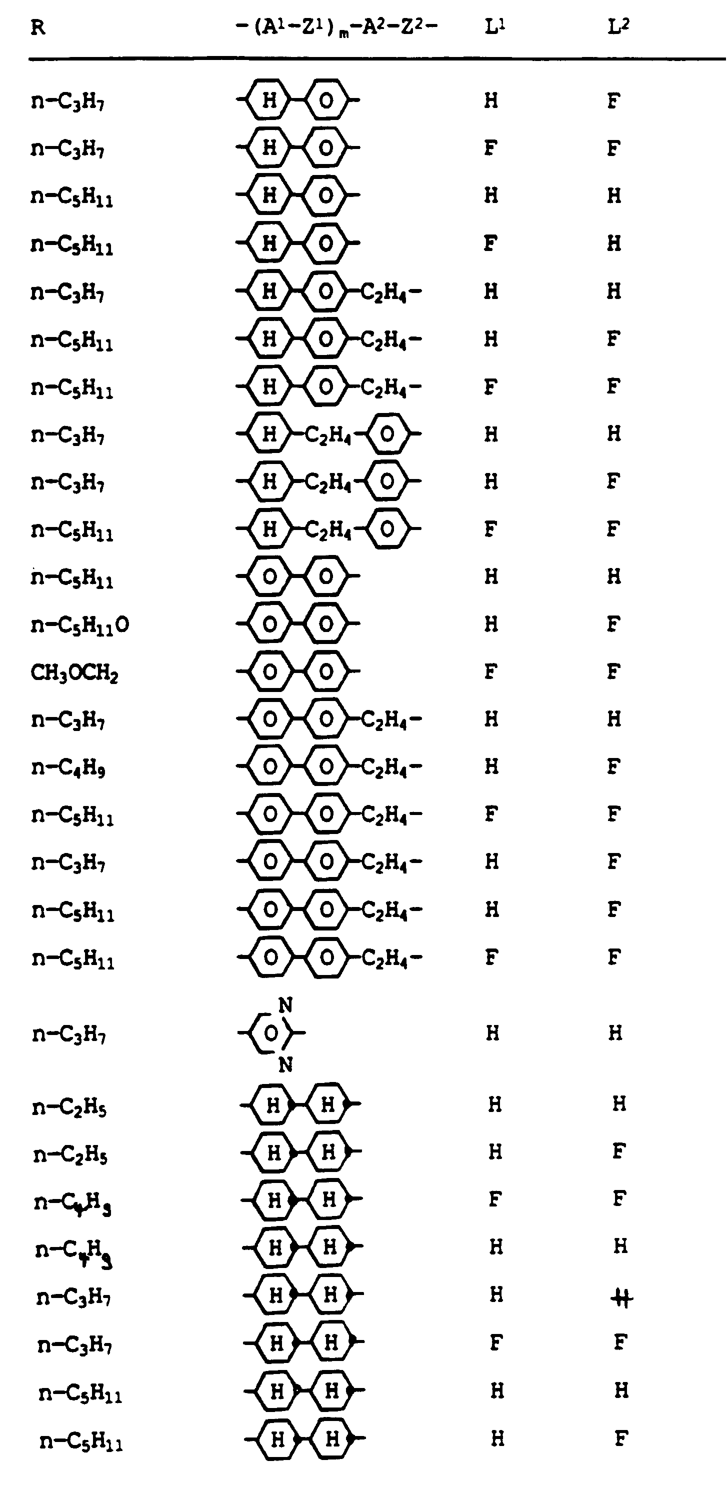

## Patentansprüche

1. Vinylverbindungen der Formel I, 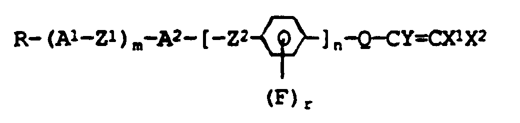 worin
R H, einen unsubstituierten, einen einfach durch CN oder CF₃ oder einen mindestens einfach durch Halogen substituierten Alkyl-oder Alkenylrest mit 1 bis 15 C-Atomen, wobei in diesen Resten auch eine oder mehrere CH₂-Gruppen jeweils unabhängig von einander durch -O-, -S-, 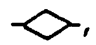 -CO-, -CO-O-, -O-CO- oder -O-CO-O- so ersetzt sein können, daß O-Atome nicht direkt miteinander verknüpft sind,
A¹ und A² jeweils unabhängig voneinander einen
(a) trans-1,4-Cyclohexylenrest, worin auch eine oder mehrere nicht benachbarte CH₂-Gruppen durch -O- und/oder -S- ersetzt sein können,
(b) 1,4-Phenylenrest, worin auch eine oder zwei CH-Gruppen durch N ersetzt sein können,
(c) Rest aus der Gruppe 1,4-Cyclohexenylen, 1,4-Bicyclo(2,2,2)-octylen, Piperidin-1,4-diyl, Naphthalin-2,6-diyl, Decahydronaphthalin-2,6-diyl und 1,2,3,4-Tetrahydronaphthalin-2,6-diyl,
wobei die Reste (a) und (b) durch CN oder Fluor substituiert sein können,
Z¹ und Z² jeweils unabhängig voneinander -CO-O-, -O-CO-, -CH₂O-, -OCH₂ -CH₂CH₂-, -CH=CH-, -C≡C- oder eine Einfachbindung, einer der Reste Z¹ und Z² auch -(CH₂)₄- oder -CH=CH- CH₂CH₂-,
Q eine Einfachbindung, -O-, -CH₂CH₂-, -C≡C-, trans-CH=CH-, -COO- oder -CH₂O-
X¹ H, F oder Cl,
X² F, Cl, CF₃ oder SF₅
Y H, F oder Cl,
r 0 bis 4,
n 0 oder 1, und
m 0, 1, 2 oder 3
bedeutet, mit der Maßgabe, daß im Falle Y = H und Q = Einfachbindung X¹ und X² Cl bedeuten.

2. Verbindungen der Formel I1 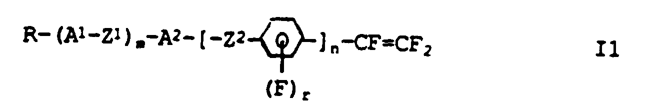 worin R, A¹, Z¹, A², Z², m, n und r die in Anspruch 1 angegebene Bedeutung haben.

3. Verbindungen der Formel I2 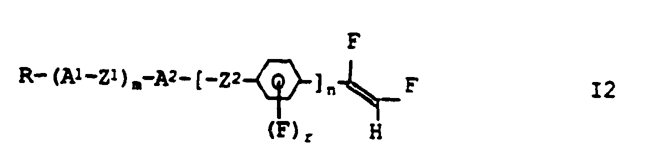 worin R, A¹, Z¹, A², Z², m, n und r die in Anspruch 1 angegebene Bedeutung haben.

4. Verbindungen der Formel I3 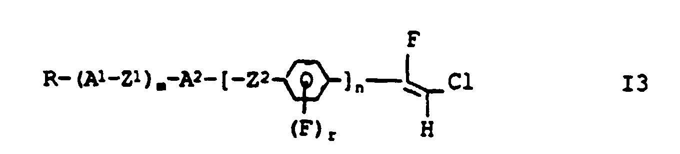 worin R, A¹, Z¹, A², Z², m, n und r die in Anspruch 1 angegebene Bedeutung haben.

5. Verbindungen der Formel I8, 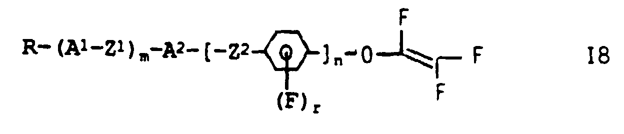 worin R, A¹, Z¹, A², Z², m, n und r die in Anspruch 1 angegebene Beudeutung haben.

6. Verbindungen der Formel I10, 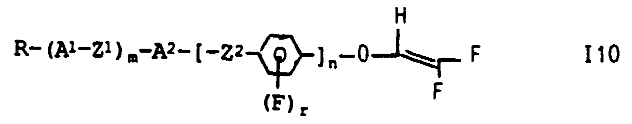 worin R, A¹, Z¹, A², Z², m, n und r die in Anspruch 1 angegebene Beudeutung haben.

7. Verbindungen der Formel I10, worin r = 0 ist.

8. Verwendung von Verbindungen der Formel I als Komponenten flüssigkristalliner Medien.

9. Flüssigkristallines Medium mit mindestens zwei flüssigkristallinen Komponenten, dadurch gekennzeichnet, daß es mindestens eine Verbindung der Formel I enthält.

10. Flüssigkristall-Anzeigeelement, dadurch gekennzeichnet, daß es ein flüssigkristallines Medium nach Anspruch 9 enthält.

11. Elektrooptisches Anzeigeelement, dadurch gekennzeichnet, daß es als Dielektrikum ein flüssigkristallines Medium nach Anspruch 9 enthält.

## Claims

1. Vinyl compounds of the formula I 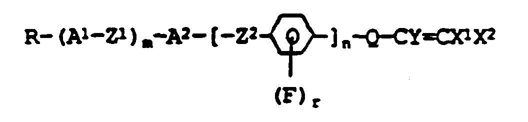 in which
R is H, an alkyl or alkenyl radical having 1 to 15 carbon atoms which is unsubstituted, monosubstituted by CN or CF₃ or at least monosubstituted by halogen, it also being possible for one or more CH₂ groups in these radicals to be replaced, in each case independently of one another, by -O-, -S-, 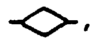 -CO-, -CO-O-, -O-CO- or -O-CO-O- in such a manner that oxygen atoms are not linked directly to one another,
A¹ and A² are each, independently of one another, a
(a) trans-1,4-cyclohexylene radical in which, in addition, one or more non-adjacent CH₂ groups may be replaced by -O- and/or -S-,
(b) 1,4-phenylene radical in which, in addition, one or two CH groups may be replaced by N,
(c) radical from the group consisting of 1,4-cyclohexenylene, 1,4-hicyclo(2,2,2)-octylene, piperidine-1,4-diyl, naphthalene-2,6-diyl, decahydronaphthalene-2,6-diyl and 1,2,3,4-tetrahydronaphthalene-2,6-diyl,
where the radicals (a) and (b) may be substituted by CN or fluorine,
Z¹ and Z² are each, independently of one another, -CO-O-, -O-CO-, -CH₂O-, -OCH₂-, -CH₂CH₂-, -CH=CH-, -C≡C- or a single bond, and one of the radicals Z¹ and Z² is alternatively -(CH₂)₄- or -CH=CH-CH₂CH₂-,
Q is a single bond, -O-, -CH₂-CH₂-, -C≡C-, trans- CH=CH-, -COO- or -CH₂O-,
X¹ is H, F or Cl,
X² is F, Cl, CF₃, or SF₅,
Y is H, F or Cl,
r is 0 to 4,
n is 0 or 1, and
m is 0, 1, 2 or 3,
with the proviso that, in the case where Y = H and Q = a single bond, X¹ and X² are Cl.

2. Compounds of the formula I1 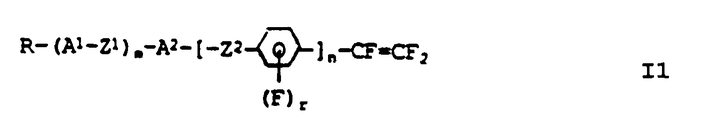 in which R, A¹, Z¹, A², Z², m, n and r are as defined in claim 1.

3. Compounds of the formula I2 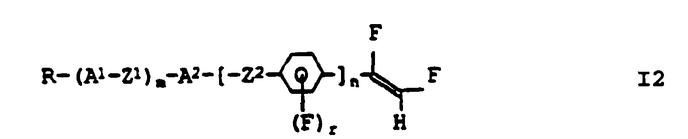 in which R, A¹, Z¹, A², Z², m, n and r are as defined in claim 1.

4. Compounds of the formula I3 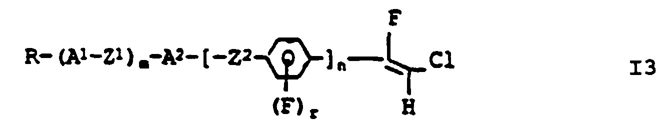 in which R, A¹, Z¹, A², Z², m, n and r are as defined in claim 1.

5. Compounds of the formula I8 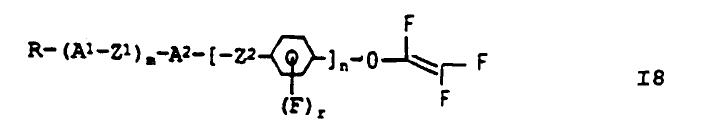 in which R, A¹, Z¹, A², Z², m, n and r are as defined in claim 1.

6. Compounds of the formula I10 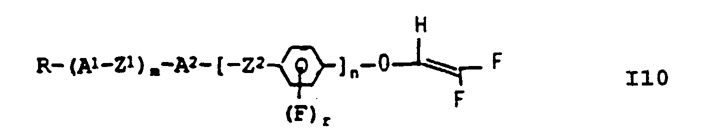 in which R, A¹, Z¹, A², Z², m, n and r are as defined in claim 1.

7. Compounds of the formula I10 in which r is 0.

8. Use of compounds of the formula I as components of liquid-crystalline media.

9. Liquid-crystalline medium containing at least two liquid-crystalline components, characterized in that it contains at least one compound of the formula I.

10. Liquid-crystal display element , characterized in that it contains a liquid-crystalline medium according to claim 9.

11. Electro-optical display element, characterized in that it contains, as dielectric, a liquid-crystalline medium according to claim 9.

## Revendications

1. Composés vinyliques de formule I 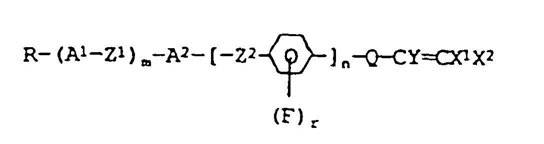 où
R représente H, un reste alkyle ou un reste alcényle comportant 1 à 15 atomes de C, non substitué ou monosubstitué par CN ou CF₃ ou au moins monosubstitué par un halogène, un ou plusieurs groupes CH₂ dans ces restes pouvant être remplacés, indépendamment les uns des autres, par -0-, -S-, 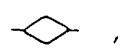 -CO-, -CO-O-, -O-CO- ou -O-CO-O-,
de telle façon que les atomes d'O ne soient pas directement reliés entre eux,
A¹ et A² représentent, indépendamment l'un de l'autre,
(a) un reste trans-1,4-cyclohexylène, un ou plusieurs groupes CH₂ non adjacents pouvant être également remplacés par -O- et/ou -S-,
(b) un reste 1,4-phénylène, un ou deux groupes CH pouvant être également remplacés par N,
(c) un reste provenant du groupe constitué par le 1,4-cyclohexénylène, le 1,4-bicyclo (2,2,2)-octylène, le pipéridine-1,4-diyle, le naphtalène-2,6-diyle, le décahydronaphtalène-2,6-diyle et le 1,2,3,4-tétrahydronaphtalène-2,6-diyle,
les restes (a) et (b) pouvant être substitués par CN ou le fluor,
Z¹ et Z² représentent, indépendamment l'un de l'autre, -CO-O-, -O-CO-, -CH₂O-, -OCH₂-, -CH₂CH₂-, -CH=CH-, -C≡C- ou une liaison simple, l'un des restes Z¹ et Z² pouvant également représenter -(CH₂)₄-ou -CH=CH-CH₂CH₂-,
Q représente une liaison simple, -O-, -CH₂CH₂-, -C≡C-, -CH=CH- trans, -COO- ou -CH₂O-,
X¹ H, F ou Cl,
X², F, Cl, CF₃ ou SF₅,
Y H, F ou Cl,
r est compris entre 0 et 4,
n est égal à 0 ou 1 et
m est égal à 0, 1, 2 ou 3,
sous réserve que, dans le cas où Y = H et Q = une liaison simple, X¹ et X² représentent Cl.

2. Composés de formule I1 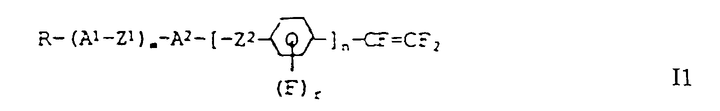 où R, A¹ , Z¹ , A² , Z² , m, n et r ont les significations indiquées dans la revendication 1.

3. Composés de formule I2 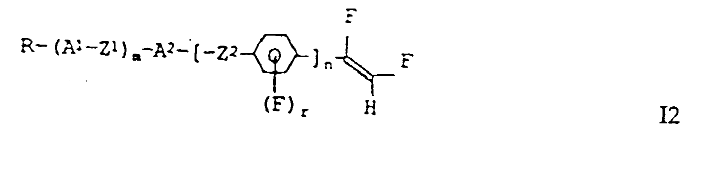 où R, A¹, Z¹ , A² , Z² , m, n et r ont les significations indiquées dans la revendication 1.

4. Composés de formule I3 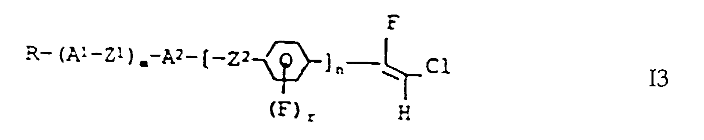 où R, A¹, Z¹, A², Z², m, n et r ont les significations indiquées dans la revendication 1.

5. Composés de formule I8 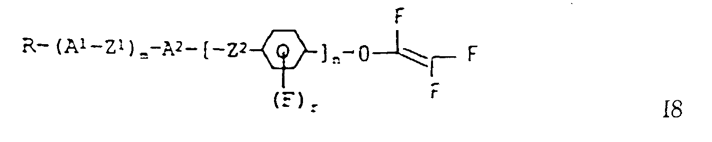 où R, A¹, Z¹, A², Z² , m, n et r ont les significations indiquées dans la revendication 1.

6. Composés de formule I10 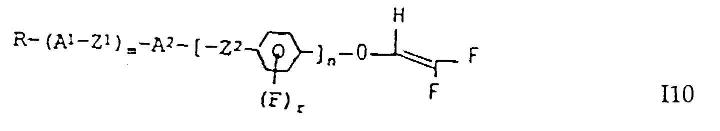 où R, A¹, Z¹, A², Z², m, n et r ont les significations indiquées dans la revendication 1.

7. Composés de formule I10 où r est égal à 0.

8. Utilisation des composés de formule I comme composants de milieux à cristaux liquides.

9. Milieu à cristaux liquides comportant au moins deux composants à cristaux liquides, caractérisé en ce qu'il contient au moins un composé de formule I.

10. Elément d'affichage à cristaux liquides, caractérisé en ce qu'il contient un milieu à cristaux liquides selon la revendication 9.

11. Elément d'affichage électro-optique, caractérisé en ce qu'il contient, en tant que diélectrique, un milieu à cristaux liquides selon la revendication 9.
